(19) Europäisches Patentamt
European Patent Office
Office européen des brevets

(11) EP 4 772 498 A1

(12) **EUROPEAN PATENT APPLICATION**
published in accordance with Art. 153(4) EPC

(43) Date of publication:
**08.07.2026 Bulletin 2026/28**

(21) Application number: **24858592.9**

(22) Date of filing: **28.08.2024**

(51) International Patent Classification (IPC):
***C07D 213/84*** *(2006.01)* ***A61K 31/44*** *(2006.01)*
***A61P 17/14*** *(2006.01)*

(52) Cooperative Patent Classification (CPC):
**A61K 31/44; A61P 17/14; C07D 213/84**

(86) International application number:
**PCT/CN2024/114989**

(87) International publication number:
**WO 2025/045058 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.08.2023 CN 202311091809**
**20.06.2024 CN 202410802546**

(71) Applicants:
- **China Pharmaceutical University**
  **Nanjing, Jiangsu 211198 (CN)**
- **Jiangsu Simcere Pharmaceutical Co., Ltd.**
  **Nanjing, Jiangsu 210042 (CN)**

(72) Inventors:
- **ZHANG, Yan**
  **Nanjing, Jiangsu 210042 (CN)**
- **JIAO, Yu**
  **Nanjing, Jiangsu 211198 (CN)**
- **HUANG, Liancheng**
  **Nanjing, Jiangsu 210042 (CN)**
- **ZHAO, Siqi**
  **Beijing 102206 (CN)**
- **TANG, Feng**
  **Shanghai 201318 (CN)**
- **GAO, Zhenna**
  **Nanjing, Jiangsu 210042 (CN)**
- **SHEN, Xiaoling**
  **Nanjing, Jiangsu 210042 (CN)**
- **LIU, Le**
  **Nanjing, Jiangsu 210042 (CN)**
- **CHEN, Yadong**
  **Nanjing, Jiangsu 211198 (CN)**
- **LU, Tao**
  **Nanjing, Jiangsu 211198 (CN)**
- **PENG, Shaoping**
  **Nanjing, Jiangsu 210042 (CN)**
- **WANG, Feng**
  **Nanjing, Jiangsu 210042 (CN)**

(74) Representative: **Richly & Ritschel Patentanwälte PartG mbB**
**Sattlerweg 20**
**51429 Bergisch Gladbach (DE)**

Remarks:
The applicant has filed a text with which it is intended to bring the translation into conformity with the application as filed (Art. 14(2) EPC).

(54) **COMPOUND ACTING AS ANDROGEN RECEPTOR ANTAGONIST**

(57) Provided are a compound represented by formula (I) or a stereoisomer or a pharmaceutical acceptable salt thereof, a pharmaceutical composition comprising same, and the use thereof acting as an androgen receptor (AR) antagonist. The compound is preferably used for the treatment of androgen receptor-mediated alopecia.

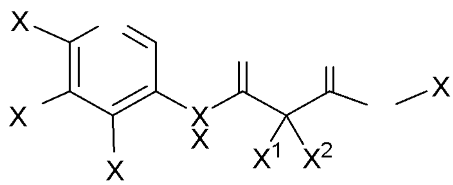

(I)

EP 4 772 498 A1

## Description

### CROSS-REFERENCE TO RELATED APPLICATIONS

The present disclosure claims the priority and benefit to the following Chinese patent applications filed with China National Intellectual Property Administration: Chinese Patent Application No. 202311091809.5 filed on August 28, 2023 and Chinese Patent Application No. 202410802546.2 filed on June 20, 2024, which are incorporated herein by reference in their entirety.

### TECHNICAL FIELD

The present application relates to the field of pharmaceutical chemistry, and in particular, to an amide compound or a stereoisomer thereof or a pharmaceutically acceptable salt thereof, a pharmaceutical composition comprising same, and use thereof as an androgen receptor (AR) antagonist.

### BACKGROUND

Androgen receptor (AR) is a member of the nuclear receptor family. AR comprises four main regions: an N-terminal domain (NTD) for transcriptional control, a DNA binding domain (DBD), a hinge region, and a C-terminal ligand binding domain (LBD). It is activated in benign prostate hyperplasia, prostate cancer, seborrhea, acne, premenstrual syndrome, lung cancer, polycystic ovary syndrome, hirsutism, and alopecia. Therefore, the androgen receptor is an important target in various fields of drug discovery. Androgenetic alopecia (AGA) is the most common type of alopecia in clinic practice at present. Although its specific pathogenesis has not been completely revealed yet, most researchers believe that it is related to androgen metabolism. Studies have shown that the effect of androgen on susceptible hair follicles is increased due to the increased expression of androgen receptor gene and/or type II 5α reductase gene in hair follicles of the alopecia area. For androgenetic alopecia, the dermal cells in susceptible hair follicles contain specific 5α reductase type II that can convert androgen testosterone circulating in the blood to this region into dihydrotestosterone, which causes a series of reactions by binding to the androgen receptor in the cells, resulting in progressive miniaturization and alopecia in the hair follicles and even atrichia. Currently, the FDA has only approved minoxidil and finasteride for the treatment of androgenetic alopecia. Minoxidil is a potassium channel opener for topical use, with its exact mechanism in androgenetic alopecia incompletely elucidated. However, minoxidil has a long time to onset, as well as adverse reactions such as pruritus, contact dermatitis, and aggravated alopecia after discontinuation. Finasteride is an inhibitor of the 5-α reductase type II that decreases the level of dihydrotestosterone in the serum and scalp for the treatment of AGA in males. However, erectile dysfunction, ejaculation disorder, and hyposexuality due to the long-term use of finasteride deter patients with alopecia. Therefore, the research on the degradation and/or antagonistic activity of novel molecules against AR is of great significance for further research on androgenetic alopecia.

### SUMMARY

The present disclosure provides a compound of formula (I) or a stereoisomer thereof or a pharmaceutically acceptable salt thereof,

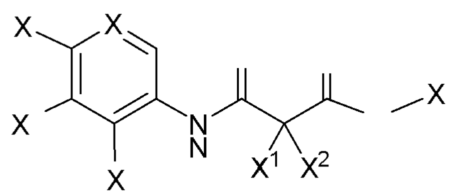

(I)

wherein

X is selected from CH or N;
Y is selected from S, O, or $NR^7$;
$R^1$ is selected from OH or $-O-C_1-C_6$ alkyl;
$R^2$ is selected from $C_1-C_6$ alkyl, $C_1-C_6$ deuterated alkyl, or $C_1-C_6$ haloalkyl;
$R^3$ is selected from $C_1-C_6$ alkyl, $C_1-C_6$ deuterated alkyl, $C_3-C_6$ cycloalkyl, 4- to 10-membered heterocyclyl, or 5- to 10-membered heteroaryl, wherein the $C_1$-$C_6$ alkyl, $C_3$-$C_6$ cycloalkyl, 4- to 10-membered heterocyclyl, or 5- to 10-membered heteroaryl is optionally substituted with $R^{3a}$;
$R^{3a}$ is selected from halogen, OH, CN, $NH_2$, -$COR^{3b}$, -$COOR^{3b}$, -$NHCOR^{3b}$, -$CONHR^{3b}$, -O-$C_1$-$C_6$ alkyl, phenyl, $C_1$-$C_6$ alkyl, 4- to 6-membered heterocyclyl, or 5- to 10-membered heteroaryl, wherein the phenyl, $C_1$-$C_6$ alkyl, 4- to 6-membered heterocyclyl, or 5- to 10-membered heteroaryl is optionally substituted with $R^{3c}$;
$R^{3b}$ is selected from H, $C_1$-$C_6$ alkyl, phenyl, or 5- to 6-membered heteroaryl;
$R^{3c}$ is selected from -$COOC_1$-$C_6$ alkyl, $C_1$-$C_6$ alkyl, -O-$C_1$-$C_6$ alkyl, phenyl, or 5- to 6-membered heteroaryl;
$R^4$ is selected from $NO_2$, halogen, or CN;
$R^5$ is selected from halogen, $C_1$-$C_6$ alkyl, $C_1$-$C_6$ haloalkyl, -$COOC_1$-$C_6$ alkyl, -S-$C_1$-$C_6$ alkyl, -O-$C_1$-$C_6$ alkyl, -Se-$C_1$-$C_6$ alkyl, -$S(O)_2$-$C_1$-$C_6$ alkyl, or -S(O)-$C_1$-$C_6$ alkyl;
$R^6$ is selected from H, halogen, $C_1$-$C_6$ alkyl, $C_1$-$C_6$ haloalkyl, or -O-$C_1$-$C_6$ alkyl;
$R^7$ is selected from H or $C_1$-$C_6$ alkyl.

In some embodiments, X is N.

In some embodiments, Y is selected from O or $NR^7$.

In some embodiments, Y is selected from S, O, or NH.

In some embodiments, Y is selected from S or O.

In some embodiments, $R^1$ is selected from OH or -O-$CH_3$.

In some embodiments, $R^1$ is OH.

In some embodiments, $R^2$ is selected from $CH_3$ or $CF_3$.

In some embodiments, $R^1$ is OH, and $R^2$ is $CH_3$.

In some embodiments, $R^3$ is selected from $C_1$-$C_6$ alkyl, $C_3$-$C_6$ cycloalkyl, 4- to 10-membered heterocyclyl, or 5- to 10-membered heteroaryl, wherein the $C_1$-$C_6$ alkyl, $C_3$-$C_6$ cycloalkyl, 4- to 10-membered heterocyclyl, or 5- to 10-membered heteroaryl is optionally substituted with $R^{3a}$.

In some embodiments, $R^3$ is selected from $C_1$-$C_6$ alkyl, 4- to 10-membered heterocyclyl, or 5- to 10-membered heteroaryl, wherein the $C_1$-$C_6$ alkyl, 4- to 10-membered heterocyclyl, or 5- to 10-membered heteroaryl is optionally substituted with $R^{3a}$,

In some embodiments, $R^3$ is selected from $C_1$-$C_6$ alkyl, 4- to 6-membered heterocyclyl, or 5- to 10-membered heteroaryl, wherein the $C_1$-$C_6$ alkyl, 4- to 6-membered heterocyclyl, or 5- to 10-membered heteroaryl is optionally substituted with $R^{3a}$,

In some embodiments, $R^3$ is selected from $C_1$-$C_6$ alkyl, $C_3$-$C_6$ cycloalkyl, 4- to 6-membered heterocyclyl, or 5- to 6-membered heteroaryl, wherein the $C_1$-$C_6$ alkyl, $C_3$-$C_6$ cycloalkyl, 4- to 6-membered heterocyclyl, or 5- to 6-membered heteroaryl is optionally substituted with $R^{3a}$.

In some embodiments, $R^3$ is selected from $C_1$-$C_6$ alkyl, 4- to 6-membered heterocyclyl, or 5- to 6-membered heteroaryl, wherein the $C_1$-$C_6$ alkyl, 4- to 6-membered heterocyclyl, or 5- to 6-membered heteroaryl is optionally substituted with $R^{3a}$,

In some embodiments, $R^3$ is selected from $C_1$-$C_6$ alkyl or 4- to 6-membered heterocyclyl, wherein the $C_1$-$C_6$ alkyl or 4- to 6-membered heterocyclyl is optionally substituted with $R^{3a}$,

In some embodiments, $R^3$ is selected from $C_1$-$C_6$ alkyl, wherein the $C_1$-$C_6$ alkyl is optionally substituted with $R^{3a}$,

In some embodiments, $R^3$ is selected from methyl, ethyl, propyl, isopropyl, n-butyl, tert-butyl, isobutyl, cyclobutyl, oxetanyl, tetrahydrofuranyl, tetrahydropyranyl, pyrazolyl, or pyridinyl, wherein the methyl, ethyl, propyl, isopropyl, n-butyl, tert-butyl, isobutyl, cyclobutyl, oxetanyl, tetrahydrofuranyl, tetrahydropyranyl, pyrazolyl, or pyridinyl is optionally substituted with $R^{3a}$,

In some embodiments, $R^3$ is selected from methyl, ethyl, propyl, isopropyl, n-butyl, tert-butyl, oxetanyl, tetrahydrofuranyl, tetrahydropyranyl, pyrazolyl, or pyridinyl, wherein the methyl, ethyl, propyl, isopropyl, n-butyl, tert-butyl, oxetanyl, tetrahydrofuranyl, tetrahydropyranyl, pyrazolyl, or pyridinyl is optionally substituted with $R^{3a}$,

In some embodiments, $R^3$ is selected from methyl, ethyl, propyl, isopropyl, n-butyl, tert-butyl, or isobutyl, wherein the methyl, ethyl, propyl, isopropyl, n-butyl, tert-butyl, or isobutyl is optionally substituted with $R^{3a}$,

In some embodiments, $R^{3a}$ is selected from halogen, OH, -$COR^{3b}$, -$COOR^{3b}$, -$NHCOR^{3b}$, -O-$C_1$-$C_6$ alkyl, phenyl, $C_1$-$C_6$ alkyl, 4- to 6-membered heterocyclyl, or 5- to 10-membered heteroaryl, wherein the phenyl, $C_1$-$C_6$ alkyl, 4- to 6-membered heterocyclyl, or 5- to 10-membered heteroaryl is optionally substituted with $R^{3c}$.

In some embodiments, $R^{3a}$ is selected from F, OH, -O-$CH_3$, -$COCH_3$, -$COOCH_2CH_3$, -CO-phenyl, - NHCO-pyridinyl, oxetanyl, pyrrolyl, 1,3-dioxolanyl, phenyl, or pyridinyl, wherein the oxetanyl, pyrrolyl, 1,3-dioxolanyl, phenyl, or pyridinyl is optionally substituted with $R^{3c}$.

In some embodiments, $R^{3a}$ is selected from F, OH, -O-$CH_3$, -$COCH_3$, -$COOCH_2CH_3$, -CO-phenyl, - NHCO-pyridinyl, oxetanyl, pyrrolyl, pyridinyl,

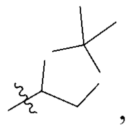, or 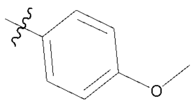.

In some embodiments, $R^{3b}$ is selected from $C_1$-$C_6$ alkyl, phenyl, or 5- to 6-membered heteroaryl.
In some embodiments, $R^{3c}$ is selected from $C_1$-$C_6$ alkyl, -O-$C_1$-$C_6$ alkyl, phenyl, or 5- to 6-membered heteroaryl.
In some embodiments, $R^{3c}$ is selected from $C_1$-$C_6$ alkyl or -O-$C_1$-$C_6$ alkyl.
In some embodiments, $R^3$ is selected from methyl, ethyl, propyl, isopropyl, n-butyl, $CH_2CH_2F$, tert-butyl, $CH_2CF_3$,

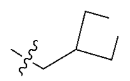,

oxetanyl, tetrahydrofuranyl, tetrahydropyranyl, $CH_2CH_2OH$, $(CH_2)_3OH$, $(CH_2)_4OH$, $(CH_2)_3OCH_3$,

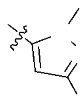

2 2 ,

$(CH_2)_3COCH_3$, $(CH_2)_3COOCH_2CH_3$,

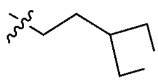,

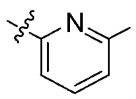 N , 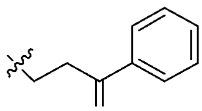, 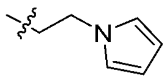, 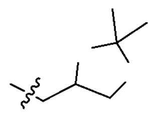, 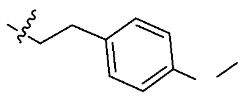,

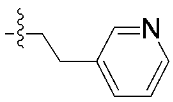, 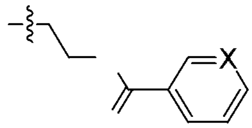,

cyclobutyl, or isobutyl.
In some embodiments, $R^4$ is selected from halogen or CN.
In some embodiments, $R^4$ is selected from F, Cl, or CN.
In some embodiments, $R^4$ is CN.
In some embodiments, $R^5$ is selected from halogen, $C_1$-$C_6$ alkyl, $C_1$-$C_6$ haloalkyl, -COO$C_1$-$C_6$ alkyl, - S-$C_1$-$C_6$ alkyl, or -O-$C_1$-$C_6$ alkyl.
In some embodiments, $R^5$ is selected from $C_1$-$C_6$ haloalkyl, -S-$C_1$-$C_6$ alkyl, -O-$C_1$-$C_6$ alkyl, -Se-$C_1$-$C_6$ alkyl, -$S(O)_2$-$C_1$-$C_6$ alkyl, or -S(O)-$C_1$-$C_6$ alkyl.
In some embodiments, $R^5$ is selected from $C_1$-$C_6$ haloalkyl or -S-$C_1$-$C_6$ alkyl.
In some embodiments, $R^5$ is selected from $CF_3$, -S-$CH_3$, -O-$CH_3$, -Se-$CH_3$, -$S(O)_2$-$CH_3$, or -S(O)-$CH_3$.
In some embodiments, $R^5$ is selected from $CF_3$ or -S-$CH_3$.
In some embodiments, $R^6$ is selected from H or halogen.
In some embodiments, $R^6$ is selected from H or F.
In some embodiments, $R^7$ is H.
In some embodiments, the compound of formula (I) or the stereoisomer thereof or the pharmaceutically acceptable salt thereof is selected from a compound of formula (I-1) or a stereoisomer thereof or a pharmaceutically acceptable salt thereof:

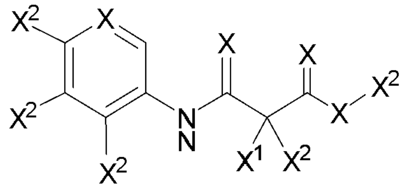

(I-1)

wherein $R^1$, $R^2$, $R^3$, $R^4$, $R^5$, $R^6$, and X are as defined for formula (I) above.

In some embodiments, the compound of formula (I) or the stereoisomer thereof or the pharmaceutically acceptable salt thereof is selected from a compound below or a stereoisomer thereof or a pharmaceutically acceptable salt thereof:

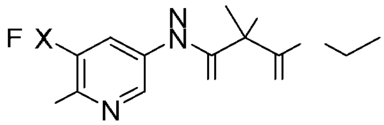
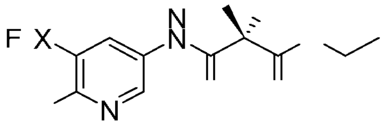
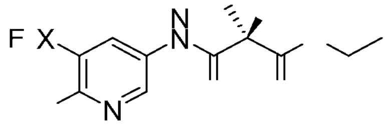

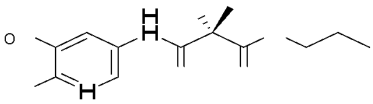
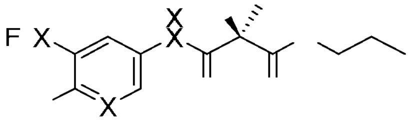
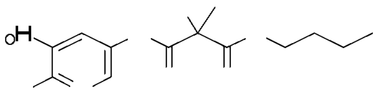

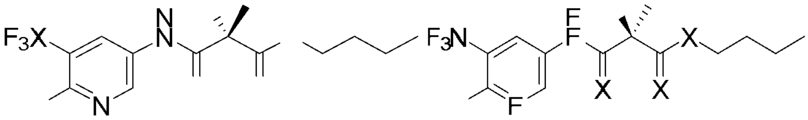
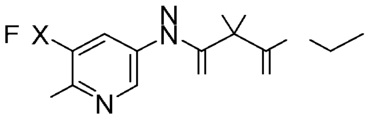

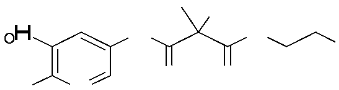
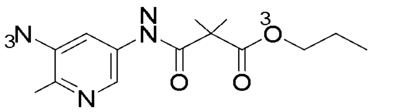
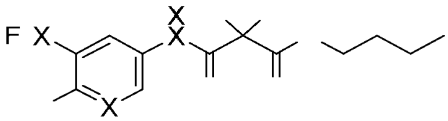

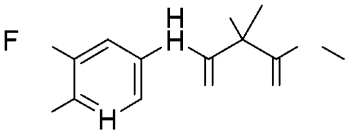
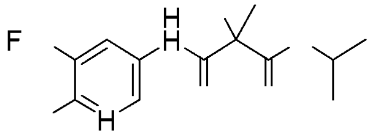
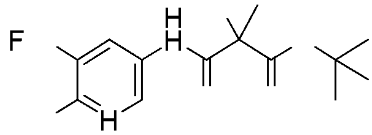

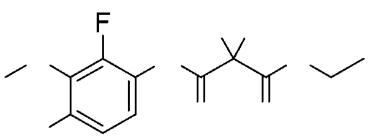
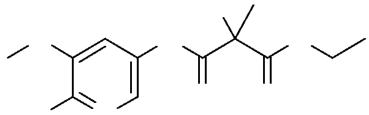
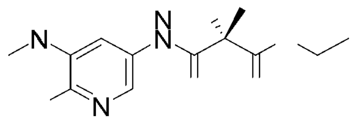

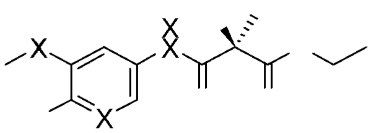
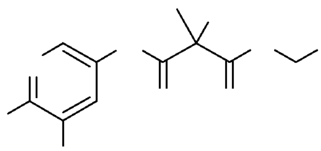
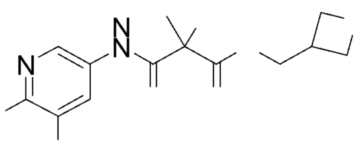

3

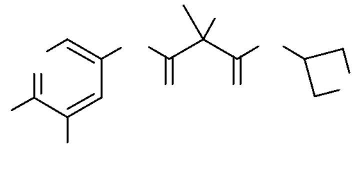
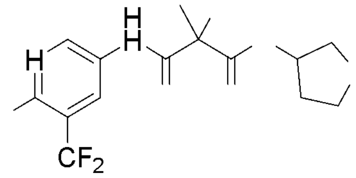
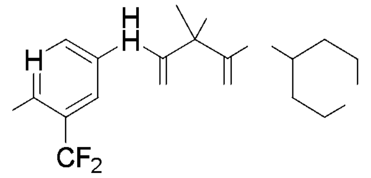

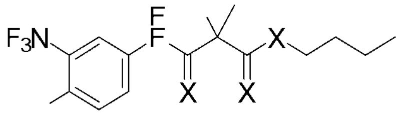
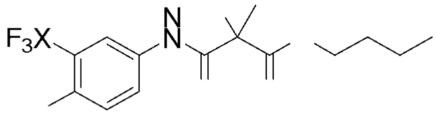
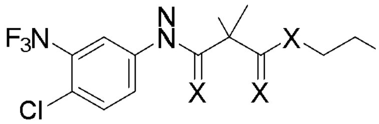

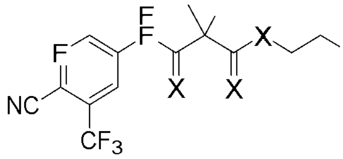
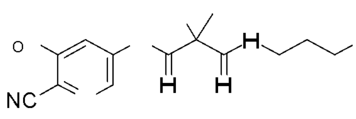
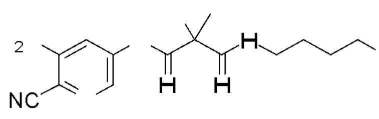

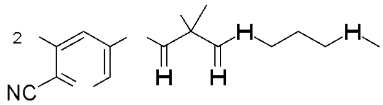
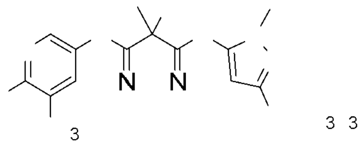
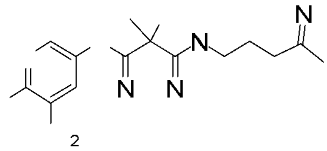

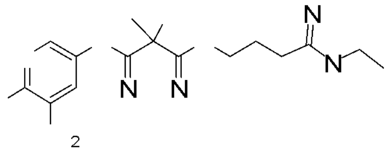
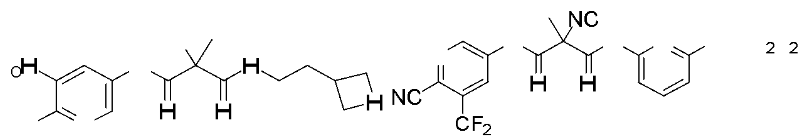

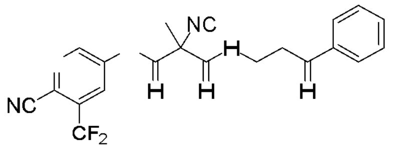
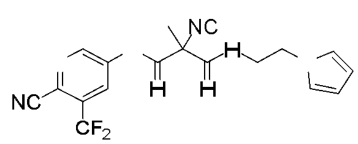
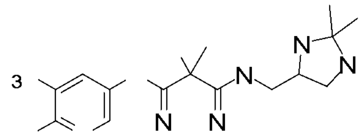

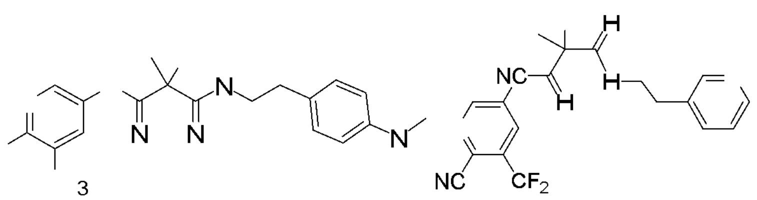
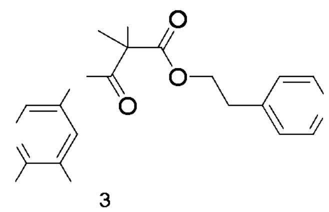

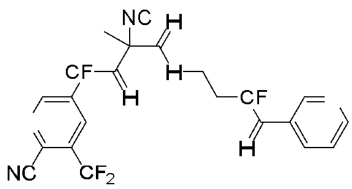
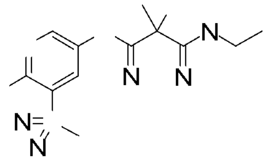
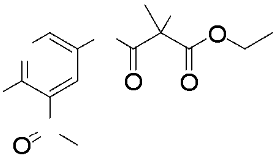

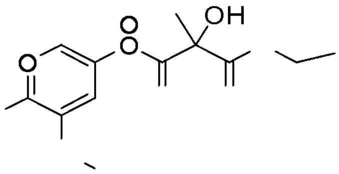 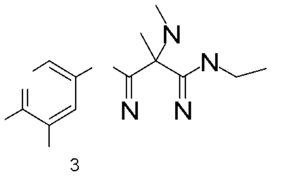 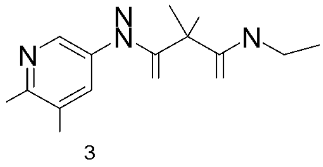

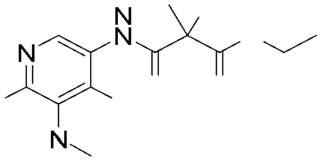 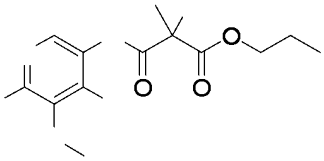 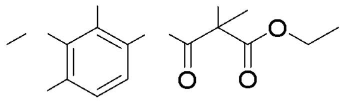

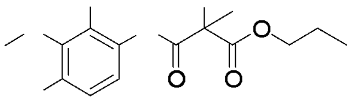 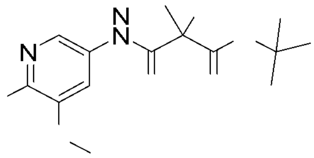 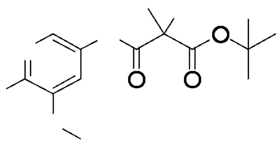

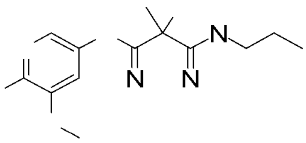 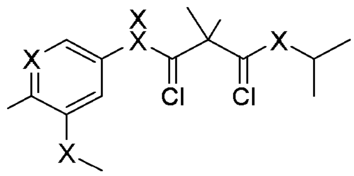 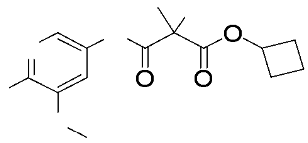

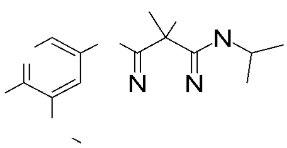 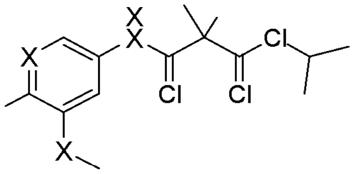 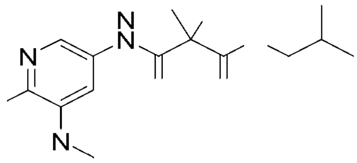

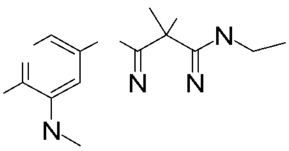 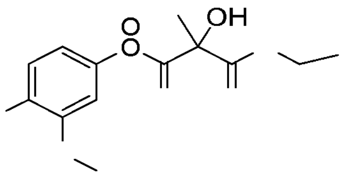 or 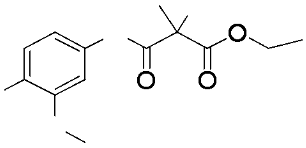 .

In another aspect, the present disclosure provides a pharmaceutical composition, comprising the compound of formula (I) or the stereoisomer thereof or the pharmaceutically acceptable salt thereof of the present disclosure and a pharmaceutically acceptable excipient.

In another aspect, the present disclosure provides a method for treating an androgen receptor-mediated disease in a mammal, comprising administering to a mammal, preferably a human, in need of the treatment a therapeutically effective amount of the compound of formula (I) or the stereoisomer thereof or the pharmaceutically acceptable salt thereof, or the pharmaceutical composition thereof.

In another aspect, the present disclosure provides use of the compound of formula (I) or the stereoisomer thereof or the pharmaceutically acceptable salt thereof or the pharmaceutical composition thereof in preparing a medicament for preventing or treating an androgen receptor-mediated disease.

In another aspect, the present disclosure provides use of the compound of formula (I) or the stereoisomer thereof or the pharmaceutically acceptable salt thereof, or the pharmaceutical composition thereof in preventing or treating an androgen receptor-mediated disease.

In another aspect, the present disclosure provides the compound of formula (I) or the stereoisomer thereof or the pharmaceutically acceptable salt thereof, or the pharmaceutical composition thereof for preventing or treating an androgen receptor-mediated disease.

In some embodiments, the androgen receptor-mediated disease is selected from androgenetic alopecia. Any

embodiment of any aspect of the present disclosure may be combined with other embodiments in the case of no contradictions. In addition, in any embodiment of any aspect of the present disclosure, any technical feature is applicable to the technical feature in other embodiments in the case of no contradictions.

Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be interpreted according to its common meaning in the field. When referring to a trade name, it is intended to refer to its corresponding commercial product or its active ingredient.

Herein, "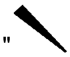" denotes a linking site.

The illustration method for the racemic or enantiomerically pure compounds herein is from Maehr, J. Chem. Ed. 1985, 62:114-120. Unless otherwise stated, the absolute configuration of a stereogenic center is represented by a wedged bond and a wedged dashed bond (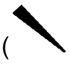 and 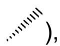), and the relative configuration of a stereogenic center (e.g., *cis-* or *trans-*configuration of alicyclic compounds) is represented by a black solid bond and a dashed bond (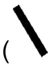 and 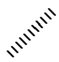).

The term "tautomer" refers to functional isomers resulting from the rapid movement of an atom in a molecule between two positions. The compounds of the present disclosure may exhibit tautomerism. Tautomeric compounds may have two or more interconvertible forms. Tautomers are generally present in an equilibrated form. Trying to separate a single tautomer usually leads to a mixture, the physicochemical properties of which are consistent with the mixture of the compound. The equilibrium depends on the chemical properties of the molecule. For example, in many aliphatic aldehydes and ketones, such as acetaldehyde, the keto form predominates; whereas in phenol, the enol form predominates. In the present disclosure, all tautomeric forms of the compounds are included.

The term "stereoisomer" refers to isomers resulting from different spatial arrangements of atoms in a molecule, including *cis-trans* isomers, enantiomers, and diastereoisomers.

The compound of the present disclosure may have an asymmetric atom such as a carbon atom, a sulfur atom, a nitrogen atom, and a phosphorus atom, or an asymmetric double bond, and thus the compound of the present disclosure may be present in the form of a particular geometric isomer or stereoisomer. The form of a particular geometric isomer or stereoisomer may be *cis* and *trans* isomers, *E* and *Z* geometric isomers, (-)- and (+)-enantiomers, (*R*)- and (*S*)-enantiomers, diastereoisomers, (*D*)-isomers, (*L*)-isomers, and racemic mixtures or other mixtures thereof, such as an enantiomer or diastereoisomer enriched mixture, and all of the above isomers, as well as mixtures thereof, are encompassed within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof involved in the substituents are also encompassed within the definition scope of the compound of the present disclosure. The compound containing an asymmetric atom of the present disclosure can be separated in an optically active pure form or in a racemic form. The optically active pure form can be obtained by resolving a racemic mixture or by synthesis using chiral starting materials or chiral reagents.

The term "substituted" means that any one or more hydrogen atoms on a specific atom are replaced by substituents, as long as the valence of the specific atom is normal and the compound resulting from the substitution is stable. When the substituent is oxo (i.e., =O), it means that two hydrogen atoms are replaced, and oxo will not be present on an aromatic group.

The term "optional" or "optionally" means that the subsequently described event or circumstance may or may not occur. The description includes instances where the event or circumstance occurs and instances where the event or circumstance does not. For example, ethyl being "optionally" substituted with halogen means that the ethyl may be unsubstituted ($CH_2CH_3$), monosubstituted ($CH_2CH_2F$, $CH_2CH_2Cl$, or the like), polysubstituted ($CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). Those skilled in the art will appreciate that for any group containing one or more substituents, no substitution or substituting pattern that is spatially impossible and/or cannot be synthesized will be introduced.

When any variable (e.g., $R^a$ or $R^b$) occurs one or more times in the constitution or structure of a compound, the variable is independently defined in each case. For example, if a group is substituted with 2 $R^b$, the definition of each $R^b$ is independent.

$C_m$-$C_n$ used herein means that the portion has an integer number of carbon atoms in the range of m-n. For example, the "$C_1$-$C_{10}$" means that the group may have 1 carbon atom, 2 carbon atoms, 3 carbon atoms, 4 carbon atoms, 5

carbon atoms, 6 carbon atoms, 7 carbon atoms, 8 carbon atoms, 9 carbon atoms, or 10 carbon atoms.

The term "alkyl" refers to a hydrocarbyl group with a general formula of $C_nH_{2n+1}$. The alkyl may be linear or branched. The term "$C_1$-$C_{10}$ alkyl" may be interpreted as a linear or branched saturated hydrocarbon group having 1, 2, 3, 4, 5, 6, 7, 8, 9, or 10 carbon atoms. Specific examples of the alkyl include, but are not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, and the like. The term "$C_1$-$C_6$ alkyl" may be interpreted as an alkyl group having 1, 2, 3, 4, 5, or 6 carbon atoms. Specific examples include, but are not limited to, methyl, ethyl, *n*-propyl, isopropyl, *n*-butyl, isobutyl, *sec*-butyl, *tert*-butyl, *n*-pentyl, 1-methylbutyl, 2-methylbutyl, 3-methylbutyl, neopentyl, hexyl, 2-methylpentyl, and the like. The term "$C_1$-$C_3$ alkyl" may be interpreted as a linear or branched saturated alkyl group having 1, 2, or 3 carbon atoms. The "$C_1$-$C_{10}$ alkyl" may include the ranges of "$C_1$-$C_6$ alkyl", "$C_1$-$C_3$ alkyl", and the like, and the "$C_1$-$C_6$ alkyl" may further include "$C_1$-$C_3$ alkyl".

The term "deuterated alkyl" refers to an alkyl in which a hydrogen is substituted with deuterium, including monodeuterated alkyl and polydeuterated alkyl. For example, the term "$C_1$-$C_6$ deuterated alkyl" refers to a $C_1$-$C_6$ alkyl as defined above substituted with one or more deuterium, including but not limited to $CD_3$, $CH_2CD_3$, and the like.

The term "haloalkyl" includes monohaloalkyl and polyhaloalkyl. For example, the term "$C_1$-$C_6$ haloalkyl" refers to $C_1$-$C_6$ alkyl as defined above substituted with one or more halogens, including but not limited to trifluoromethyl, 2,2,2-trifluoroethyl, 4-chlorobutyl, 3-bromopropyl, trichloromethyl, pentafluoroethyl, pentachloroethyl, and the like. The term "cycloalkyl" refers to a fully saturated carbon ring that is present in the form of a monocyclic ring, a fused ring, a bridged ring, a spiro ring, or the like. Unless otherwise specified, the carbon ring is generally a 3- to 10-membered ring. The term "$C_3$-$C_{10}$ cycloalkyl" may be interpreted as a saturated monocyclic, fused, spiro, or bridged ring having 3, 4, 5, 6, 7, 8, 9, or 10 carbon atoms. Specific examples of the cycloalkyl include, but are not limited to, cyclopropyl, cyclobutyl, cyclopentyl, cyclohexyl, cycloheptyl, cyclooctyl, cyclononyl, cyclodecyl, norbornyl (bicyclo[2.2.1]heptyl), bicyclo[2.2.2]octyl, adamantyl, spiro[4.5]decyl, and the like. The term "$C_3$-$C_{10}$ cycloalkyl" may include "$C_3$-$C_6$ cycloalkyl", and the term "$C_3$-$C_6$ cycloalkyl" may be interpreted as a saturated monocyclic or bicyclic hydrocarbon ring having 3, 4, 5, or 6 carbon atoms. Specific examples include, but are not limited to, cyclopropyl, cyclobutyl, cyclopentyl, cyclohexyl, and the like.

The term "heterocyclyl" refers to a fully saturated or partially saturated (non-aromatic heteroaromatic group on the whole) monocyclic, fused cyclic, spiro cyclic, or bridged cyclic group, and ring atoms of the group include 1, 2, 3, 4, or 5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), $-S(=O)_2-$, -S(=O)-, $-P(=O)_2-$, -P(=O)-, -NH-, -S(=O)(=NH)-, -C(=O)NH-, -NHC(=O)NH-, and the like. The term "3- to 10-membered heterocyclyl" refers to a heterocyclyl group with 3, 4, 5, 6, 7, 8, 9, or 10 ring atoms, and ring atoms of the group include 1, 2, 3, 4, or 5 heteroatoms or heteroatom groups independently selected from those described above. The "3- to 10-membered heterocyclyl" includes "4- to 7-membered heterocyclyl", wherein specific examples of 4-membered heterocyclyl include, but are not limited to, azetidinyl, thietanyl, and oxetanyl; specific examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, and 2,5-dihydro-1*H*-pyrrolyl; specific examples of 6-membered heterocyclyl include, but are not limited to, tetrahydropyranyl, piperidyl, morpholinyl, dithianyl, thiomorpholinyl, piperazinyl, trithianyl, tetrahydropyridinyl, and 4*H*-[1,3,4]thiadiazinyl; specific examples of 7-membered heterocyclyl include, but are not limited to, diazepanyl. The heterocyclyl may also be a bicyclic group, wherein specific examples of 5,5-membered bicyclic groups include, but are not limited to, hexahydrocyclopenta[c]pyrrol-2(1*H*)-yl; specific examples of 5,6-membered bicyclic groups include, but are not limited to, hexahydropyrrolo[1,2-*a*]pyrazin-2(1*H*)-yl, 5,6,7,8-tetrahydro-[1,2,4]triazolo[4,3-*a*]pyrazinyl, and 5,6,7,8-tetrahydroimidazo[1,5-*a*]pyrazinyl. Optionally, the heterocyclyl may be a benzo-fused ring group of the 4- to 7-membered heterocyclyl described above. Specific examples include, but are not limited to, dihydroisoquinolyl and the like. The "4- to 10-membered heterocyclyl" may include the ranges of "5- to 10-membered heterocyclyl", "4- to 7-membered heterocyclyl", "5- to 6-membered heterocyclyl", "6- to 8-membered heterocyclyl", "4- to 10-membered heterocycloalkyl", "5- to 10-membered heterocycloalkyl", "4- to 7-membered heterocycloalkyl", "5- to 6-membered heterocycloalkyl", "6- to 8-membered heterocycloalkyl", and the like, and the "4- to 7-membered heterocyclyl" may further include the ranges of "4- to 6-membered heterocyclyl", "5- to 6-membered heterocyclyl", "4- to 7-membered heterocycloalkyl", "4- to 6-membered heterocycloalkyl", "5- to 6-membered heterocycloalkyl", and the like. Although some bicyclic heterocyclyl groups herein comprise, in part, one benzene ring or one heteroaromatic ring, the heterocyclyl is still non-aromatic on the whole.

The term "aryl" refers to an aromatic all-carbon monocyclic or fused polycyclic group with a conjugated $\pi$-electron system. The aryl may have 6-20 carbon atoms, 6-14 carbon atoms, or 6-12 carbon atoms. The term "$C_6$-$C_{20}$ aryl" may be interpreted as an aryl group having 6 to 20 carbon atoms, particularly a ring having 6 carbon atoms ("$C_6$ aryl"), such as phenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthryl. The term "$C_6$-$C_{10}$ aryl" may be interpreted as an aryl group having 6 to 10 carbon atoms, particularly a ring having 6 carbon atoms ("$C_6$ aryl"), such as phenyl, or a ring having 9 carbon

atoms ("$C_9$ aryl"), such as indanyl or indenyl, or a ring having 10 carbon atoms ("$C_{10}$ aryl"), such as tetrahydronaphthyl, dihydronaphthyl, or naphthyl. The term "$C_6$-$C_{20}$ aryl" may include "$C_6$-$C_{10}$ aryl".

The term "heteroaryl" refers to an aromatic cyclic group having an aromatic monocyclic or fused polycyclic system, which contains at least one ring atom selected from N, O, and S, with the remaining ring atoms being C. The term "5- to 10-membered heteroaryl" may be interpreted as including an aromatic monocyclic or bicyclic ring system, which has 5, 6, 7, 8, 9, or 10 ring atoms, particularly 5, 6, 9, or 10 ring atoms, and comprises 1, 2, 3, 4, or 5, preferably 1, 2, or 3, heteroatoms independently selected from N, O, and S. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, and the like, and benzo derivatives thereof, such as benzofuranyl, benzothienyl, benzothiazolyl, benzoxazolyl, benzoisoxazolyl, benzimidazolyl, benzotriazolyl, indazolyl, indolyl, isoindolyl, and the like; and pyridyl, pyridazinyl, pyrimidinyl, pyrazinyl, triazinyl, and the like, and benzo derivatives thereof, such as quinolyl, quinazolinyl, isoquinolyl, and the like; and azocinyl, indolizinyl, purinyl, and the like, and benzo derivatives thereof; and cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. The term "5- to 6-membered heteroaryl" refers to an aromatic ring system, which has 5 or 6 ring atoms and comprises 1, 2, or 3, preferably 1-2, heteroatoms independently selected from N, O, and S. The term "halo" or "halogen" refers to fluorine, chlorine, bromine, or iodine.

The term "hydroxy" refers to the -OH group.

The term "cyano" refers to the -CN group.

The term "amino" refers to the $-NH_2$ group.

The term "therapeutically effective amount" means
an amount of the compound of the present disclosure for (i) treating a specific disease, condition, or disorder; (ii) alleviating, ameliorating, or eliminating one or more symptoms of a specific disease, condition, or disorder; or (iii) delaying the onset of the one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the content of the present disclosure.

The term "treat" or "treatment" means administering the compound or formulation described herein to ameliorate or eliminate a disease or one or more symptoms associated with the disease, and includes:

(i) inhibiting a disease or a disease state, i.e., arresting its development; and
(ii) alleviating a disease or a disease state, i.e., causing its regression.

The term "prevent" or "prevention" means administering the compound or formulation described herein to prevent a disease or one or more symptoms associated with the disease, and includes: preventing the development of the disease or disease state in a mammal, particularly when such a mammal is predisposed to the disease state but has not yet been diagnosed with it.

In the present application, examples of the term "mammal" include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like. The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acid addition or base addition salts, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The word "comprise" and variations thereof such as "comprises" or "comprising" may be interpreted in an open and non-exclusive sense, i.e., "including but not limited to".

The present disclosure also includes isotopically labeled compounds of the present disclosure that are identical to those documented herein but have one or more atoms replaced by an atom having an atomic mass or mass number different from the atomic mass or mass number usually found in nature. Examples of isotopes that can be incorporated into

the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Certain isotopically labeled compounds of the present disclosure (e.g., those labeled with $^{3}H$ and $^{14}C$) can be used to analyze compounds and/or substrate tissue distribution. Tritiation (i.e., $^{3}H$) and carbon-14 (i.e., $^{14}C$) isotopes are particularly preferred for their ease of preparation and detectability. Positron emitting isotopes, such as $^{13}O$, $^{13}N$, $^{11}C$, and $^{18}F$, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical composition of the present disclosure can be prepared by combining the compound of the present disclosure with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present disclosure include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present disclosure can be manufactured by methods well known in the art, such as conventional methods of mixing, dissolving, granulating, emulsifying, freeze-drying, and the like.

In some embodiments, the pharmaceutical composition is in an oral form. For oral administration, the pharmaceutical composition can be formulated by mixing the active compound with pharmaceutically acceptable excipients well known in the art. These excipients enable the compounds of the present disclosure to be formulated into tablets, pills, lozenges, dragees, capsules, liquids, gels, slurries, suspensions, and the like, for oral administration to patients.

A solid oral composition can be prepared by conventional mixing, filling, or tableting. For example, it can be obtained by the following method: mixing the active compound with a solid excipient, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, flavoring agents, and the like.

The pharmaceutical composition may also be suitable for parenteral administration, such as a sterile solution, suspension, or lyophilized product in a suitable unit dosage form.

In all of the administration methods for the compound of general formula (I) described herein, the daily dose is from 0.01 mg/kg to 500 mg/kg of body weight, given in individual or separated doses.

## DETAILED DESCRIPTION

The present disclosure is described in detail below by way of examples, which, however, are not intended to disadvantageously limit the scope of the present disclosure in any way. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially available and can be used without further purification.

Unless otherwise stated, the ratios expressed for mixed solvents are volume mixing ratios.

Unless otherwise stated, % refers to wt%.

Compounds are named either manually or by ChemDraw® software, and supplier's product names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS). The "$IC_{50}$" refers to the half inhibitory concentration, which is the concentration at which half of the maximal inhibitory effect is achieved.

The following eluents may be mixed eluents formed by two or more solvents in a certain volume ratio. For example, "0-10% methanol/dichloromethane" represents that the volume ratio of methanol to dichloromethane in the mixed eluent is 0:100-10:90 during the gradient elution, or "ethyl acetate/petroleum ether = 5/1" represents that the volume ratio of ethyl acetate to petroleum ether in the mixed eluent is 5:1 during the gradient elution.

Abbreviations:

TEA: triethylamine; THF: tetrahydrofuran; n-propanol: n-propanol; n-butanol or n-BuOH: n-butanol; t-BuOH: tert-butanol; ACN: acetonitrile; TMAF: tetramethylammonium fluoride; $TMSCF_3$: (trifluoromethyl)trimethylsilane; AcOH: acetic acid; MeOH: methanol; i-PrOH: isopropanol; EA: ethyl acetate; Cyclohexane: cyclohexane; DMF: N,N-dimethylformamide; NaSMe: sodium thiomethoxide; NBS: N-bromosuccinimide; $SOCl_2$: thionyl chloride; $PPh_3$: triphenylphosphine; DIAD: diisopropyl azodicarboxylate; DEAD: diethyl azodicarboxylate; HATU: 2-(7-azabenzotriazole)-N,N,N',N'-tetramethyluronium hexafluorophosphate; DCM: dichloromethane; m-CPBA: m-chloroperoxybenzoic acid; DIEA: N,N-diisopropylethylamine; $LiOH{\cdot}H_2O$: lithium hydroxide monohydrate; EtOH: ethanol; DCC: dicyclohexylcarbodiimide; DMAP: 4-dimethylaminopyridine; TBAF: tetrabutylammonium fluoride.

**Example 1: Ethyl 3-[[6-cyano-5-(trifluoromethyl)-pyridin-3-yl]amino]-2-hydroxy-2-methyl-3-oxopropanoate (compound 001)**

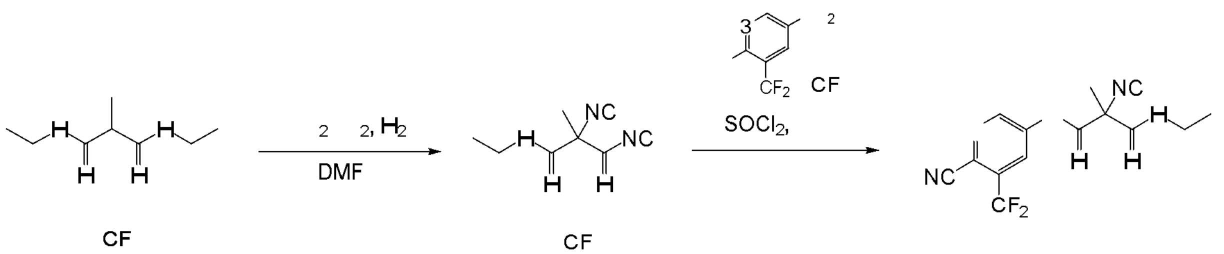


**Step 1: Synthesis of 3-ethoxy-2-hydroxy-2-methyl-3-oxopropanoic acid**

Intermediate **1-1** (1 g, 5.74 mmol) and cesium carbonate (3.73 g, 11.48 mmol) were added to N,N-dimethylformamide (20 mL), and the reaction mixture was stirred in air at room temperature for 18 h. After LCMS indicated the completion of the reaction, the reaction mixture was diluted with 150 mL of water. The mixture was extracted with ethyl acetate (50 mL × 3), and the organic phases were combined, washed with water (40 mL × 2) and saturated brine (40 mL), dried over anhydrous sodium sulfate, and filtered. The filtrate was concentrated at reduced pressure, and the residue was purified by column chromatography (silica, ethyl acetate/petroleum ether = 5/1) to give intermediate compound **1-2** (0.6 g). MS (ESI): m/z= 161.10 $[M-H]^-$.

**Step 2: Synthesis of ethyl 3-[[6-cyano-5-(trifluoromethyl)-pyridin-3-yl]amino]-2-hydroxy-2-methyl-3-oxopropanoate**

Intermediate **1-2** (1.56 g, 9.62 mmol) was added to tetrahydrofuran (10 mL), before $SOCl_2$ (953.68 mg, 8.02 mmol, 582.22 μL) was added in an ice-water bath. The mixture was stirred at this temperature for 2 h. Triethylamine (973.39 mg, 9.62 mmol, 1.34 mL) was added, and the mixture was stirred for 20 min. Intermediate **1-3** (1 g, 5.34 mmol) was added, and the mixture was stirred at room temperature for 18 h. After LCMS indicated the completion of the reaction, the reaction mixture was concentrated, and the residue was purified by RP-FLASH [column: C18 column, 120 g, 20-35 μm silica, 100 Å; a mixture of water (containing 0.1% formic acid) and acetonitrile with decreasing polarity was used as the eluent, acetonitrile gradient: 45%-55%; flow rate: 65 mL/min; elution time: 15 min] to give compound **001** (1 g). MS (ESI): m/z= 332.10 $[M+H]^+$.

$^1H$ NMR (400 MHz, DMSO-$d_6$) δ 10.99 (s, 1H), 9.40 - 9.34 (m, 1H), 8.89 - 8.83 (m, 1H), 7.08 (s, 1H), 4.26 - 4.09 (m, 2H), 1.58 (s, 3H), 1.26 - 1.15 (m, 3H).

**Examples 2 & 3: Ethyl (2S)-3-[[6-cyano-5-(trifluoromethyl)pyridin-3-yl]amino]-2-hydroxy-2-methyl-3-oxopropanoate and ethyl (2R)-3-[[6-cyano-5-(trifluoromethyl)pyridin-3-yl]amino]-2-hydroxy-2-methyl-3-oxopropanoate (compounds 002 & 003)**

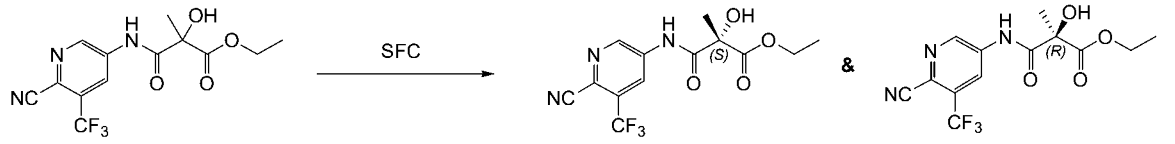


001

&

002 &003

Compound **001** (1 g) was separated by SFC [instrument: WATERS 150 preparative SFC (SFC-26); column: Chiral Pak AD, 250 × 30 mm, I.D., 10 μm; mobile phase: carbon dioxide as phase A, methanol as phase B; gradient (B%): 20%; flow rate: 150 mL/min; back pressure: 100 bar; column temperature: 38 °C; detection wavelength: 220 nm; cycle time: 8 min] and purified to give compound **002** (484 mg) and compound **003** (444 mg).

**Compound 002:**

SFC retention time: 2.081 min.

MS (ESI): m/z = 332.1 $[M+H]^+$.

$^1$H NMR (400 MHz, DMSO-$d_6$) δ 10.93 (s, 1H), 9.40 - 9.34 (m, 1H), 8.89 - 8.83 (m, 1H), 7.08 (s, 1H), 4.26 - 4.09 (m, 2H), 1.58 (s, 3H), 1.26 - 1.15 (m, 3H).

**Compound 003:**

SFC retention time: 2.484 min.

MS (ESI): m/z = 332.1 $[M+H]^+$.

$^1$H NMR (400 MHz, DMSO-$d_6$) δ 10.95 (s, 1H), 9.37 (d, *J* = 2.2 Hz, 1H), 8.85 (d, *J* = 2.3 Hz, 1H), 7.09 (s, 1H), 4.26 - 4.09 (m, 2H), 1.59 (s, 3H), 1.26 - 1.15 (m, 3H).

**Examples 4 & 5: Propyl (2S)-3-[[6-cyano-5-(trifluoromethyl)-pyridin-3-yl]amino]-2-hydroxy-2-methyl-3-oxopropanoate and propyl (2R)-3-[[6-cyano-5-(trifluoromethyl)-pyridin-3-yl]amino]-2-hydroxy-2-methyl-3-oxopropanoate (compounds 004 & 005)**

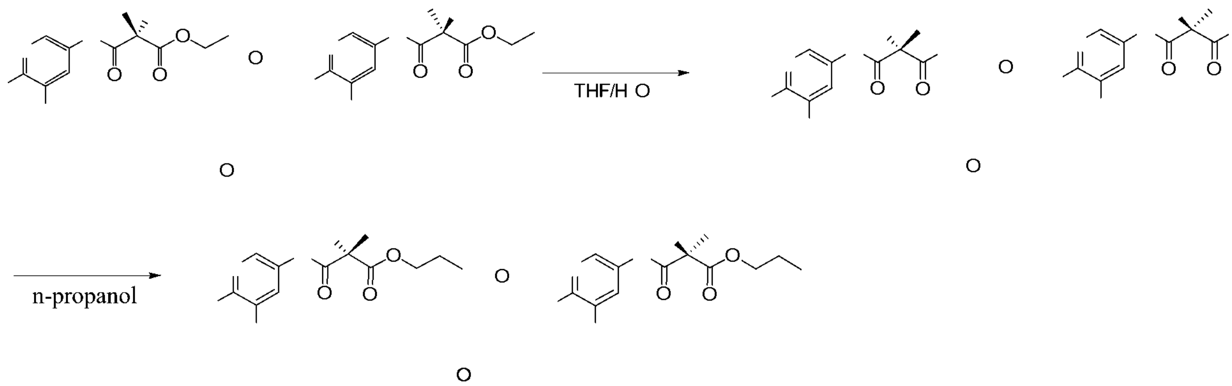


**Step 1: Synthesis of (2S)-3-[[6-cyano-5-(trifluoromethyl)-pyridin-3-yl]amino]-2-hydroxy-2-methyl-3-oxopropanoic acid and (2R)-3-[[6-cyano-5-(trifluoromethyl)-pyridin-3-yl]amino]-2-hydroxy-2-methyl-3-oxopropanoic acid**

Compound **002 (or 003)** (100 mg, 301.89 μmol) and lithium hydroxide (36.15 mg, 1.51 mmol) were added to tetrahydrofuran (2 mL) and water (3 mL). The reaction mixture was stirred at room temperature for 2 h. After LCMS indicated the completion of the reaction, the reaction mixture was poured into a 0.1 M aqueous HCl solution (30 mL), and the resulting mixture was extracted with dichloromethane (10 mL × 3). The organic phases were combined, washed with saturated brine (50 mL), dried over anhydrous sulfuric acid, and filtered. The filtrate was concentrated to give intermediate compound **4-1 (or 5-1)** (80 mg). MS (ESI): m/z = 304.00 $[M+H]^+$.
$^1$H NMR (400 MHz, DMSO-$d_6$) δ 9.32 (s, 1H), 8.86 (s, 1H), 1.42 (s, 3H).

**Step 2: Synthesis of propyl (2S)-3-[[6-cyano-5-(trifluoromethyl)-pyridin-3-yl]amino]-2-hydroxy-2-methyl-3-oxo-propanoate and propyl (2R)-3-[[6-cyano-5-(trifluoromethyl)-pyridin-3-yl]amino]-2-hydroxy-2-methyl-3-oxopropanoate (compounds 004 & 005)**

Intermediate **4-1 (or 5-1)** (50 mg, 164.91 μmol) was dissolved in n-propanol (1 mL), and concentrated sulfuric acid (258.79 mg, 2.64 mmol, 140.65 μL) was added in an ice bath. After the addition, the mixture was stirred at room temperature for 15 h. After LCMS indicated the completion of the reaction, the reaction mixture was concentrated, and the residue was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-70%, elution time: 12 min] to give target compound **004 (or 005)** (20 mg).
Compound 004 was prepared from compound 002 and compound 005 was prepared from compound 003 by using the preparation method described above.

**Compound 004:**

MS (ESI): m/z = 346.10 $[M+H]^+$.
$^1H$ NMR (400 MHz, DMSO-$d_6$) δ 10.94 (s, 1H), 9.36 (d, $J$ = 2.3 Hz, 1H), 8.84 (d, $J$ = 2.2 Hz, 1H), 7.08 (s, 1H), 4.12 - 4.02 (m, 2H), 1.64 - 1.51 (m, 5H), 0.89 - 0.80 (m, 3H).

**Compound 005:**

MS (ESI): m/z = 346.10 $[M+H]^+$.
$^1H$ NMR (400 MHz, DMSO-$d_6$) δ 10.94 (s, 1H), 9.36 (d, $J$ = 2.2 Hz, 1H), 8.84 (d, $J$ = 2.3 Hz, 1H), 7.08 (s, 1H), 4.12 - 4.02 (m, 2H), 1.66 - 1.50 (m, 5H), 0.89 - 0.80 (m, 3H).

**Examples 6 & 7: Synthesis of butyl (2S)-3-[[6-cyano-5-(trifluoromethyl)pyridin-3-yl]amino]-2-hydroxy-2-methyl-3-oxopropanoate and butyl (2R)-3-[[6-cyano-5-(trifluoromethyl)pyridin-3-yl]amino]-2-hydroxy-2-methyl-3-oxopropanoate (compounds 006 & 007)**

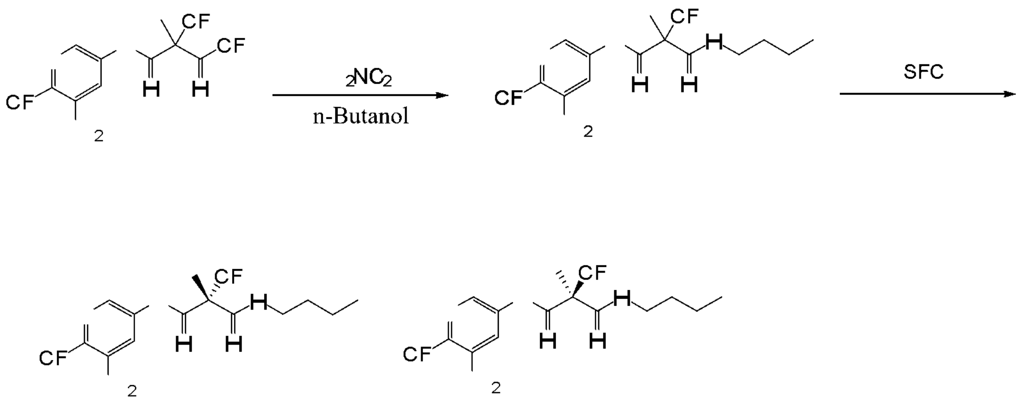


**Step 1: Synthesis of butyl 3-[[6-cyano-5-(trifluoromethyl)pyridin-3-yl]amino]-2-hydroxy-2-methyl-3-oxopropanoate**

Compound 9-1 (40 mg, 131.9 μmol) was added to n-butanol (1 mL). Concentrated sulfuric acid (25.88 mg, 263.86 μmol, 14.06 μL) was added at room temperature, and the reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of purified water and acetonitrile with decreasing polarity was used as the eluent, acetonitrile gradient: 50%-69%, elution time: 9.2 min] to give target compound **6-1** (11.2 mg).
MS (ESI): m/z =360.10 $[M+H]^+$.
$^1H$ NMR (400 MHz, DMSO-$d_6$) δ 9.33 (d, $J$ = 2.2 Hz, 1H), 8.83 (d, $J$ = 2.3 Hz, 1H), 4.16 - 4.05 (m, 2H), 1.60 - 1.48 (m, 5H), 1.36 - 1.21 (m, 2H), 0.87 - 0.78 (m, 3H).

**Step 2: Synthesis of butyl (2S)-3-[[6-cyano-5-(trifluoromethyl)pyridin-3-yl]amino]-2-hydroxy-2-methyl-3-oxopropanoate and butyl (2R)-3-[[6-cyano-5-(trifluoromethyl)pyridin-3-yl]amino]-2-hydroxy-2-methyl-3-oxopropanoate (006 & 007)**

Intermediate **6-1** was prepared on a greater scale according to the method described in step 1. Intermediate **6-1** (1 g) was separated by SFC [instrument: WATERS 150 preparative SFC (SFC-26); column: Chiral Pak AD, 250 × 30 mm, I.D., 10 μm; mobile phase: carbon dioxide as phase A, ethanol (containing 0.1% $NH_3H_2O$) as phase B; gradient (B%): 15%; flow rate: 150 mL/min; back pressure: 100 bar; column temperature: 38 °C; detection wavelength: 220 nm; cycle time: 5 min] and purified to give compound **006** (464 mg) and compound **007** (468 mg).

**Compound 006:**

**SFC retention time: 1.552 min.**

MS (ESI): m/z = 360.10 $[M+H]^+$.
$^1$H NMR (400 MHz, DMSO-$d_6$) δ 10.95 (s, 1H), 9.37 (d, *J* = 2.2 Hz, 1H), 8.84 (d, *J* = 2.2 Hz, 1H), 7.07 (s, 1H), 4.16 - 4.05 (m, 2H), 1.61 - 1.48 (m, 5H), 1.36 - 1.21 (m, 2H), 0.87 - 0.78 (m, 3H).

**Compound 007:**

**SFC retention time: 1.903 min.**

MS (ESI): m/z = 360.10 $[M+H]^+$.
$^1$H NMR (400 MHz, DMSO-$d_6$) δ 10.95 (s, 1H), 9.36 (d, *J* = 2.3 Hz, 1H), 8.84 (d, *J* = 2.3 Hz, 1H), 7.07 (s, 1H), 4.16 - 4.05 (m, 2H), 1.61 - 1.48 (m, 5H), 1.36 - 1.22 (m, 2H), 0.87 - 0.78 (m, 3H).

**Example 8: Ethyl 2-[[6-cyano-5-(trifluoromethyl)-pyridin-3-yl]carbamoyl]-3,3,3-trifluoro-2-hydroxypropanoate (compound 008)**

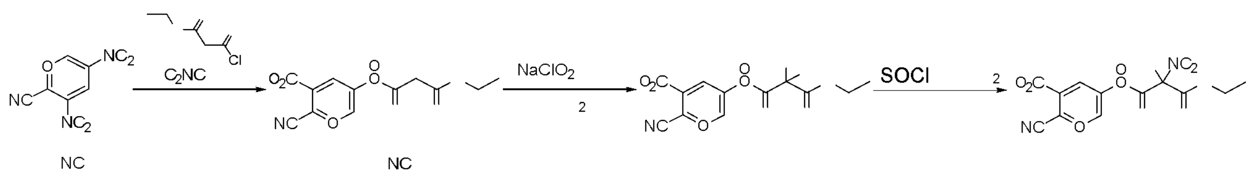


**Step 1: Synthesis of ethyl 3-[[6-cyano-5-(trifluoromethyl)-pyridin-3-yl]amino]-3-oxopropanoate**

The starting materials **1-3** (10 g, 53.44 mmol) and potassium phosphate (16.91 g, 80.16 mmol) were added to tetrahydrofuran (15 mL). Ethyl 3-chloro-3-oxopropanoate (12.07 g, 80.16 mmol) was added to the reaction mixture in an ice-water bath. The reaction mixture was stirred at room temperature for 18 h. After LCMS indicated the completion of the reaction, the reaction mixture was filtered, and the filtrate was purified by RP-flash [column: Welch XB-C18 column, 5 μm silica, 21.2 mm diameter, 250 mm length; a mixture of water (containing 0.1% formic acid) and acetonitrile with decreasing polarity was used as the eluent, acetonitrile gradient: 40%-63%, elution time: 9 min] to give intermediate compound **8-1** (12 g).
MS (ESI): m/z= 302.1 $[M+H]^+$.
$^1$H NMR (400 MHz, DMSO-$d_6$) δ 11.30 (s, 1H), 9.01 (d, *J* = 2.3 Hz, 1H), 8.66 (d, *J* = 2.3 Hz, 1H), 4.20 - 4.09 (m, 2H), 3.61 (s, 2H), 1.27 - 1.17 (m, 3H).

**Step 2: Synthesis of ethyl 3-((6-cyano-5-(trifluoromethyl)pyridin-3-yl)amino)-2,2-dihydroxy-3-oxopropanoate**

Intermediate **8-1** (3 g, 9.96 mmol) was added to acetic acid (8 mL), acetonitrile (10 mL), and water (2 mL), before $NaClO_2$ (2.96 g, 26.18 mmol, content: 80%) was added in an ice-water bath. The reaction mixture was stirred at room temperature for 2 h. After LCMS indicated the completion of the reaction, the reaction mixture was poured into ethyl acetate (50 mL) and water (20 mL), and the phases were separated. The aqueous phase was extracted twice with ethyl acetate (40 mL). The organic phases were combined, washed with saturated brine (50 mL), dried over anhydrous sodium sulfate, and

filtered, and the filtrate was concentrated to give intermediate compound **8-2** (3 g).
MS (ESI): m/z= 334.1 $[M+H]^+$.
$^1H$ NMR (400 MHz, DMSO-$d_6$) δ 11.01 (s, 1H), 9.39 (d, *J* = 2.2 Hz, 1H), 8.85 (d, *J* = 2.3 Hz, 1H), 7.81 (s, 2H), 4.22 - 4.10 (m, 2H), 1.30 - 1.06 (m, 3H).

**Step 3: Synthesis of ethyl 2-[[6-cyano-5-(trifluoromethyl)-pyridin-3-yl]carbamoyl]-3,3,3-trifluoro-2-hydroxypropanoate**

Intermediate **8-2** (100 mg, 317.25 μmol), tetramethylammonium fluoride (59.10 mg, 634.51 μmol), and (trifluoromethyl)trimethylsilane (90.10 mg, 634.51 μmol) were added to tetrahydrofuran (1 mL). The reaction mixture was stirred at room temperature for 18 h. The reaction mixture was concentrated, and the residue was purified by Prep-HPLC [Phenomenex C18, 5 μm silica, 30 mm diameter, 80 mm length; a mixture of purified water and acetonitrile (acetonitrile content: 60%-70%) was used as the eluent] to give target compound **008** (25 mg).
MS (ESI): m/z = 386.00 $[M+H]^+$.
$^1H$ NMR (400 MHz, DMSO-$d_6$) δ 11.33 (s, 1H), 9.38 (d, *J* = 2.3 Hz, 1H), 9.17 (s, 1H), 8.82 (d, *J* = 2.3 Hz, 1H), 4.33 - 4.21 (m, 2H), 1.26 - 1.18 (m, 3H).

**Example 9: 2-Fluoroethyl 3-[[6-cyano-5-(trifluoromethyl)-pyridin-3-yl]amino]-2-hydroxy-2-methyl-3-oxo-propanoate (compound 009)**

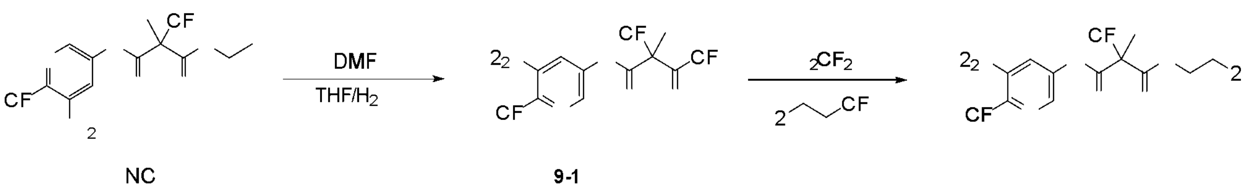


9-1

**Step 1: 3-[[6-Cyano-5-(trifluoromethyl)-pyridin-3-yl]amino]-2-hydroxy-2-methyl-3-oxopropanoic acid (compound 9-1)**

Compound **001** (200 mg, 603.78 μmol) and lithium hydroxide (72.30 mg, 3.02 mmol) were added to tetrahydrofuran (2 mL) and water (3 mL). The reaction mixture was stirred at room temperature for 2 h. After LCMS indicated the completion of the reaction, the reaction mixture was concentrated, and the residue was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 20%-50%, elution time: 7.5 min] to give title compound **9-1** (50 mg).
MS (ESI): m/z = 304.00 $[M+H]^+$.
$^1H$ NMR (400 MHz, DMSO-$d_6$) δ 10.80 (s, 1H), 9.38 - 9.33 (m, 1H), 8.89 - 8.84 (m, 1H), 1.51 (s, 3H).

**Step 2: 2-Fluoroethyl 3-[[6-cyano-5-(trifluoromethyl)-pyridin-3-yl]amino]-2-hydroxy-2-methyl-3-oxo-propanoate**

Compound **9-1** (30 mg, 98.95 μmol) was added to 2-fluoroethanol (1 mL). Concentrated sulfuric acid (19.41 mg, 197.89 μmol, 10.55 μL) was added in an ice-water bath. The reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-63%, elution time: 8.6 min] to give target compound 009 (13.5 mg).
MS m/z (ESI): = 350.0$[M+H]^+$.
$^1H$ NMR (400 MHz, DMSO-$d_6$) δ 9.32 (s, 1H), 8.83 (s, 1H), 4.70 - 4.63 (m, 1H), 4.58 - 4.51 (m, 1H), 4.46 - 4.26 (m, 2H), 1.60 (s, 3H).

**Example 10: Propyl 2-[[6-cyano-5-(trifluoromethyl)-pyridin-3-yl]carbamoyl]-3,3,3-trifluoro-2-hydroxypropanoate (compound 010)**

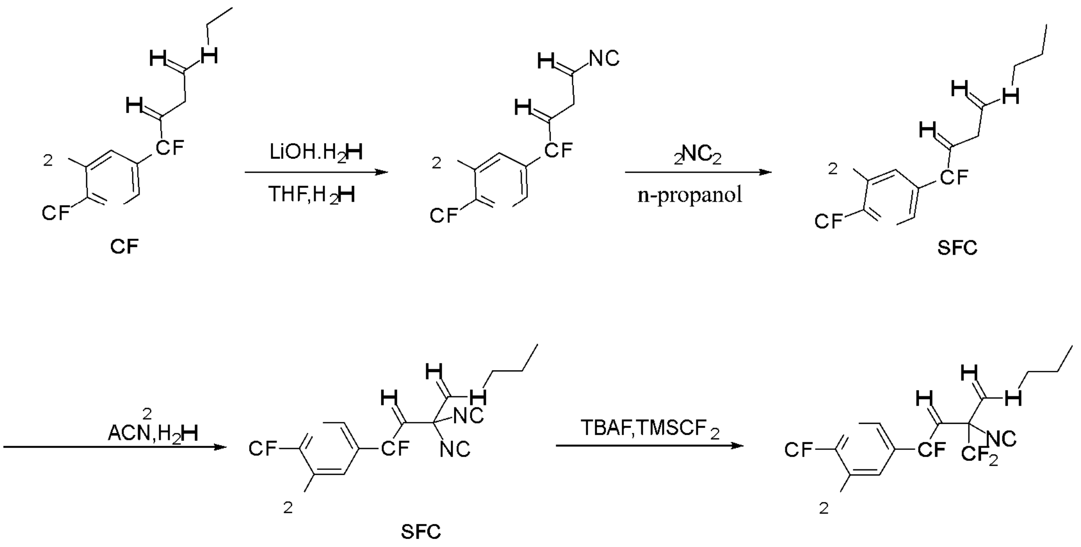


**Step 1: Synthesis of 3-[[6-cyano-5-(trifluoromethyl)-pyridin-3-yl]amino]-3-oxopropanoic acid**

Intermediate **8-1** (100 mg, 331.98 μmol) and lithium hydroxide monohydrate (23.90 mg, 597.57 μmol) were added to tetrahydrofuran (20 mL) and water (1 mL). The reaction mixture was stirred at room temperature for 18 h. After LCMS indicated the completion of the reaction, the reaction mixture was adjusted to pH = 3 with a 1 M aqueous HCl solution and extracted with ethyl acetate (50 mL × 3). The organic phases were combined, washed with saturated brine (50 mL), dried over anhydrous sulfuric acid, and filtered. The filtrate was concentrated to give intermediate compound **10-1** (80 mg).
MS (ESI): m/z= 274.1 $[M+H]^+$.

**Step 2: Synthesis of propyl 3-[[6-cyano-5-(trifluoromethyl)-pyridin-3-yl]amino]-3-oxopropanoate**

Intermediate **10-1** (100 mg, 366.08 μmol) was added to n-propanol (2 mL), and concentrated sulfuric acid (35.90 mg, 366.08 μmol, 19.51 μL) was added in an ice-water bath. The reaction mixture was stirred at room temperature for 18 h. The reaction mixture was poured into ethyl acetate (10 mL) and water (10 mL), and the phases were separated. The aqueous phase was extracted twice with ethyl acetate (10 mL). The organic phases were combined, washed once with saturated brine (50 mL), dried over anhydrous sodium sulfate, and filtered, and the filtrate was concentrated to give intermediate compound **10-2** (90 mg).
MS (ESI): m/z= 316.1 $[M+H]^+$.

**Step 3: Synthesis of propyl 3-[[6-cyano-5-(trifluoromethyl)-pyridin-3-yl]amino]-2,2-dihydroxy-3-oxopropanoate**

Intermediate **10-2** (14 g, 44.41 mmol) was added to a mixed solution of acetonitrile (10 mL), acetic acid (10 mL), and water (2 mL), before sodium chlorite (15.06 g, 133.23 mmol, content: 80%) was added in an ice-water bath. The reaction mixture was stirred at room temperature for 18 h. After LCMS indicated the completion of the reaction, the reaction mixture was concentrated, and the residue was purified by RP-flash [column: Welch XB-C18 column, 5 μm silica, 21.2 mm diameter, 250 mm length; a mixture of water (containing 0.1% formic acid) and acetonitrile with decreasing polarity was used as the eluent, acetonitrile gradient: 40%-63%, elution time: 9 min] to give intermediate compound **10-3** (8 g).
MS (ESI): m/z= 348.1 $[M+H]^+$.

**Step 4: Synthesis of propyl 2-[[6-cyano-5-(trifluoromethyl)-pyridin-3-yl]carbamoyl]-3,3,3-trifluoro-2-hydroxypropanoate**

Intermediate **10-3** (100 mg, 287.98 μmol), $TMSCF_3$ (408.93 mg, 2.88 mmol), and tetrabutylammonium fluoride (67.77 mg, 259.18 μmol) were added to tetrahydrofuran (2 mL). The reaction mixture was stirred at room temperature for 18 h. After LCMS indicated the completion of the reaction, the reaction mixture was diluted with 20 mL of water. The mixture was extracted with ethyl acetate (20 mL × 3), and the organic phases were combined, washed with water (30 mL × 2), dried over anhydrous sodium sulfate, and filtered to remove the desiccant. The filtrate was concentrated at reduced pressure and purified by column chromatography (silica, ethyl acetate/petroleum ether = 9/1) to give target compound **010**

(10 mg).
MS (ESI): m/z = 400.00 [M+H]+.
$^1$H NMR (400 MHz, DMSO-$d_6$) δ 11.36 (s, 1H), 9.41 - 9.36 (m, 1H), 9.19 (s, 1H), 8.84 - 8.79 (m, 1H), 4.28 - 4.12 (m, 2H), 1.68 - 1.54 (m, 2H), 0.90 - 0.82 (m, 3H).

**Example 11: Butyl 2-[[6-cyano-5-(trifluoromethyl)-pyridin-3-yl]carbamoyl]-3,3,3-trifluoro-2-hydroxypropanoate (compound 011)**

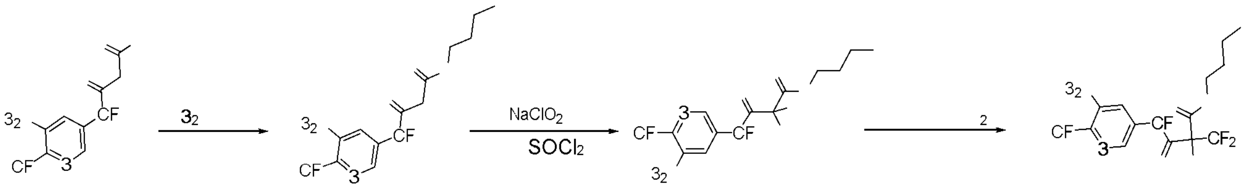


**Step 1: Synthesis of butyl 3-[[6-cyano-5-(trifluoromethyl)-pyridin-3-yl]amino]-3-oxopropanoate**

Intermediate **10-1** (100 mg, 366.08 μmol) was added to n-butanol (2 mL), and concentrated sulfuric acid (35.90 mg, 366.08 μmol, 19.51 μL) was added in an ice-water bath. The reaction mixture was stirred at room temperature for 18 h. The reaction mixture was poured into ethyl acetate (10 mL) and water (10 mL) for extraction, and the phases were separated. The aqueous phase was extracted twice with ethyl acetate (10 mL). The organic phases were combined, washed once with saturated brine (50 mL), dried over anhydrous sodium sulfate, and filtered, and the filtrate was concentrated to give intermediate compound **11-1** (120 mg).
MS (ESI): m/z= 330.1 [M+H]+.

**Step 2: Synthesis of butyl 3-[[6-cyano-5-(trifluoromethyl)-pyridin-3-yl]amino]-2,2-dihydroxy-propanoate**

Intermediate **11-1** (600 mg, 1.82 mmol) was added to a mixed solvent of acetonitrile (4 mL), acetic acid (3 mL), and water (2 mL), before sodium chlorite (411.5 mg, 3.64 mmol, content: 80%) was added in an ice-water bath. The reaction mixture was stirred at room temperature for 18 h. After LCMS indicated the completion of the reaction, the reaction mixture was concentrated, and the residue was purified by RP-flash [Welch XB-C18 column, 5 μm silica, 21.2 mm diameter, 250 mm length; a mixture of water (containing 0.1% formic acid) and acetonitrile with decreasing polarity was used as the eluent, acetonitrile gradient: 40%-63%, elution time: 9 min] to give compound **11-2** (400 mg). MS (ESI): m/z = 362.1 [M+H]+.

**Step 3: Synthesis of butyl 2-[[6-cyano-5-(trifluoromethyl)-pyridin-3-yl]carbamoyl]-3,3,3-trifluoro-2-hydroxypropanoate**

Intermediate **11-2** (115 mg, 317.25 μmol), tetrabutylammonium fluoride (58 mg, 222.08 μmol), and $TMSCF_3$ (450.50 mg, 3.17 mmol) were added to tetrahydrofuran (2 mL). The reaction mixture was stirred at room temperature for 1 h. After LCMS indicated the completion of the reaction, the reaction mixture was diluted with 20 mL of water. The mixture was extracted with ethyl acetate (20 mL × 3), and the organic phases were combined, washed with water (30 mL × 2), dried over anhydrous sodium sulfate, and filtered. The filtrate was concentrated at reduced pressure and purified by column chromatography (silica, ethyl acetate/petroleum ether = 9/1) to give target compound **011** (10 mg).
MS (ESI): m/z= 414.00 [M+H]+.
$^1$H NMR (400 MHz, DMSO-$d_6$) δ 9.07 - 9.02 (m, 1H), 8.66 - 8.60 (m, 1H), 4.25 - 4.12 (m, 2H), 1.57 - 1.45 (m, 2H), 1.32 - 1.07 (m, 2H), 0.82 - 0.71 (m, 3H).

**Example 12: Methyl 3-[[6-cyano-5-(trifluoromethyl)-pyridin-3-yl]amino]-2-hydroxy-2-methyl-3-oxopropanoate (compound 012)**

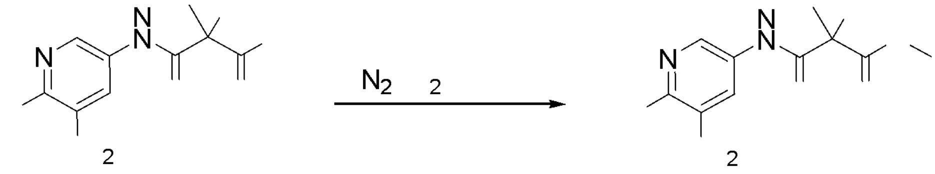


Compound **9-1** (40 mg, 131.9 μmol) was added to methanol (1 mL). Concentrated sulfuric acid (25.88 mg, 263.86 μmol, 14.06 μL) was added at room temperature. The reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of purified water and acetonitrile with decreasing polarity was used as the eluent, acetonitrile gradient: 37%-67%, elution time: 9.5 min] to give target compound **012** (12 mg).
MS (ESI): m/z= 318.10 $[M+H]^+$.
$^1H$ NMR (400 MHz, DMSO-$d_6$) δ 9.04 (d, *J* = 2.1 Hz, 1H), 8.79 (d, *J* = 2.3 Hz, 1H), 6.07 (s, 1H), 3.63 (s, 3H), 1.51 (s, 3H).

**Example 13: Isopropyl 3-[[6-cyano-5-(trifluoromethyl)-pyridin-3-yl]amino]-2-hydroxy-2-methyl-3-oxopropanoate (compound 013)**

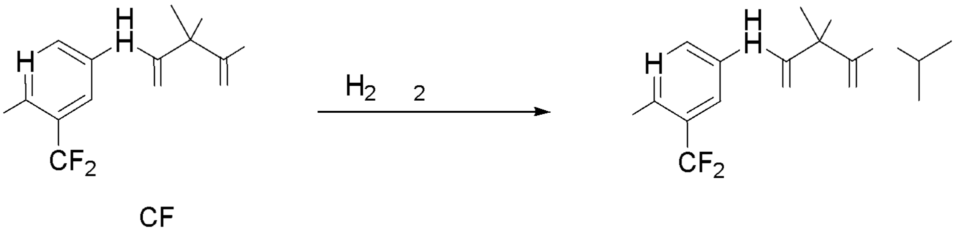


Compound 9-1 (40 mg, 131.9 μmol) was added to isopropanol (1 mL), and concentrated sulfuric acid (25.88 mg, 263.86 μmol, 14.06 μL) was added. The reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 50%-69%, elution time: 9.2 min] to give target compound **013** (13.5 mg).
MS (ESI): m/z= 346.10 $[M+H]^+$.
$^1H$ NMR (400 MHz, DMSO-$d_6$) δ 9.33 (d, *J* = 2.1 Hz, 1H), 8.83 (d, *J* = 2.1 Hz, 1H), 4.99 - 4.88 (m, 1H), 1.56 (s, 3H), 1.22 - 1.14 (m, 6H).

**Example 14: tert-Butyl 3-[[6-cyano-5-(trifluoromethyl)-pyridin-3-yl]amino]-2-hydroxy-2-methyl-3-oxopropanoate (compound 014)**

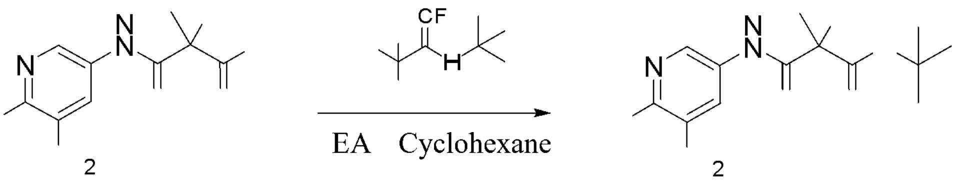


Compound **9-1** (50 mg, 164.91 μmol) was added to ethyl acetate (1 mL) and cyclohexane (1 mL). At room temperature, tert-butyl 2,2,2-trichloroethanimidate (54.05 mg, 247.37 μmol) was added to the reaction mixture, and the mixture was stirred for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 50%-69%, elution time: 9.2 min] to give target compound **014** (15 mg).
MS (ESI): m/z= 360.10$[M+H]^+$.
$^1H$ NMR (400 MHz, DMSO-$d_6$) δ 9.31 (d, *J* = 2.2 Hz, 1H), 8.83 (d, *J* = 2.2 Hz, 1H), 1.52 (s, 3H), 1.39 (s, 9H).

**Example 15: Ethyl 2-[(4-cyano-2-fluoro-3-methylsulfanyl-phenyl)carbamoyl]-3,3,3-trifluoro-2-hydroxy-propanoate (compound 015)**

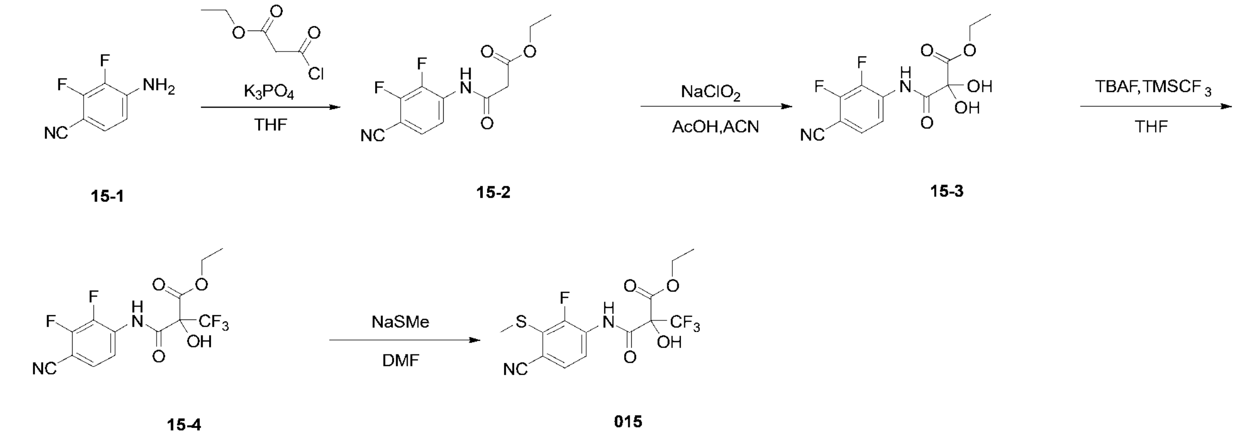


15-1 15-2 15-3 15-4 015

**Step 1: Synthesis of ethyl 2-[(4-cyano-2,3-difluorophenyl)carbamoyl]-3-oxopropanoate**

The starting materials **15-1** (100 mg, 648.86 μmol) and potassium phosphate (205.36 mg, 973.29 μmol) were added to tetrahydrofuran (20 mL), before ethyl 3-chloro-3-oxopropanoate (127.00 mg, 843.52 μmol) was added dropwise in an ice-water bath. The reaction mixture was stirred at room temperature for 2 h. After LCMS indicated the completion of the reaction, water (10 mL) was added to the reaction mixture to quench the reaction, and ethyl acetate (10 mL × 3) was added for extraction. The organic phases were combined, rinsed with saturated brine (50 mL × 2), dried, filtered, and concentrated to give intermediate compound **15-2** (150 mg).
MS (ESI): m/z = 269.1 $[M+H]^+$.

**Step 2: Synthesis of ethyl 2-[(4-cyano-2,3-difluorophenyl)carbamoyl]-2,2-dihydroxy-3-oxopropanoate**

Intermediate **15-2** (1.5 g, 5.59 mmol) was added to a mixed solvent of acetonitrile (10 mL), acetic acid (10 mL), and water (2 mL), before sodium chlorite (1.9 g, 16.78 mmol, content: 80%) was added in an ice-water bath. The reaction mixture was stirred at room temperature for 18 h. After LCMS indicated the completion of the reaction, the reaction mixture was quenched with a saturated aqueous sodium sulfite solution (10 mL) and extracted twice with ethyl acetate (10 mL). The organic phases were combined, rinsed with saturated brine (50 mL × 2), dried over anhydrous sodium sulfate, filtered, and concentrated to give a crude product of intermediate compound **15-3** (2 g).
MS (ESI): m/z= 301.1 $[M+H]^+$.

**Step 3: Synthesis of ethyl 2-[(4-cyano-2,3-difluorophenyl)carbamoyl]-3,3,3-trifluoro-2-hydroxy-propanoate**

Crude intermediate **15-3** (300 mg), $TMSCF_3$ (1.36 g, 9.55 mmol), and TBAF (224.65 mg, 859.21 μmol) were added to tetrahydrofuran (1 mL). The reaction mixture was stirred at room temperature for 1 h. After LCMS indicated the completion of the reaction, the reaction mixture was diluted with 20 mL of water. The mixture was extracted with ethyl acetate (20 mL × 3), and the organic phases were combined, washed with water (30 mL × 2), dried over anhydrous sodium sulfate, and filtered to remove the desiccant. The filtrate was concentrated at reduced pressure and purified by column chromatography (silica, ethyl acetate/petroleum ether = 5/1) to give title compound **15-4** (100 mg). MS (ESI): m/z= 353.1 $[M+H]^+$.

**Step 4: Synthesis of ethyl 2-[(4-cyano-2-fluoro-3-methylsulfanyl-phenyl)carbamoyl]-3,3,3-trifluoro-2-hydroxy-propanoate**

Intermediate **15-4** (50 mg, 141.96 μmol) was added to N,N-dimethylformamide (1 mL). Sodium thiomethoxide (11.92 mg, 170.35 μmol) was added to the reaction mixture in an ice-water bath. The reaction mixture was stirred at 100 °C for 18 h. After LCMS indicated the completion of the reaction, the reaction system was diluted with 10 mL of water and extracted with ethyl acetate (50 mL). The organic phase was concentrated, and the residue was purified by Prep-HPLC

[YMC-TA column, 5 μm silica, 30 mm diameter, 150 mm length; a mixture of purified water and acetonitrile with decreasing polarity was used as the eluent, acetonitrile gradient: 50%-90%, elution time: 10 min] to give target compound **015** (2 mg).
MS (ESI): m/z= 381.1[M+H]$^+$.
$^1$H NMR (400 MHz, DMSO-$d_6$) δ 10.24 (s, 1H), 9.10 (s, 1H), 7.80 - 7.70 (m, 2H), 4.34 - 4.19 (m, 2H), 2.56 (s, 3H),1.25 - 1.20 (m, 3H).

**Example 16: Ethyl 3-[(6-cyano-5-methylthio-pyridin-3-yl)amino]-2-hydroxy-2-methyl-3-oxopropanoate (compound 016) and ethyl (2S)-3-[(6-cyano-5-methylthio-pyridin-3-yl)amino]-2-hydroxy-2-methyl-3-oxopropanoate/ ethyl (2R)-3-(6-cyano-5-methylthio-pyridin-3-yl)amino]-2-hydroxy-2-methyl-3-oxopropanoate (016A & 016B)**

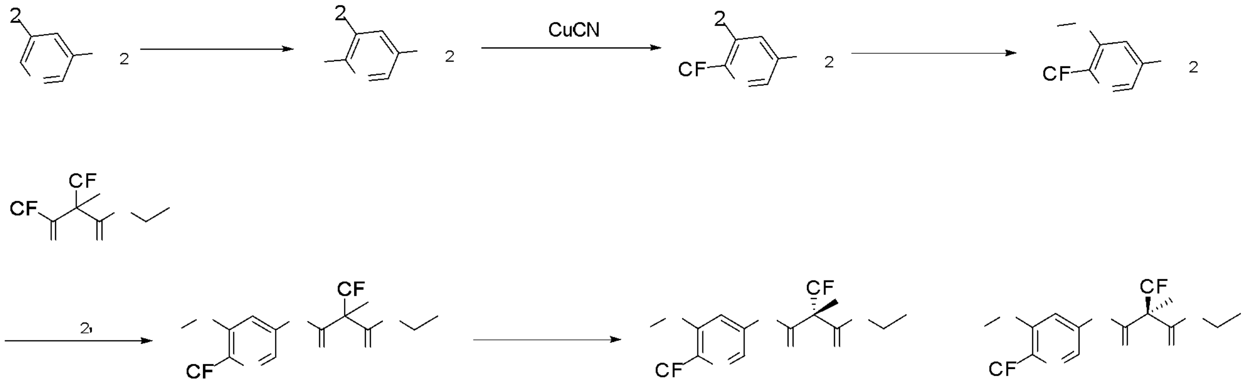


**Step 1: Synthesis of 6-bromo-5-fluoropyridin-3-amine**

5-Fluoropyridin-3-amine (13.0 g, 116 mmol) was dissolved in DMF (60 mL), and then the mixture was cooled in an ice bath. A solution of N-bromosuccinimide (20.64 g, 116 mmol) in DMF (50 mL) was slowly and dropwise added to the mixture in the ice bath, and then the mixture was allowed to react for 30 min in the ice bath. After LC-MS monitoring indicated the completion of the reaction, the reaction mixture was poured into 500 mL of water, and the resulting mixture was extracted with ethyl acetate (200 mL × 3). The organic phases were combined, washed with water (300 mL × 2) and saturated brine (300 mL × 2) in sequence, dried over anhydrous sodium sulfate, and filtered. The filtrate was concentrated at reduced pressure to give a brown-yellow crude product, which was purified by column chromatography (silica, petroleum ether/ethyl acetate = 10/1-1/1) to give intermediate 16-2 (8.4 g).
MS (ESI): m/z = 190.90, 192.90 [M+H]$^+$.

**Step 2: Synthesis of 5-amino-3-fluoro-2-picolinonitrile**

6-Bromo-5-fluoropyridin-3-amine (3 g, 15.71 mmol) and cuprous cyanide (5.63 g, 62.83 mmol) were dispersed in DMF (50 mL). A nitrogen balloon was provided, and the mixture was purged with nitrogen three times and then transferred into a 130 °C oil bath in a nitrogen atmosphere for 6 h of reaction. After LC-MS indicated the completion of the reaction, the reaction mixture was cooled to room temperature and filtered. The filtrate was concentrated at reduced pressure, and the residue was purified by column chromatography (silica, petroleum ether/ethyl acetate = 1/2) to give title compound 16-3 (1.3 g).
MS (ESI): m/z = 138.0 [M+H]$^+$.

**Step 3: Synthesis of 5-amino-3-(methylthio)picolinonitrile**

5-Amino-3-fluoro-2-picolinonitrile (1 g, 7.29 mmol) was added to a reaction flask and dissolved in DMF (15 mL), and sodium thiomethoxide (1.02 g, 14.56 mmol) was added with stirring at room temperature. The resulting reaction mixture was stirred at room temperature for 12 h. The reaction mixture was poured into water (200 mL), and the resulting mixture was extracted with ethyl acetate (80 mL × 5). The organic phases were combined, washed with water (200 mL × 2) and saturated brine (400 mL) in sequence, dried over anhydrous sodium sulfate, and filtered. The filtrate was concentrated at reduced pressure to give title compound 16-4 (0.85 g).
MS (ESI): m/z = 166.0 [M+H]$^+$.
$^1$H NMR (400 MHz, DMSO-d6) δ 7.75 (d, J = 2.3 Hz, 1H), 6.84 (d, J = 2.3 Hz, 1H), 6.47 (s, 2H), 2.50 (s, 3 H).

**Step 4: Synthesis of ethyl 3-[(6-cyano-5-methylthio-pyridin-3-yl)amino]-2-hydroxy-2-methyl-3-oxopropanoate**

Intermediate **1-2** (237 mg, 1.47 mmol) was added to tetrahydrofuran (20 mL), before thionyl chloride (348.62 mg, 2.93 mmol) was added in an ice bath. The mixture was stirred at this temperature for 2 h. Triethylamine (395.35 mg, 3.91 mmol) was added in an ice bath, and the mixture was stirred for 10 min with the temperature maintained. Intermediate **16-4** (161.4 mg, 0.977 mmol) was added, and the reaction mixture was stirred at room temperature for 6 h. After LC-MS indicated the completion of the reaction, water (30 mL) was added to quench the reaction, and the resulting mixture was extracted three times with ethyl acetate (20 mL × 3). The organic phases were combined, washed with a saturated sodium chloride solution (10 mL × 3), dried over anhydrous sodium sulfate, and filtered. The filtrate was concentrated, and the residue was purified by Prep-HPLC [column: YMC TA column; mobile phase: water as phase A; acetonitrile as phase B; gradient (B%): 35%-65%; retention time: 8.5 min] to give target compound **016** (54 mg).
MS (ESI): m/z= 310.10 $[M+H]^+$.
$^1H$ NMR (400 MHz, DMSO-$d_6$) $\delta$ 8.91 (d, *J* = 2.0 Hz, 1H), 8.36 (d, *J* = 2.1 Hz, 1H), 4.18 - 4.13 (m, 2H), 2.58 (s, 3H), 1.57 (s, 3H), 1.21 - 1.15 (m, 3H).

Compound **016** was prepared on a greater scale according to the method described above: compound **016** (100 mg) was separated by SFC [instrument: Waters 200 preparative SFC (QC-R-LC-07); column: ChiralPak AS, 250 × 50 mm I.D., 10 μm; mobile phase: carbon dioxide as phase A, methanol (0.05% $NH_3 \cdot H_2O$) as phase B; the proportion of mobile phase B was 25%; flow rate: 150 mL/min; back pressure: 100 bar; column temperature: 40 °C; detection wavelength: 210 nm] and purified to give compound **016A** (44.8 mg) and compound **016B** (36.5 mg).

**Compound 016A:**

**SFC retention time: 2.175 min.**

MS (ESI): m/z= 310.10 $[M+H]^+$.
$^1H$ NMR (400 MHz, DMSO-$d_6$) $\delta$ 10.55 (s, 1H), 8.92 (d, *J* = 2.1 Hz, 1H), 8.37 (d, *J* = 2.1 Hz, 1H), 7.01 (s, 1H), 4.18 - 4.13 (m, 2H), 2.58 (s, 3H), 1.57 (s, 3H), 1.19 (t, *J* = 7.1 Hz, 3H).

**Compound 016B:**

**SFC retention time: 2.359 min.**

MS (ESI): m/z= 310.10 $[M+H]^+$.
$^1H$ NMR (400 MHz, DMSO-$d_6$) $\delta$ 10.54 (s, 1H), 8.93 (d, *J* = 2.1 Hz, 1H), 8.37 (d, *J* = 2.1 Hz, 1H), 7.00 (s, 1H), 4.19 - 4.11 (m, 2H), 2.59 (s, 3H), 1.58 (s, 3H), 1.19 (t, *J* = 7.1 Hz, 3H).

**Example 17: 2,2,2-Trifluoroethyl 3-[[6-cyano-5-(trifluoromethyl)-pyridin-3-yl]amino]-2-hydroxy-2-methyl-3-oxo-propanoate (compound 017)**

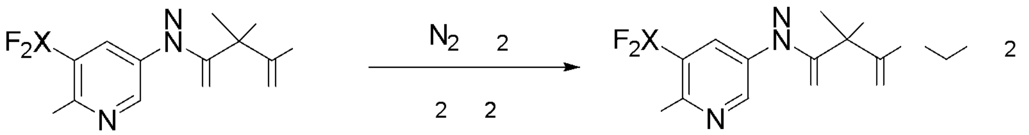

Compound **9-1** (50 mg, 164.91 μmol) was added to 2,2,2-trifluoroethanol (1 mL). Concentrated sulfuric acid (32 mg, 329.82 μmol, 18 μL) was added at room temperature, and the mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 36%-66%, elution time: 8.0 min] to give compound **017** (15 mg).
MS(ESI): m/z = 386.00 $[M+H]^+$.
$^1H$ NMR (400 MHz, DMSO-$d_6$) $\delta$ 9.31 (s, 1H), 8.82 (s, 1H), 4.92 - 4.80 (m, 2H), 1.63 (s, 3H).

**Example 18: Oxetan-3-ylmethyl 3-[[6-cyano-5-(trifluoromethyl)-pyridin-3-yl]amino]-2-hydroxy-2-methyl-3-oxo-propanoate (compound 018)**

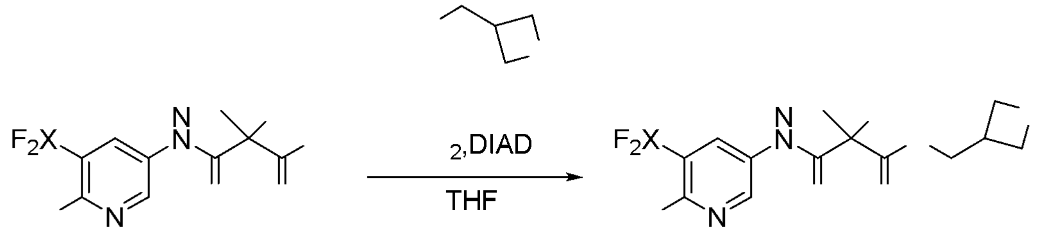


Compound **9-1** (51 mg, 168.21 μmol), oxetan-3-ylmethanol (18 mg, 201.85 μmol), and triphenylphosphine (53 mg, 201.85 μmol) were added to tetrahydrofuran (2 mL). The mixture was purged with argon three times, and diisopropyl azodicarboxylate (41 mg, 201.85 μmol) was added dropwise in an ice bath. After the dropwise addition, the mixture was warmed to room temperature and stirred for 12 h. After LC-MS monitoring indicated the completion of the reaction, the reaction mixture was concentrated. The residue was purified by Prep-HPLC [column: YMC TAR-C18, 30 × 150 mm, 5 μm; mobile phase A: water (7 mmol/L $NH_4HCO_3$), mobile phase B: acetonitrile; flow rate: 25 mL/min; mobile phase B gradient: 55%-75%, elution time: 9 min; 254/220 nm; RT: 8 min] to give compound **018** (17 mg).
MS (ESI): m/z = 374.1 $[M+H]^+$.
$^1$H NMR (400 MHz, Chloroform-*d*) δ 9.26 (s, 1H), 8.91 - 8.83 (m, 1H), 8.76 - 8.70 (m, 1H), 4.89 - 4.82 (m, 2H), 4.60 - 4.45 (m, 4H), 4.42 (s, 1H), 3.46 - 3.28 (m, 1H), 1.79 (s, 3H).

**Example 19: Oxetan-3-yl 3-[[6-cyano-5-(trifluoromethyl)-pyridin-3-yl]amino]-2-hydroxy-2-methyl-3-oxo-propanoate (compound 019)**

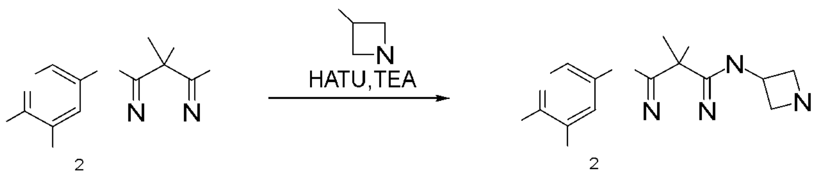


Compound **9-1** (55 mg, 181.40 μmol), oxetan-3-ol (16 mg, 217.68 μmol), 2-(7-azabenzotriazole)-N,N,N',N'-tetramethyluronium hexafluorophosphate (75 mg, 199.54 μmol), and triethylamine (36 mg, 362.80 μmol) were added to N,N-dimethylformamide (1 mL). The mixture was stirred at room temperature for 12 h. After LC-MS monitoring indicated the completion of the reaction, the reaction mixture was purified by Prep-HPLC [column: YMC TAR-C18, 30 × 150 mm, 5 μm; mobile phase A: water (7 mmol/L $NH_4HCO_3$), mobile phase B: acetonitrile; flow rate: 25 mL/min; mobile phase B gradient: 45%-70%, gradient elution for 8 min; 254/220 nm; RT: 9 min] to give compound **019** (3 mg). MS (ESI): m/z = 360.00 $[M+H]^+$.
$^1$H NMR (400 MHz, DMSO-$d_6$) δ 11.01 (s, 1H), 9.42 - 9.31 (m, 1H), 8.90 - 8.80 (m, 1H), 7.24 (s, 1H), 5.54 - 5.39 (m, 1H), 4.90 - 4.74 (m, 2H), 4.53 - 4.40 (m, 2H), 1.62 (s, 3H).

**Example 20: Tetrahydrofuran-3-yl 3-[[6-cyano-5-(trifluoromethyl)pyridin-3-yl]amino]-2-hydroxy-2-methyl-3-oxopropanoate (compound 020)**

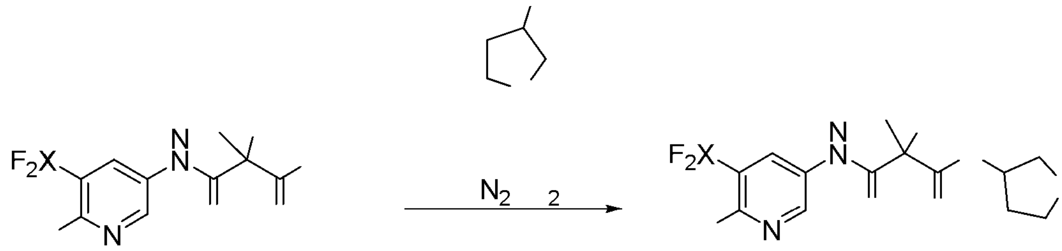


Compound **9-1** (40 mg, 131.93 μmol) was dissolved in tetrahydrofuran-3-ol (232 mg, 2.64 mmol), and concentrated sulfuric acid (39 mg, 395.79 μmol, 21 μL) was added. The mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 36%-66%, elution time: 8.0 min] to give target compound **020** (7.47 mg).
MS (ESI): m/z = 374.10 $[M+H]^+$.
$^1H$ NMR (400 MHz, DMSO-$d_6$) δ 9.33 (s, 1H), 8.86 - 8.81 (m, 1H), 5.33 - 5.26 (m, 1H), 3.84 - 3.61 (m, 4H), 2.21 - 2.09 (m, 1H), 1.92 - 1.80 (m, 1H), 1.58 (s, 3H).

**Example 21: Tetrahydropyran-4-yl 3-[[6-cyano-5-(trifluoromethyl)pyridin-3-yl]amino]-2-hydroxy-2-methyl-3-oxo-propanoate (compound 021)**

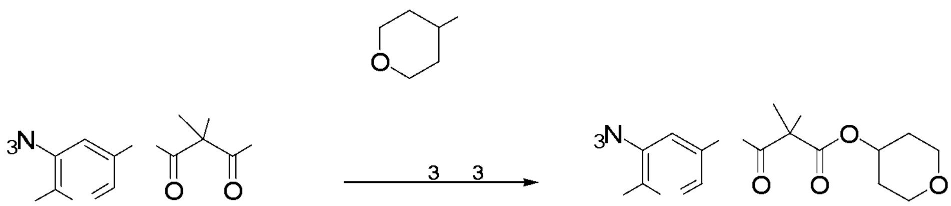


Compound **9-1** (40 mg, 131.93 μmol) was added to tetrahydropyran-4-ol (14 mg, 131.93 μmol), and concentrated sulfuric acid (26 mg, 263.86 μmol, 14 μL) was added at room temperature. The reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-63%, elution time: 8.6 min] to give compound **021** (11.6 mg).
MS (ESI): m/z = 388.00 $[M+H]^+$.
$^1H$ NMR (400 MHz, DMSO-$d_6$) δ 9.25 (d, *J* = 2.2 Hz, 1H), 8.81 (d, *J* = 2.3 Hz, 1H), 4.98 - 4.87 (m, 1H), 3.77 - 3.63 (m, 2H), 3.52 - 3.40 (m, 2H), 1.89 - 1.73 (m, 2H), 1.59 - 1.34 (m, 5H).

**Example 22: Butyl 3-[4-cyano-3-(trifluoromethyl)anilino]-2-hydroxy-2-methyl-3-oxo-propanoate (compound 022)**

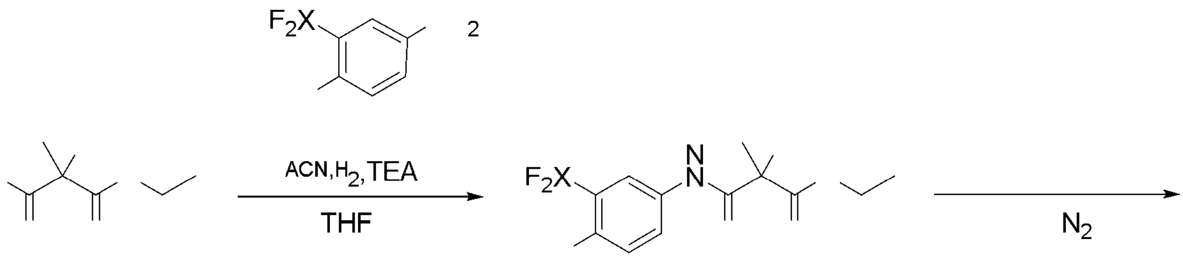


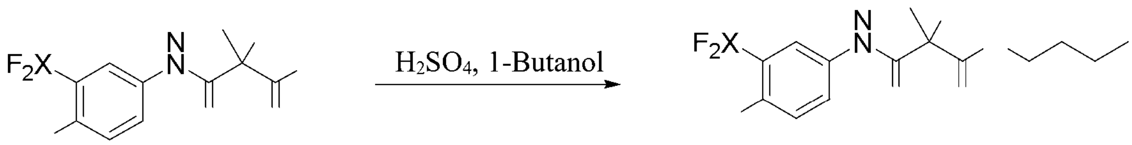


**Step 1: Synthesis of ethyl 3-[4-cyano-3-(trifluoromethyl)anilino]-2-hydroxy-2-methyl-3-oxo-propanoate**

The starting material **1-2** (65 mg, 400.89 μmol) was dissolved in tetrahydrofuran (1 mL), and thionyl chloride (95 mg, 801.73 μmol) was added dropwise. The mixture was stirred overnight at room temperature. Triethylamine (162 mg, 1.60 mmol) was added dropwise in an ice bath, and the mixture was stirred for 20 min. Then a solution of 4-cyano-3-(trifluoromethyl)aniline (89 mg, 481.07 μmol) in tetrahydrofuran (1 mL) was added dropwise to the reaction mixture. The mixture was stirred at room temperature for another 2 h. After LC-MS monitoring indicated the completion of the reaction, the mixture was subjected to rotary evaporation at reduced pressure to remove the solvent, and the residue was purified by column chromatography (silica, ethyl acetate/petroleum ether = 5/1) to give title compound **22-2** (70 mg).
MS (ESI): m/z = 331.1 $[M+H]^+$.

**Step 2: Synthesis of 3-[4-cyano-3-(trifluoromethyl)anilino]-2-hydroxy-2-methyl-3-oxopropanoic acid**

Intermediate **22-2** (70 mg, 212.12 μmol) and lithium hydroxide (7.2 mg, 300.65 μmol) were added to tetrahydrofuran (1 mL) and water (0.5 mL), and the mixture was stirred at room temperature for 4 h. After LC-MS monitoring indicated the completion of the reaction, 20 mL of water was added to the reaction system. The mixture was adjusted to weak acidity with diluted hydrochloric acid and extracted 3 times with ethyl acetate (30 mL × 3). The organic phases were combined, dried over anhydrous sodium sulfate, and filtered. The filtrate was concentrated to give title compound **22-3** (50 mg).
MS (ESI): m/z = 303.0 $[M+H]^+$.

**Step 3: Synthesis of butyl 3-[4-cyano-3-(trifluoromethyl)anilino]-2-hydroxy-2-methyl-3-oxo-propanoate**

Intermediate **22-3** (50 mg, 165.55 μmol) was added to 1-butanol (1 mL), and concentrated sulfuric acid (87 mg) was added dropwise in an ice bath. The reaction mixture was stirred at room temperature overnight. After LC-MS monitoring indicated the completion of the reaction, 40 mL of ethyl acetate was added to the reaction system. The mixture was washed once with water and once with saturated brine and concentrated, and the residue was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 50%-90%, elution time: 10 min] to give target compound **022** (18 mg).
MS (ESI): m/z = 359.0 $[M+H]^+$.
$^1$H NMR (400 MHz, DMSO-$d_6$) δ 8.51 - 8.45 (m, 1H), 8.29 - 8.21 (m, 1H), 8.14 - 8.07 (m, 1H), 4.15 - 4.05 (m, 2H), 1.59 - 1.47 (m, 5H), 1.35 - 1.21 (m, 2H), 0.92 - 0.78 (m, 3H).

**Example 23: Butyl 3-[4-chloro-3-(trifluoromethyl)anilino]-2-hydroxy-2-methyl-3-oxo-propanoate (compound 023)**

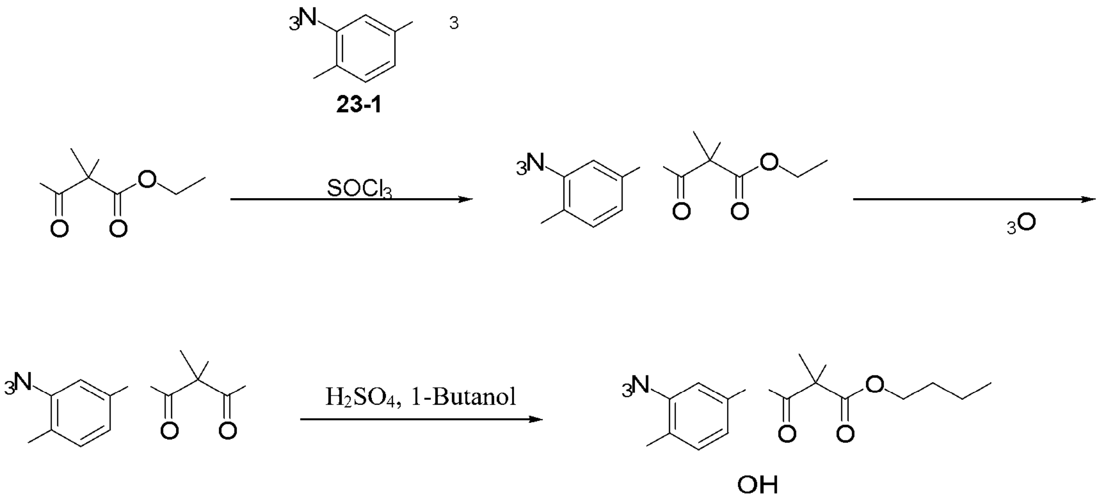


23-1

**Step 1: Synthesis of ethyl 3-[4-chloro-3-(trifluoromethyl)anilino]-2-hydroxy-2-methyl-3-oxo-propanoate**

Intermediate **1-2** (65 mg, 400.89 μmol) was dissolved in tetrahydrofuran (1 mL), and thionyl chloride (95 mg, 801.73 μmol) was added dropwise. The mixture was stirred overnight. Triethylamine (162 mg, 1.60 mmol) was added dropwise in an ice bath, and the mixture was stirred for 20 min. Then a solution of 4-chloro-3-(trifluoromethyl)aniline (94 mg, 481.07 μmol) in tetrahydrofuran (1 mL) was added dropwise to the reaction mixture. The mixture was stirred at room temperature for 2 h. After LC-MS monitoring indicated the completion of the reaction, the reaction mixture was purified by column chromatography (silica, ethyl acetate/petroleum ether = 5/1) to give title compound **23-2** (72 mg).
MS (ESI): m/z = 340.1 $[M+H]^+$.

**Step 2: Synthesis of 3-[4-chloro-3-(trifluoromethyl)anilino]-2-hydroxy-2-methyl-3-oxo-propanoic acid**

Intermediate **23-2** (72 mg, 211.96 μmol) and lithium hydroxide (7.2 mg, 300.65 μmol) were added to tetrahydrofuran (1 mL) and water (0.5 mL), and the mixture was stirred at room temperature for 4 h. After LC-MS monitoring indicated the completion of the reaction, 15 mL of water was added to the reaction system. The mixture was adjusted to weak acidity with diluted hydrochloric acid and extracted 3 times with ethyl acetate. The organic phases were combined, dried over anhydrous sodium sulfate, and filtered. The filtrate was concentrated at reduced pressure to give title compound **23-3** (56 mg).
MS (ESI): m/z = 310.0 $[M-H]^-$.
$^1$H NMR (400 MHz, DMSO-$d_6$) δ 10.27 (s, 1H), 8.39 (d, *J* = 2.6 Hz, 1H), 8.14 - 8.03 (m, 1H), 7.67 (d, *J* = 8.8 Hz, 1H), 1.52 (s, 3H).

**Step 3: Synthesis of butyl 3-[4-chloro-3-(trifluoromethyl)anilino]-2-hydroxy-2-methyl-3-oxopropanoate**

Intermediate **23-3** (46 mg, 147.61 μmol) was added to 1-butanol (1 mL), and concentrated sulfuric acid (87 mg, 47.21 μL) was added dropwise in an ice bath. The reaction mixture was stirred at room temperature overnight. After LC-MS monitoring indicated the completion of the reaction, 50 mL of ethyl acetate was added to the reaction system. The mixture was washed once with water and once with saturated brine, concentrated, and purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 50%-90%, elution time: 10 min] to give target compound **023** (28 mg).
MS (ESI): m/z = 368.00 $[M+H]^+$.
$^1$H NMR (400 MHz, DMSO-$d_6$) δ 10.33 (s, 1H), 8.36 (d, *J* = 2.6 Hz, 1H), 8.12 - 8.01 (m, 1H), 7.67 (d, *J* = 8.8 Hz, 1H), 6.86 (s, 1H), 4.17 - 4. 01 (m, 2H), 1.57 - 1.49 (m, 5H), 1.35 - 1.21 (m, 2H), 0.83 (t, *J* = 7.4 Hz, 3H).

**Example 24: 2-Hydroxyethyl 3-((6-cyano-5-(trifluoromethyl)pyridin-3-yl)amino)-2-hydroxy-2-methyl-3-oxopropanoate (compound 024)**

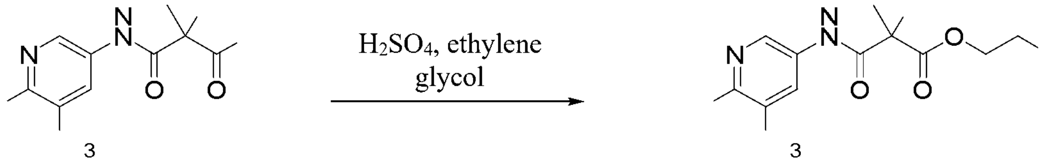


23-1

Compound **9-1** (50 mg, 164.91 μmol) was added to ethylene glycol (2 mL), and concentrated sulfuric acid (32 mg, 329.82 μmol) was added. The mixture was stirred at room temperature overnight. After LCMS indicated the completion of the reaction, the reaction mixture was poured into dichloromethane (20 mL) and water (10 mL) for extraction, and the phases were separated. The organic phase was sequentially washed with water (10 mL) and saturated brine (10 mL), dried, and concentrated. The residue was purified by Prep-HPLC [YMC TAC18 column, 5 μm silica, 30 mm diameter, 150 mm length; a mixture of water (containing 7 mmol/L $NH_4HCO_3$) and acetonitrile with decreasing polarity was used as the eluent, acetonitrile gradient: 33%-73%, elution time: 7.5 min] to give target compound **024** (20 mg).
MS (ESI): m/z = 348.00 $[M+H]^+$.
$^1$H NMR (400 MHz, DMSO-$d_6$) δ 9.10 (d, *J* = 2.3 Hz, 1H), 8.78 (d, *J* = 2.3 Hz, 1H), 4.15-4.01 (m, 2H), 3.55 (t, *J* = 5.2 Hz, 2H), 1.53 (s, 3H).

**Example 25: 3-Hydroxypropyl 3-[[6-cyano-5-(trifluoromethyl)-pyridin-3-yl]amino]-2-hydroxy-2-methyl-3-oxo-propanoate (compound 025)**

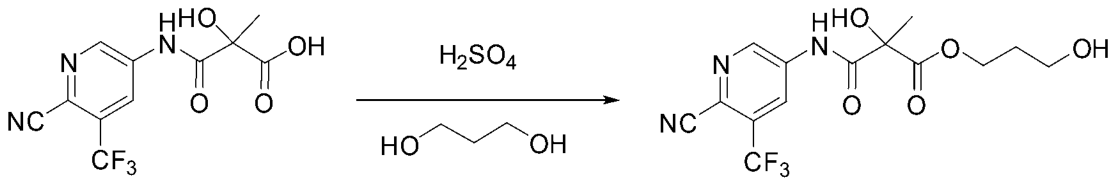


9-1 025

Compound **9-1** (20 mg, 65.96 μmol) was dissolved in 1,3-propanediol (1 mL), and concentrated sulfuric acid (13 mg, 131.93 μmol, 7 μL) was added dropwise in an ice bath. After the dropwise addition, the mixture was stirred at room temperature for 15 h. After LCMS indicated the completion of the reaction, the reaction mixture was purified by Prep-HPLC [YMC TA-C18 column, 5 μm silica, 30 mm diameter, 150 mm length; a mixture of water (containing 7 mmol/L $NH_4HCO_3$) and acetonitrile with decreasing polarity was used as the eluent, acetonitrile gradient: 50%-80%, elution time: 7.2 min] to give target compound **025** (10 mg).
MS (ESI): m/z = 362.0 $[M+H]^+$.
$^1$H NMR (400 MHz, DMSO-$d_6$) δ 9.23 - 9.17 (m, 1H), 8.83 - 8.77 (m, 1H), 4.20 - 4.08 (m, 2H), 3.45 - 3.37 (m, 2H), 1.75 - 1.64 (m, 2H), 1.54 (s, 3H).

**Example 26: 4-Hydroxybutyl 3-[[6-cyano-5-(trifluoromethyl)-pyridin-3-yl]amino]-2-hydroxy-2-methyl-3-oxopropanoate (compound 026)**

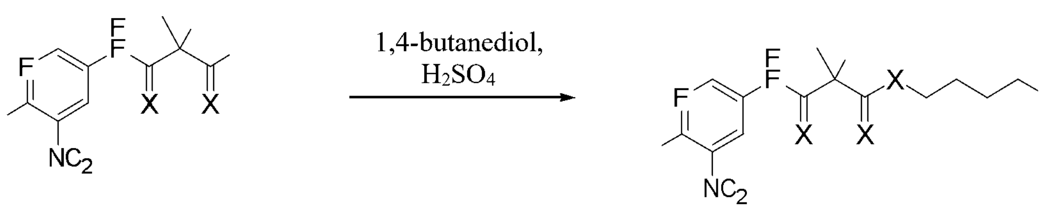


NC

1,4-Butanediol (26.75 mg, 296.84 μmol) and compound **9-1** (30 mg, 98.95 μmol) were added to DCM (1 mL). In

an ice-water bath, concentrated sulfuric acid (2 mg, 19.79 μmol, 1 μL) was added. The reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-63%, elution time: 8.6 min] to give target compound **026** (13 mg).
MS(ESI): m/z= 376.0 $[M+H]^+$.
$^1$H NMR (400 MHz, DMSO-$d_6$) δ 10.94 (s, 1H), 9.36 (s, 1H), 8.81 (s, 1H), 7.07 (s, 1H), 4.43 - 4.35 (m, 1H), 4.15 - 4.07 (m, 2H), 3.40 - 3.32 (m, 2H), 1.65 - 1.53 (m, 5H), 1.47 - 1.35 (m, 2H).

**Example 27: 3-Methoxypropyl 3-[[6-cyano-5-(trifluoromethyl)-pyridin-3-yl]amino]-2-hydroxy-2-methyl-3-oxo-propanoate (compound 027)**

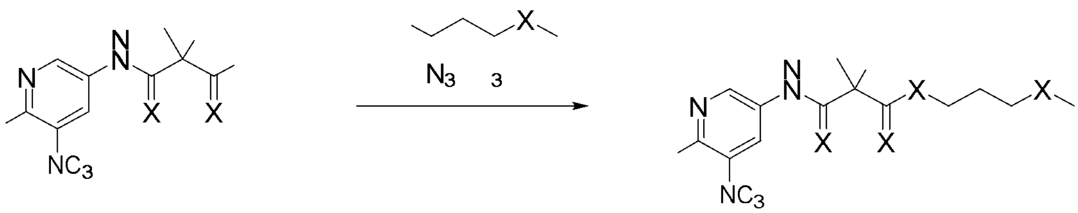

Compound **9-1** (30 mg, 98.95 μmol) and 1,3-propanediol monomethyl ether (26.75 mg, 296.84 μmol) were added to dichloromethane (3 mL). In an ice-water bath, concentrated sulfuric acid (19 mg, 197.89 μmol, 11 μL) was added. The reaction mixture was stirred at room temperature for 18 h. After LC-MS indicated that the product predominated, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-63%, elution time: 8.6 min] to give target compound **027** (10 mg).
MS(ESI): m/z = 376.00 $[M+H]^+$.
$^1$H NMR (400 MHz, DMSO-$d_6$) δ 10.97 (s, 1H), 9.37 (s, 1H), 8.85 (s, 1H), 7.10 (s, 1H), 4.20 - 4.09 (m, 2H), 3.36 - 3.27 (m, 2H), 3.16 (s, 3H), 1.85 - 1.73 (m, 2H), 1.58 (s, 3H).

**Example 28: Ethyl 5-(3-((6-cyano-5-(trifluoromethyl)pyridin-3-yl)amino)-2-hydroxy-2-methyl-3-oxopropanamido)-1-methyl-1H-pyrazole-3-carboxylate (compound 028)**

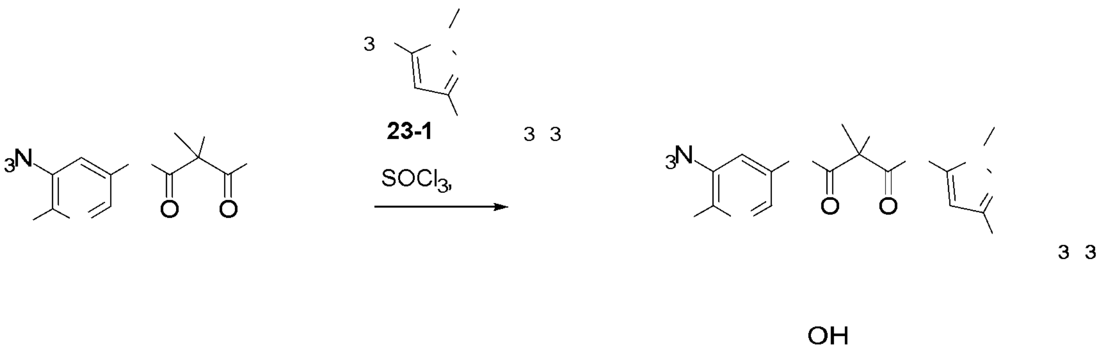


Compound **9-1** (40 mg, 131.93 μmol) was added to tetrahydrofuran (3 mL), and thionyl chloride (25 mg, 211.09 μmol) was added in an ice bath. The mixture was stirred at 0 °C for 6 h. Triethylamine (40 mg, 395.79 μmol) was added, and the mixture was stirred at 0 °C for 20 min. Intermediate **28-1** (22 mg, 131.93 μmol) was added, and the mixture was warmed to room temperature and stirred for 20 min. After LCMS indicated the completion of the reaction, the reaction mixture was poured into dichloromethane (20 mL), and the resulting mixture was washed twice with water (10 mL × 2). The organic phase was dried, filtered, and concentrated to give a crude product. The crude product was purified by HPLC [YMC-TAC column, 5 μm silica, 30 mm diameter, 150 mm length; a mixture of water (containing 7 mmol/L $NH_4HCO_3$) and acetonitrile with decreasing polarity was used as the eluent, acetonitrile gradient: 50%-80%, elution time: 9.5 min] to give target compound **028** (12 mg).

MS (ESI): m/z = 455.10 $[M+H]^+$.
$^1$H NMR (400 MHz, DMSO-$d_6$) δ 9.28 (d, *J* = 2.3 Hz, 1H), 8.83 (d, *J* = 2.3 Hz, 1H), 6.61 (s, 1H), 4.28-4.18 (m, 2H), 3.70 (s, 3H), 1.62 (s, 3H), 1.26 (t, *J* = 7.1 Hz, 3H).

**Example 29: 4-Oxopentyl 3-[[6-cyano-5-(trifluoromethyl)-pyridin-3-yl]amino]-2-hydroxy-2-methyl-3-oxopropanoate (compound 029)**

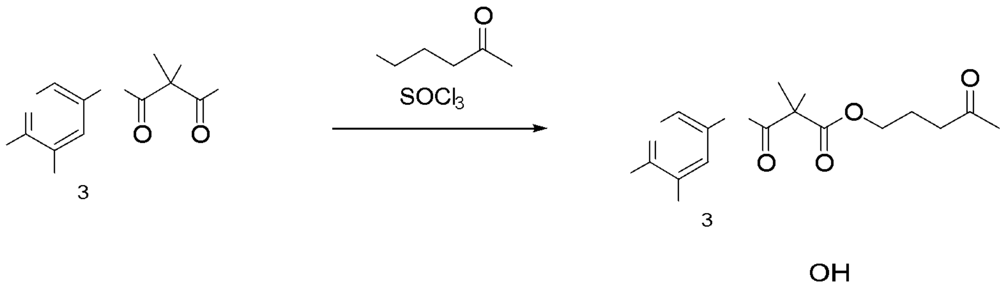


Compound **9-1** (100 mg, 329.82 μmol) was added to 5-hydroxy-2-pentanone (2 mL), and thionyl chloride (79 mg, 659.64 μmol, 48 μL) was added in an ice bath. The reaction mixture was stirred at 25 °C for 15 h. After LCMS indicated the completion of the reaction, the mixture was subjected to rotary evaporation at reduced pressure to remove the solvent, and the residue was purified by Prep-HPLC [Phenomenex $C_{18}$, 5 μm silica, 30 mm diameter, 80 mm length; a mixture of water and acetonitrile (acetonitrile content: 40%-70%) was used as the eluent] to give target compound **029** (9.6 mg).
MS (ESI): m/z =388.0 $[M+H]^+$.
$^1$H NMR (400 MHz, DMSO-$d_6$) δ 10.97 (s, 1H), 9.41 - 9.30 (m, 1H), 8.96 - 8.80 (m, 1H), 7.11 (s, 1H), 4.10 - 4.04 (m, 2H), 2.48 - 2.43 (m, 2H), 2.02 (s, 3H), 1.78 - 1.68 (m, 2H), 1.58 (s, 3H).

**Example 30: Ethyl 4-[[3-[[6-cyano-5-(trifluoromethyl)-pyridin-3-yl]amino]-2-hydroxy-2-methyl-3-oxopropanoyl]amino]butanoate (compound 030)**

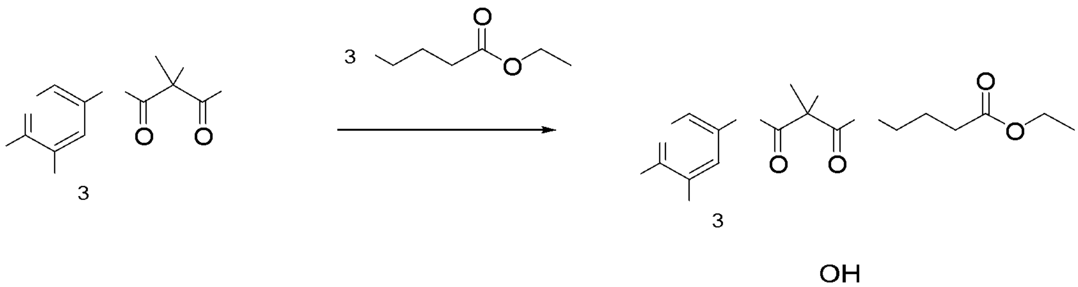

Ethyl aminobutyrate (22 mg, 164.91 μmol), compound **9-1** (50 mg, 164.91 μmol), triethylamine (33.37 mg, 329.82 μmol, 46.00 μL), and HATU (68.44 mg, 181.40 μmol) were added to DMF (2 mL). The reaction mixture was stirred at room temperature for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-63%, elution time: 8.6 min] to give target compound **030** (13 mg).
MS(ESI): m/z = 417.10 $[M+H]^+$.
$^1$H NMR (400 MHz, DMSO-$d_6$) δ 9.20 - 9.14 (m, 1H), 8.81 - 8.76 (m, 1H), 7.97 - 7.89 (m, 1H), 6.02 (s, 1H), 4.06 - 3.95 (m, 2H), 3.16 - 3.06 (m, 2H), 2.29 - 2.20 (m, 2H), 1.73 - 1.61 (m, 2H), 1.52 (s, 3H), 1.18 - 1.10 (m, 3H).

**Example 31: 2-(Oxetan-3-yl)ethyl 3-[[6-cyano-5-(trifluoromethyl)-pyridin-3-yl]amino]-2-hydroxy-2-methyl-3-oxo-propanoate (compound 031)**

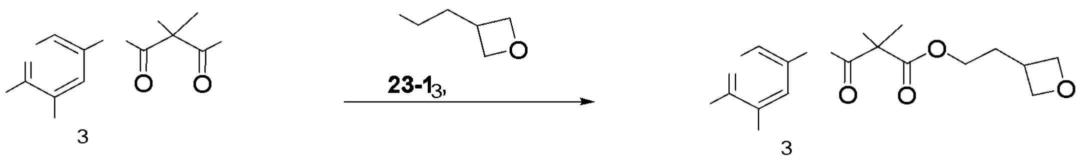


Compound **9-1** (40 mg, 131.93 μmol) and 2-(oxetan-3-yl)ethanol (16 mg, 158.31 μmol) were dissolved in tetrahydrofuran (1 mL), and triphenylphosphine (42 mg, 158.31 μmol) was added. In a nitrogen atmosphere, diethyl azodicarboxylate (32 mg, 158.31 μmol) was added dropwise in an ice bath. After the addition, the mixture was stirred at room temperature for 2 h. After LCMS indicated the completion of the reaction, the mixture was filtered, and the filtrate was concentrated. The residue was purified by Prep-HPLC [Column: YMC TA-C column, 5 μm silica, 30 mm diameter, 150 mm length; a mixture of water and acetonitrile with decreasing polarity was used as the eluent, acetonitrile gradient: 37%-66%, elution time: 7.8 min] to give target compound **031** (15 mg).
MS (ESI): m/z = 388.10 [M+H]$^+$.
$^1$H NMR (400 MHz, DMSO-$d_6$) δ 10.95 (s, 1H), 9.44 - 9.32 (m, 1H), 8.91 - 8.81 (m, 1H), 7.11 (s, 1H), 4.63 - 4.51 (m, 2H), 4.32 - 4.21 (m, 2H), 4.14 - 4.04 (m, 2H), 3.05 - 2.93 (m, 1H), 2.00 - 1.89 (m, 2H), 1.57 (s, 3H).

**Example 32: Ethyl 6-(3-((6-cyano-5-(trifluoromethyl)pyridin-3-yl)amino)-2-hydroxy-2-methyl-3-oxopropanamido)picolinate (compound 032)**

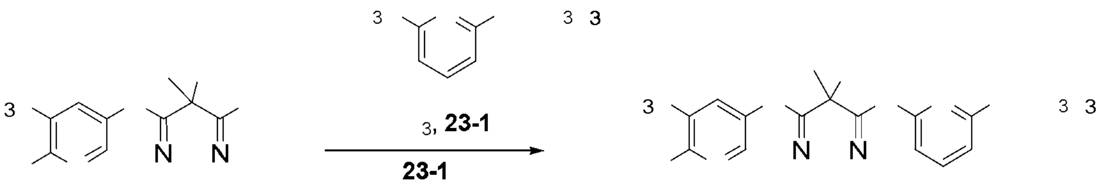


Compound **9-1** (40 mg, 131.93 μmol) was added to tetrahydrofuran (3 mL), and thionyl chloride (25 mg, 211.09 μmol) was added in an ice bath. The mixture was stirred at 0 °C for 6 h. Triethylamine (40.05 mg, 395.79 μmol) was added, and the mixture was stirred at 0 °C for 20 min. Intermediate **32-1** (22 mg, 131.93 μmol) was added, and the mixture was heated to 50 °C and stirred for 12 h. After LCMS indicated the completion of the reaction, the reaction mixture was poured into dichloromethane (20 mL), and the resulting mixture was washed twice with water (10 mL × 2). The organic phase was dried over anhydrous sodium sulfate, filtered, and concentrated to give a crude product. The crude product was purified by Prep-HPLC [YMC-TAC column, 5 μm silica, 30 mm diameter, 150 mm length; a mixture of water (containing 7 mmol/L $NH_4HCO_3$) and acetonitrile with decreasing polarity was used as the eluent, acetonitrile gradient: 42%-72%, elution time: 11 min] to give compound **032** (20 mg).
MS (ESI): m/z = 452.00 [M+H]$^+$.
$^1$H NMR (400 MHz, DMSO-$d_6$) δ 9.30 (d, $J$ = 2.3 Hz, 1H), 8.79 (d, $J$ = 2.3 Hz, 1H), 8.31 - 8.23 (m, 1H), 8.08 - 8.00 (m, 1H), 7.88 - 7.80 (m, 1H), 4.40 - 4.31 (m, 2H), 1.68 (s, 3H), 1.33 (t, $J$ = 7.1 Hz, 3H).

**Example 33: 3-Oxo-3-phenylpropyl 3-((6-cyano-5-(trifluoromethyl)pyridin-3-yl)amino)-2-hydroxy-2-methyl-3-oxopropanoate (compound 033)**

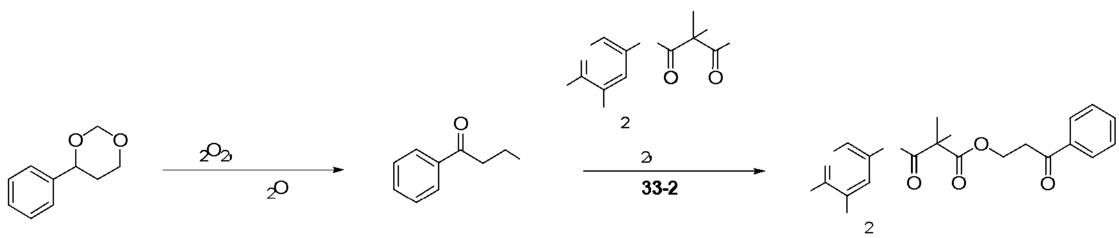


**33-2**

**Step 1: Synthesis of 3-Hydroxy-1-phenylpropan-1-one**

The starting material **33-1** (5 g, 30.45 mmol) was dissolved in dichloromethane (24 mL), and water (10 mL), hydrogen peroxide (10 mL, 37% $H_2O_2$), and an aqueous hydrogen bromide solution (2.1 g, 12.20 mmol, 47% HBr) were added. The reaction mixture was stirred at room temperature for 5 h. After LC-MS indicated the completion of the reaction, the reaction was quenched with a saturated aqueous sulfurous acid solution (40 mL), and the mixture was extracted three times with dichloromethane (20 mL × 3). The organic phases were combined, dried, and concentrated. The residue was separated and purified by column chromatography (silica, ethyl acetate/petroleum ether = 1/1) to give compound **33-2** (1 g).
MS(ESI): m/z = 151.10$[M+H]^+$.

**Step 2: Synthesis of 3-oxo-3-phenylpropyl 3-((6-cyano-5-(trifluoromethyl)pyridin-3-yl)amino)-2-hydroxy-2-methyl-3-oxopropanoate**

Compound **9-1** (40 mg, 131.93 μmol) and intermediate **33-2** (20 mg, 131.93 μmol) were dissolved in tetrahydrofuran (2 mL), and triphenylphosphine (45 mg, 171.51 μmol) and diisopropyl azodicarboxylate (35 mg, 171.51 μmol) were added. The mixture was stirred overnight at room temperature. After LC-MS indicated the completion of the reaction, the reaction mixture was concentrated, and the residue was purified by Prep-HPLC [YMC-TAR column, 5 μm silica, 30 mm diameter, 150 mm length; a mixture of water (containing 7 mmol/L $NH_4HCO_3$) and acetonitrile with decreasing polarity was used as the eluent, acetonitrile gradient: 60%-90%, elution time: 9.7 min] to give target compound **033** (15 mg).
MS(ESI): m/z = 436.00$[M+H]^+$.
$^1$H NMR (400 MHz, DMSO-$d_6$) δ 9.23 (d, $J$ = 2.2 Hz, 1H), 8.73 (d, $J$ = 2.2 Hz, 1H), 7.88- 7.81 (m, 2H), 7.65- 7.55 (m, 1H), 7.50- 7.39 (m, 2H), 4.51- 4.43 (m, 2H), 3.40 - 3.34 (m, 2H), 1.53 (s, 3H).

**Example 34: 2-Pyrrol-1-ylethyl 3-[[6-cyano-5-(trifluoromethyl)-pyridin-3-yl]amino]-2-hydroxy-2-methyl-3-oxo-propanoate (compound 034)**

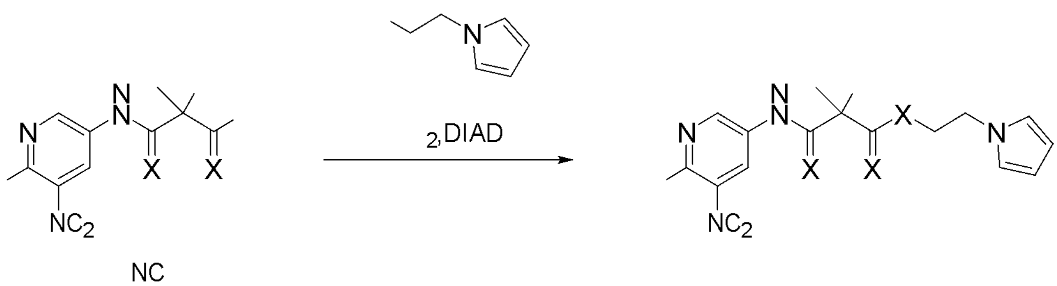


Compound **9-1** (50 mg, 164.91 μmol), 2-pyrrol-1-ylethanol (37 mg, 329.82 μmol), and triphenylphosphine (56 mg, 214.38 μmol) were added to tetrahydrofuran (1 mL). In a nitrogen atmosphere, a solution of diisopropyl azodicarboxylate (37 mg, 214.38 μmol) in tetrahydrofuran (1 mL) was added in an ice bath. The reaction mixture was stirred at 25 °C for 2 h. After LCMS indicated the completion of the reaction, the reaction mixture was filtered, and the filtrate was concentrated. The residue was purified by Prep-HPLC [Phenomenex $C_{18}$, 5 μm silica, 30 mm diameter, 80 mm length; a mixture of water and acetonitrile (acetonitrile content: 45%-75%) was used as the eluent] to give target compound **034** (2 mg).
MS (ESI): m/z=397.1 $[M+H]^+$.
$^1$H NMR (400 MHz, DMSO-$d_6$) δ 10.95 (s, 1H), 9.43 - 9.28 (m, 1H), 8.98 - 8.71 (m, 1H), 7.15 (s, 1H), 6.70 - 6.57 (m, 2H), 5.90 - 5.74 (m, 2H), 4.38 - 4.24 (m, 2H), 4.14 - 4.07 (m, 2H), 1.57 (s, 3H).

**Example 35: (2,2-Dimethyl-1,3-dioxolan-4-yl)methyl 3-[[6-cyano-5-(trifluoromethyl)-pyridin-3-yl]amino]-2-hydroxy-2-methyl-3-oxo-propanoate (compound 035)**

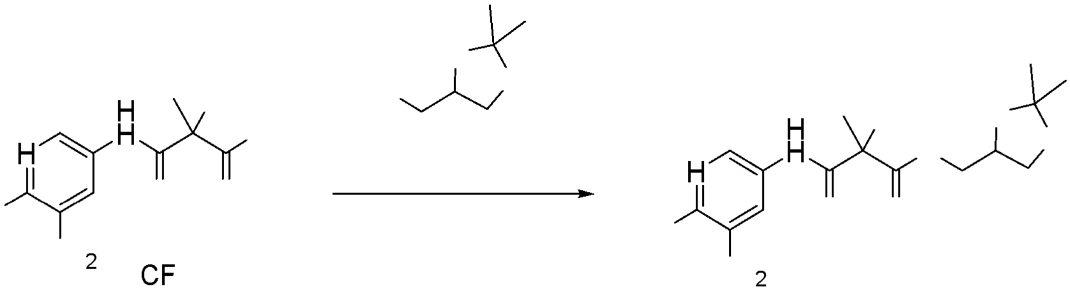

Compound **35-1** (65.38 mg, 494.73 μmol) and triethylamine (50.06 mg, 494.73 μmol) were added to N,N-dimethylformamide (3 mL), and the mixture was stirred for 10 min in advance. HATU (80.88 mg, 214.38 μmol) and intermediate **9-1** (50 mg, 164.91 μmol) were added to $SOCl_2$ (91.13 mg, 766.01 μmol) in an ice-water bath. The mixture was transferred to 80 °C and stirred for 6 h. The reaction mixture was concentrated, and the residue was purified by Prep-HPLC [column: YMC TA column; mobile phase: water as phase A; acetonitrile as phase B; gradient (B%): 55%-85%; retention time: 9.5 min] to give target compound 035 (4 mg).
MS (ESI): m/z = 418.1 $[M+H]^+$.
$^1$H NMR (400 MHz, DMSO-$d_6$) δ 9.33 (d, *J* = 2.2 Hz, 1H), 8.84 (d, *J* = 2.2 Hz, 1H), 4.29 - 4.21 (m, 1H), 4.20 - 4.02 (m, 2H), 3.98 - 3.93 (m, 1H), 3.70 - 3.62 (m, 1H), 1.59 (s, 3H), 1.27 - 1.19 (m, 6H).

**Example 36: 2-(4-Methoxyphenyl)ethyl 3-[[6-cyano-5-(trifluoromethyl)-pyridin-3-yl]amino]-2-hydroxy-2-methyl-3-oxo-propanoate (compound 036)**

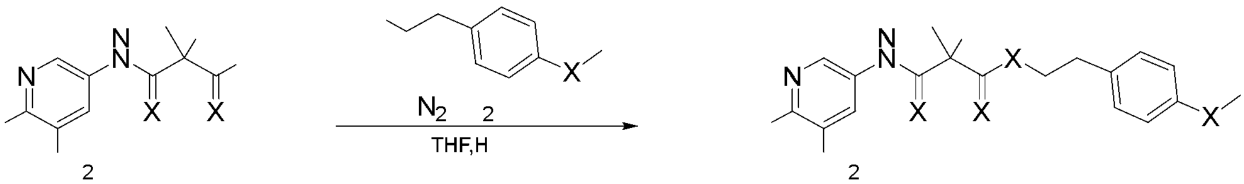


Compound **9-1** (30 mg, 98.95 μmol) and intermediate **36-1** (45 mg, 296.84 μmol) were added to dichloromethane (2 mL), and concentrated sulfuric acid (20 mg, 197.89 μmol, 11 μL) was added in an ice-water bath. The reaction mixture was stirred at room temperature for 18 h. The reaction mixture was concentrated, and the residue was purified by Prep-HPLC [column: YMC TA column; mobile phase: water as phase A; acetonitrile as phase B; gradient (B%): 65%-85%; retention time: 9.5 min] to give target compound **036** (5 mg).
MS (ESI): m/z = 438.10 $[M+H]^+$.
$^1$H NMR (400 MHz, DMSO-$d_6$) δ 10.76 (s, 1H), 9.36 - 9.31 (m, 1H), 8.84 - 8.79 (m, 1H), 7.12 - 7.03 (m, 2H), 6.69 - 6.60 (m, 2H), 4.34 - 4.20 (m, 2H), 3.64 (s, 3H), 2.84 - 2.74 (m, 2H), 1.55 (s, 3H).

**Example 37: 2-(Pyridin-3-yl)ethyl 3-[[6-cyano-5-(trifluoromethyl)-pyridin-3-yl]amino]-2-hydroxy-2-methyl-3-oxo-propanoate (compound 037)**

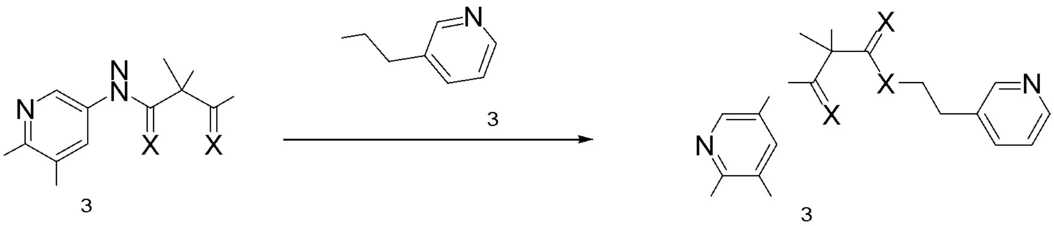


Intermediate **9-1** (30 mg, 98.95 μmol), intermediate **37-1** (12.19 mg, 98.95 μmol), diisopropyl azodicarboxylate (26.01 mg, 128.63 μmol), and triphenylphosphine (33.74 mg, 128.63 μmol) were added to tetrahydrofuran (3 mL), and the mixture was stirred at room temperature for 6 h. The reaction mixture was concentrated, and the residue was purified by

Prep-HPLC [column: YMC TA column; mobile phase: water as phase A; acetonitrile as phase B; gradient (B%): 45%-75%; retention time: 9.8 min] to give target compound **037** (7.92 mg).
MS (ESI): m/z = 409.00 $[M+H]^+$.
$^1H$ NMR (400 MHz, DMSO-$d_6$) δ 10.88 (s, 1H), 9.31 (s, 1H), 8.79 (s, 1H), 8.38 (s, 1H), 8.29 (s, 1H), 7.61 (d, *J* = 7.8 Hz, 1H), 7.18 - 7.14 (m, 1H), 4.37 - 4.33 (m, 2H), 2.92 - 2.88 (m, 2H), 1.54 (s, 3H).

**Example 38: 2-(4-Pyridinyl)ethyl 3-[[6-cyano-5-(trifluoromethyl)-pyridin-3-yl]amino]-2-hydroxy-2-methyl-3-oxo-propanoate (compound 038)**

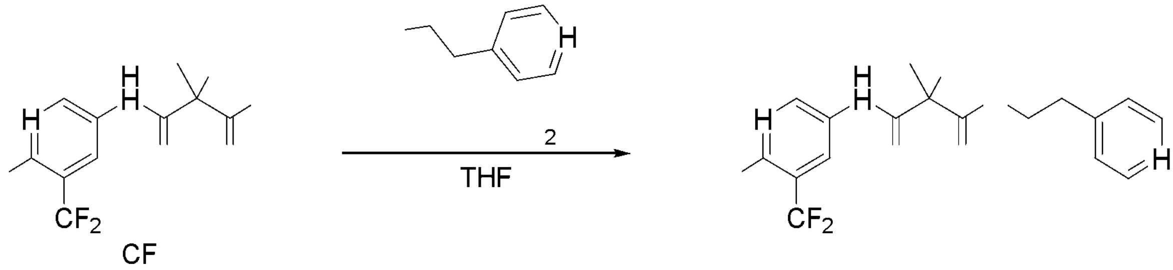


Compound **9-1** (40 mg, 131.93 μmol), intermediate **38-1** (16 mg, 131.93 μmol), diisopropyl azodicarboxylate (35 mg, 171.51 μmol), and triphenylphosphine (45 mg, 171.51 μmol) were added to tetrahydrofuran (3 mL), and the mixture was stirred at room temperature for 6 h. After LC-MS indicated the completion of the reaction, the reaction mixture was concentrated, and the residue was purified by Prep-HPLC [column: YMC TA column; mobile phase: water as phase A; acetonitrile as phase B; gradient (B%): 45%-75%; retention time: 9.8 min] to give target compound **038** (8.17 mg). MS (ESI): m/z = 409.10 $[M+H]^+$.
$^1H$ NMR (400 MHz, DMSO-$d_6$) δ 10.87 (s, 1H), 9.31 (d, *J* = 2.3 Hz, 1H), 8.77 (d, *J* = 2.3 Hz, 1H), 8.32 - 8.25 (m, 2H), 7.20 (d, *J* = 1.5 Hz, 1H), 7.19 (d, *J* = 1.6 Hz, 1H), 7.08 (s, 1H), 4.39 - 4.35 (m, 2H), 2.94 - 2.90 (m, 2H), 1.53 (s, 3H).

**Example 39: 2-(Pyridine-3-carbonylamino)ethyl 3-[[6-cyano-5-(trifluoromethyl)-pyridin-3-yl]amino]-2-hydroxy-2-methyl-3-oxo-propanoate (compound 039)**

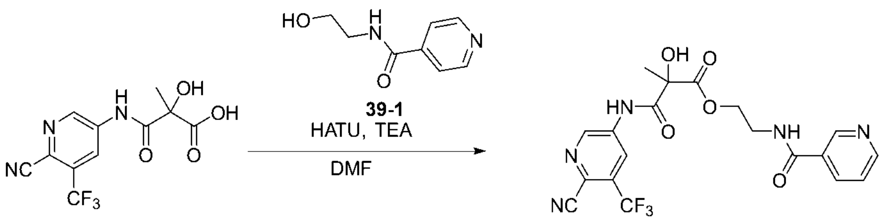


9-1

039

Compound **9-1** (100 mg, 329.82 μmol), N-(2-hydroxyethyl)isonicotinamide (110 mg, 659.64 μmol), and triethylamine (50 mg, 494.73 μmol, 69 μL) were added to N,N-dimethylformamide (3 mL), and HATU (186.65 mg, 494.73 μmol) was added in an ice bath. The reaction mixture was stirred at 25 °C for 15 h. After LCMS indicated the completion of the reaction, the reaction mixture was filtered, and the filtrate was concentrated. The residue was purified by Prep-HPLC [Phenomenex $C_{18}$, 5 μm silica, 30 mm diameter, 80 mm length; a mixture of water and acetonitrile (acetonitrile content: 30%-60%) was used as the eluent] to give target compound **039** (10 mg).
MS (ESI): m/z=452.0 $[M+H]^+$.
$^1H$ NMR (400 MHz, DMSO-d6) δ 10.90 (s, 1H), 9.31 - 9.22 (m, 1H), 8.85 - 8.81 (m, 1H), 8.79 - 8.75 (m, 1H), 8.69 - 8.62 (m, 2H), 8.06 - 7.99 (m, 1H), 7.46 - 7.38 (m, 1H), 7.05 (s, 1H), 4.33 - 4.21 (m, 2H), 3.62 - 3.46 (m, 2H), 1.58 (s, 3H).

**Example 40: Ethyl 3-[(6-cyano-5-methylsulfonylpyridin-3-yl)amino]-2-hydroxy-2-methyl-3-oxo-propanoate (compound 040)**

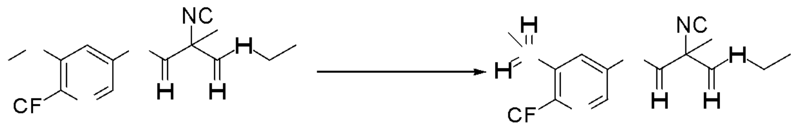

Compound **016** (50 mg, 161.63 μmol) was added to dichloromethane (3 mL), and m-chloroperoxybenzoic acid (83.68 mg, 484.90 μmol) was added. The mixture was stirred at room temperature for 6 h. After LCMS indicated the completion of the reaction, the reaction mixture was concentrated, and the residue was purified by Prep-HPLC [Phenomenex C18, 5 μm silica, 30 mm diameter, 80 mm length; a mixture of purified water and acetonitrile (acetonitrile content: 30%-60%) was used as the eluent] to give target compound 040 (11 mg).
MS (ESI): m/z = 342.1 $[M+H]^+$.
$^1H$ NMR (400 MHz, DMSO-$d_6$) δ 9.30 (d, $J$ = 2.3 Hz, 1H), 9.01 (d, $J$ = 2.3 Hz, 1H), 4.21 - 4.08 (m, 2H), 3.45 (s, 3H), 1.58 (s, 3H), 1.23 - 1.11 (m, 3H).

**Example 41: Ethyl 3-[(6-cyano-5-(methylsulfinyl)pyridin-3-yl)amino]-2-hydroxy-2-methyl-3-oxo-propanoate (compound 041)**

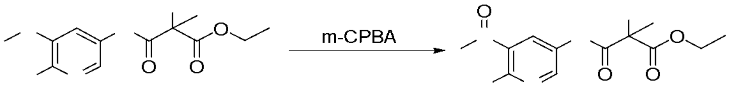


Compound **016** (30 mg, 96.98 μmol) was added to dichloromethane (3 mL), and m-chloroperoxybenzoic acid (25.10 mg, 145.47 μmol) was added. The mixture was stirred at room temperature for 3 h. After LCMS indicated the completion of the reaction, the reaction mixture was purified by prep-HPLC [column: YMC TAR column; a mixture of water (containing 0.5% formic acid) and acetonitrile (acetonitrile content: 30%-60%) was used as the eluent] to give target compound **041** (11.2 mg).
MS (ESI): m/z = 324.1 $[M-H]^-$.
$^1H$ NMR (400 MHz, DMSO-$d_6$) δ 9.13 - 9.07 (m, 1H), 8.93 - 8.86 (m, 1H), 4.20 - 4.10 (m, 2H), 2.94 - 2.87 (m, 3H), 1.59 - 1.55 (m, 3H), 1.23 - 1.14 (m, 3H).

**Example 42: Ethyl 3-((6-cyano-5-(methylselanyl)pyridin-3-yl)amino)-2-hydroxy-2-methyl-3-oxopropanoate (compound 042)**

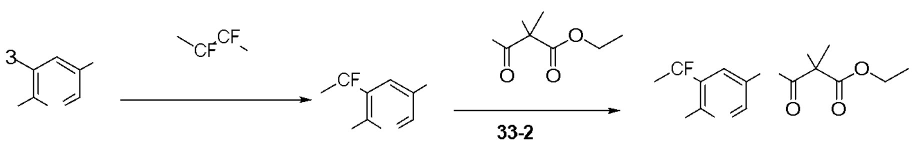


**33-2** **42-2**

**Step 1: Synthesis of 5-amino-3-(methylselanyl)picolinonitrile**

Dimethyl diselenide (2.74 g, 14.59 mmol), 5-amino-3-fluoropyridine-2-carbonitrile (1 g, 7.29 mmol), N,N-diisopropylethylamine (1.89 g, 14.59 mmol, 2.54 mL), and potassium carbonate (2.01 g, 14.59 mmol) were added to N,N-dimethylformamide (10 mL). The reaction mixture was stirred at 100 °C for 18 h. After LCMS indicated the completion of the reaction, the reaction mixture was cooled to room temperature and filtered, and the filtrate was purified by RP-flash [Welch XB-C18 column, 21.2 × 250 mm, 5 μm; a mixture of water (containing 0.1% formic acid) and acetonitrile (acetonitrile content: 40%-63%) was used as the eluent] to give title compound **42-2** (600 mg).
MS (ESI): m/z = 213.6 $[M+H]^+$.

**Step 2: Synthesis of ethyl 3-((6-cyano-5-(methylselanyl)pyridin-3-yl)amino)-2-hydroxy-2-methyl-3-oxopropanoate**

Intermediate **42-2** (100 mg, 471.45 μmol) was dissolved in tetrahydrofuran (2 mL), and the reaction mixture was

stirred in an ice-water bath for 2 h. Thionyl chloride (112.18 mg, 942.91 μmol) was added, and the mixture was stirred for 20 min. Intermediate **1-2** (122.31 mg, 754.32 μmol) was added to the reaction mixture, and the mixture was stirred for 2 h in an ice-water bath. After LCMS indicated the completion of the reaction, water (20 mL) was added to the reaction mixture in an ice-water bath to quench the reaction, and the mixture was extracted with ethyl acetate (30 mL × 3). The organic phases were combined, washed with water (40 mL × 2) and saturated brine (40 mL) in sequence, dried over anhydrous sodium sulfate, and filtered to remove the desiccant. The filtrate was concentrated at reduced pressure, and the residue was purified by column chromatography (silica, ethyl acetate/petroleum ether = 1/1) to give title compound **042** (130 mg).
MS (ESI): m/z = 357.4 $[M+H]^+$.
$^1H$ NMR (400 MHz, DMSO-$d_6$) δ 10.52 (s, 1H), 8.97 (d, *J* = 2.1 Hz, 1H), 8.45 (d, *J* = 2.2 Hz, 1H), 6.98 (s, 1H), 4.15 (q, *J* = 7.1 Hz, 2H), 2.48 (s, 3H), 1.57 (s, 3H), 1.18 (t, *J* = 7.1 Hz, 3H).

**Example 44: Ethyl 3-((6-cyano-5-(trifluoromethyl)pyridin-3-yl)amino)-2-methoxy-2-methyl-3-oxopropanoate (compound 044)**

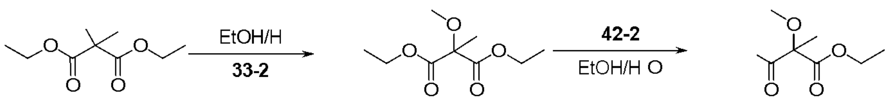


33-2 33-2

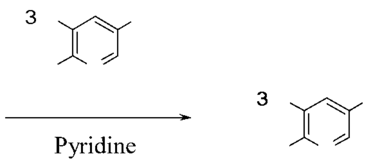


**Step 1: Synthesis of diethyl 2-methoxy-2-methylmalonate**

Diethyl 2-bromo-2-methylmalonate (10 g, 39.51 mmol) and sodium methoxide (3.20 g, 59.27 mmol) were added to tetrahydrofuran (100 mL). The reaction mixture was stirred at room temperature for 1 h. After LCMS indicated the completion of the reaction, the reaction mixture was diluted with water (500 mL) and extracted with ethyl acetate (200 mL × 4). The organic phases were combined, dried over anhydrous sodium sulfate, and filtered to remove the desiccant. The filtrate was concentrated at reduced pressure to give title compound **44-2** (6.5 g).
MS (ESI): m/z =205.20 $[M+H]^+$.
$^1H$ NMR (400 MHz, Chloroform-d) δ 4.30 - 4.23 (m, 4H), 3.82 - 3.78 (m, 3H), 1.65 - 1.63 (m, 3H), 1.31 - 1.26 (m, 6H).

**Step 2: Synthesis of 3-ethoxy-2-methoxy-2-methyl-3-oxopropanoic acid**

Intermediate **44-2** (1 g, 4.90 mmol) was added to a mixed solution of ethanol (4 mL) and water (8 mL), and potassium hydroxide (274.75 mg, 4.90 mmol) was added at 0 °C. The reaction mixture was stirred at room temperature for 3 h. After LCMS indicated the completion of the reaction, the reaction mixture was adjusted to pH 7-8 with a saturated sodium bicarbonate solution and washed with ethyl acetate (20 mL), and the aqueous phase was collected, adjusted to acidity (pH 2-3) with diluted hydrochloric acid (10 mL), and extracted with ethyl acetate (20 mL × 3). The organic phases were combined, dried over anhydrous sodium sulfate, filtered, and concentrated at reduced pressure to give title compound **44-3** (600 mg).
MS (ESI): m/z =177.10 $[M+H]^+$.
$^1H$ NMR (400 MHz, DMSO-$d_6$) δ 13.02 (s, 1H), 4.20 - 4.12 (m, 2H), 3.53 - 3.22 (m, 3H), 1.50 - 1.47 (m, 3H), 1.22 - 1.17 (m, 3H).

**Step 3: Synthesis of ethyl ((6-cyano-5-(trifluoromethyl)pyridin-3-yl)amino)-2-methoxy-2-methyl-3-oxopropanoate**

Intermediate **1-3** (297.41 mg, 1.59 mmol) and intermediate **44-3** (420 mg, 2.38 mmol) were dissolved in pyridine (4 mL) in a nitrogen atmosphere. Phosphorus oxychloride (487.41 mg, 3.18 mmol) was added in an ice bath. The mixture was stirred at room temperature for 15 h. After LCMS indicated the completion of the reaction, the reaction mixture was subjected to rotary evaporation at reduced pressure to remove pyridine, diluted with water (20 mL), and extracted with

ethyl acetate (20 mL × 3). The organic phases were combined, washed with saturated sodium chloride (20 mL × 2), and filtered. The filtrate was concentrated, and the residue was purified by Prep-HPLC [Phenomenex C18, 5 μm silica, 30 mm diameter, 80 mm length; a mixture of water (containing 0.05% ammonium bicarbonate) and acetonitrile (acetonitrile content: 50%-80%) was used as the eluent] to give target compound **044** (60 mg).
MS (ESI): m/z = 346.00 $[M+H]^+$.
$^1$H NMR (400 MHz, DMSO-$d_6$) δ 10.89 (s, 1H), 9.34 (d, *J* = 2.2 Hz, 1H), 8.81 (d, *J* = 2.3 Hz, 1H), 4.25 - 4.14 (m, 2H), 3.35 (s, 3H), 1.62 (s, 3H), 1.20 (t, *J* = 7.1 Hz, 3H).

**Example 45: S-Ethyl 3-[(6-cyano-5-trifluoromethylpyridin-3-yl)amino]-2-hydroxy-2-methyl-3-oxo-propanethioate (compound 045)**

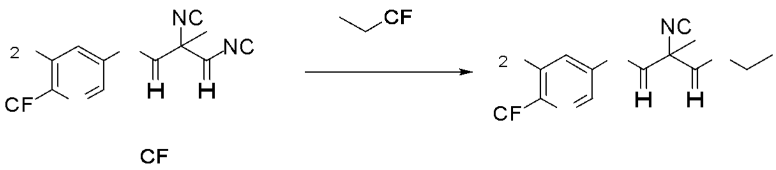

Compound **9-1** (50 mg, 164.91 μmol) was dissolved in dichloromethane (5 mL), and the solution was cooled to 0 °C. 4-Dimethylaminopyridine (2.03 mg, 16.49 μmol), ethanethiol (12.67 mg, 197.89 μmol), and dicyclohexylcarbodiimide (40.83 mg, 197.89 μmol) were added sequentially. The mixture was slowly warmed to room temperature and stirred overnight. After LCMS indicated the completion of the reaction, the reaction mixture was diluted with ethyl acetate (20 mL), washed twice with water (10 mL × 2), dried over anhydrous sodium sulfate, and filtered, and the filtrate was concentrated. The residue was separated and purified by column chromatography (silica, petroleum ether/ethyl acetate = 1/1) to give target compound **045** (20 mg).
MS (ESI): m/z =347.90 $[M+H]^+$.
$^1$H NMR (400 MHz, DMSO-$d_6$) δ 10.94 (s, 1H), 9.39 (d, *J* = 2.3 Hz, 1H), 8.85 (d, *J* = 2.3 Hz, 1H), 7.58 (s, 1H), 2.88 - 2.70 (m, 2H), 1.58 (s, 3H), 1.16 (t, *J* = 7.4 Hz, 3H).

**Example 46: Ethyl 3-(4-cyano-2-fluoro-3-methylthioanilino)-2-hydroxy-2-methyl-3-oxopropanoate (compound 046)**

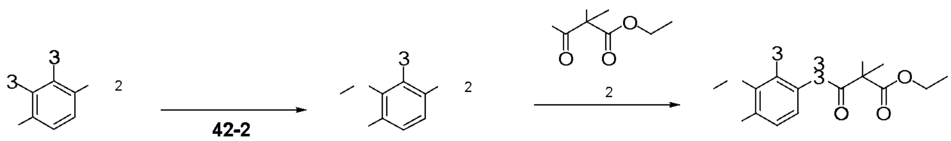


**Step 1: Synthesis of 4-cyano-2-fluoro-3-methylthioaniline**

4-Amino-2,3-difluorobenzonitrile (10 g, 64.89 mmol) was added to N,N-dimethylformamide (100 mL), and sodium thiomethoxide (5.46 g, 77.86 mmol) was added in an ice bath. The reaction mixture was stirred at 25 °C for 2 h. After LCMS indicated the completion of the reaction, the system was diluted with water (20 mL) and filtered, and the filtrate was purified by prep-HPLC [Phenomenex $C_{18}$, 5 μm silica, 30 mm diameter, 80 mm length; a mixture of water (containing 0.05% ammonium bicarbonate) and acetonitrile (acetonitrile content: 60%-70%) was used as the eluent] to give title compound **46-1** (10.3 g).
MS (ESI): m/z =183.0 $[M+H]^+$.

**Step 2: Synthesis of ethyl 3-(4-cyano-2-fluoro-3-methylthioanilino)-2-hydroxy-2-methyl-3-oxopropanoate**

Intermediate **1-2** (66.74 mg, 411.60 μmol) was dissolved in tetrahydrofuran (3 mL), and thionyl chloride (65.29 mg, 548.79 μmol) was added in an ice bath. The mixture was stirred for 1 h. Triethylamine (83.30 mg, 823.19 μmol) was added in an ice bath, and the mixture was stirred for 1 h. Intermediate **46-1** (50 mg, 274.40 μmol) was added, and the mixture was transferred to room temperature and stirred for 2 h. After LC-MS indicated the completion of the reaction, the reaction mixture was purified by prep-HPLC [column: YMC TA column; a mixture of water (containing 0.05% ammonium

bicarbonate) and acetonitrile (acetonitrile content: 55%-85%) was used as the eluent] to give title compound **046** (19 mg).
MS (ESI): m/z = 327.1 $[M+H]^+$.
$^1$H NMR (400 MHz, DMSO-$d_6$) δ 8.10-8.02 (m, 1H), 7.73 (d, *J* = 8.6 Hz, 1H), 4.19-4.10 (m, 2H), 2.57 (s, 3H), 1.56 (s, 3H), 1.18 (t, *J* = 7.1 Hz, 3H).

**Example 47: Propyl 3-(4-cyano-2-fluoro-3-methylthioanilino)-2-hydroxy-2-methyl-3-oxopropanoate (compound 047)**

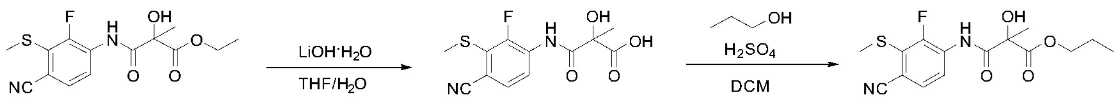


046 47-1 047

**Step 1: Synthesis of 3-(4-cyano-2-fluoro-3-methylthioanilino)-2-hydroxy-2-methyl-3-oxopropanoic acid**

Compound **046** (300 mg, 919.28 μmol) was added to a mixed solution of tetrahydrofuran (5 mL) and water (5 mL), and lithium hydroxide monohydrate (53.62 mg, 1.28 mmol) was added. The reaction mixture was stirred at room temperature for 4 h. After LCMS indicated the completion of the reaction, the reaction mixture was adjusted to weak acidity (pH 2-3) with a 1 N aqueous HCl solution and extracted with ethyl acetate (10 mL × 3). The organic phases were combined, dried over anhydrous sodium sulfate, filtered, and concentrated. The residue was purified by column chromatography (silica, ethyl acetate/petroleum ether = 1/1) to give title compound **47-1** (120 mg).
MS (ESI): m/z = 299.0 $[M+H]^+$.

**Step 2: Synthesis of propyl 3-(4-cyano-2-fluoro-3-methylthioanilino)-2-hydroxy-2-methyl-3-oxopropanoate**

Intermediate **47-1** (50 mg, 167.62 μmol) was added to dichloromethane (5 mL), and n-propanol (50.37 mg, 838.11 μmol) and concentrated sulfuric acid (16.44 mg, 167.62 μmol) were added at 0 °C. The mixture was slowly warmed to room temperature and stirred for 12 h. After LCMS indicated the completion of the reaction, the reaction mixture was purified by Prep-HPLC [column: YMC TA column; a mixture of water (containing 0.05% ammonium bicarbonate) and acetonitrile (acetonitrile content: 55%-85%) was used as the eluent] to give target compound **047** (12 mg).
MS (ESI): m/z = 341.1 $[M+H]^+$.
$^1$H NMR (400 MHz, DMSO-$d_6$) δ 8.07 - 8.03 (m, 1H), 7.75 - 7.70 (m, 1H), 4.19 - 4.00 (m, 2H), 2.60 - 2.50 (m, 3H), 1.62 - 1.57 (m, 2H), 1.56 (s, 3H), 0.88 - 0.82 (m, 3H).

**Example 48: tert-Butyl 3-[(6-cyano-5-methoxypyridin-3-yl)amino]-2-hydroxy-2-methyl-3-oxo-propanoate (compound 048)**

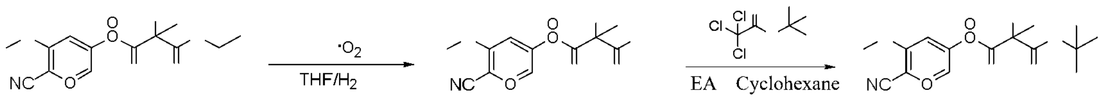


048 048

**Step 1: Synthesis of 3-[(6-cyano-5-methoxypyridin-3-yl)amino]-2-hydroxy-2-methyl-3-oxo-propanoic acid**

Compound **056** (100.00 mg, 340.98 μmol) was added to a mixed solution of tetrahydrofuran (2 mL) and water (2 mL), and lithium hydroxide monohydrate (21.78 mg, 519.02 μmol) was added. The reaction mixture was stirred at room temperature for 4 h. After LCMS indicated the completion of the reaction, the reaction mixture was adjusted to weak acidity (pH 2-3) with a 1 N aqueous HCl solution and extracted three times with ethyl acetate (10 mL × 3). The organic phases were combined, dried over anhydrous sodium sulfate, filtered, and concentrated to give title compound 48-1 (80 mg).
MS (ESI): m/z = 266.0 $[M+H]^+$.

**Step 2: Synthesis of tert-butyl 3-[(6-cyano-5-methoxypyridin-3-yl)amino]-2-hydroxy-2-methyl-3-oxo-propanoate**

Compound **48-1** (50 mg, 164.91 μmol) was added to ethyl acetate (3 mL) and cyclohexane (3 mL). At room temperature, tert-butyl 2,2,2-trichloroethanimidate (111.22 mg, 509.01 μmol) was added to the reaction mixture, and the mixture was stirred for 18 h. After LC-MS indicated the completion of the reaction, the reaction mixture was purified by Prep-HPLC [YMC-TA column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-70%] to give target compound **048** (5 mg).
MS (ESI): m/z = 322.20 $[M+H]^+$.
$^1$H NMR (400 MHz, DMSO-$d_6$) δ 10.44 (s, 1H), 8.72 (d, *J* = 2.0 Hz, 1H), 8.22 (d, *J* = 1.9 Hz, 1H), 6.84 (s, 1H), 3.93 (s, 3H), 1.52 (s, 3H), 1.40 (s, 9H).

**Example 49: tert-Butyl 3-[(6-cyano-5-methylthiopyridin-3-yl)amino]-2-hydroxy-2-methyl-3-oxo-propanoate (compound 049)**

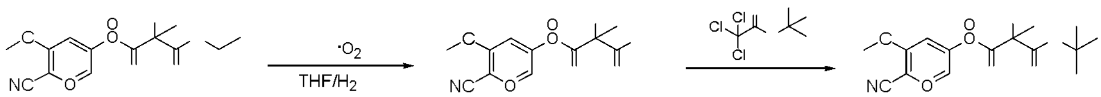


**048** **048**

**Step 1: Synthesis of 3-[(6-cyano-5-methylthiopyridin-3-yl)amino]-2-hydroxy-2-methyl-3-oxo-propanoic acid**

Compound **016** (500 mg, 1.62 mmol) was added to a mixed solution of tetrahydrofuran (6 mL) and water (6 mL), and lithium hydroxide monohydrate (81.39 mg, 1.94 mmol) was added. The reaction mixture was stirred at room temperature for 1 h. After LCMS indicated the completion of the reaction, the reaction mixture was diluted with water (20 mL) and washed with ethyl acetate (30 mL × 2). The aqueous phase was adjusted to weak acidity (pH 2-3) with a 1 N aqueous HCl solution and extracted three times with ethyl acetate (30 mL × 3). The organic phases were combined, dried over anhydrous sodium sulfate, filtered, and concentrated to give title compound **49-1** (320 mg).
MS (ESI): m/z = 282.0 $[M+H]^+$.

**Step 2: Synthesis of tert-butyl 3-[(6-cyano-5-methylthiopyridin-3-yl)amino]-2-hydroxy-2-methyl-3-oxo-propanoate**

Intermediate **49-1** (180 mg, 639.92 μmol) was added to dichloromethane (3 mL), before tert-butanol (0.3 mL) and tert-butyl 2,2,2-trichloroethanimidate (1.4 g, 6.40 mmol) were added in sequence. The mixture was stirred at room temperature for 12 h. After LCMS indicated the completion of the reaction, the reaction mixture was concentrated, and the residue was purified by Prep-HPLC [Phenomenex $C_{18}$, 5 μm silica, 30 mm diameter, 80 mm length; a mixture of purified water and acetonitrile (acetonitrile content: 35%-60%) was used as the eluent] to give target compound **049** (9 mg).
MS (ESI): m/z = 336.1 $[M-H]^-$.
$^1$H NMR (400 MHz, DMSO-$d_6$) δ 10.46 (s, 1H), 8.93 (d, *J* = 2.1 Hz, 1H), 8.34 (d, *J* = 2.1 Hz, 1H), 2.58 (s, 3H), 1.53 (s, 3H), 1.40 (s, 9H).

**Example 50** The following compound was synthesized with reference to the synthesis methods of the corresponding examples in the table and with the replacement of the corresponding starting materials:

| Compound | Starting materials | Reference example | Structural formula | MS m/z (ESI) | Nuclear magnetic resonance ($^1$H NMR) |
|---|---|---|---|---|---|
| 050 | 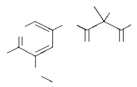 49-1 | Referring to Examples 6 and 7; compound **9-1** was replaced | 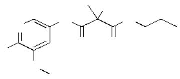 | 322.1 [M-H]$^-$ | $^1$H NMR (400 MHz, DMSO-$d_6$) δ 10.54 (s, 1H), 8.92 (d, *J* = 2.1 Hz, 1H), 8.36 (d, *J* = 2.1 Hz, 1H), 7.07 (s, 1H), 4.12 - 4.01 (m, 2H), 2.58 (s, 3H), 1.60 - 1.51 (m, 5H), 0.91 - 0.78 (m, 3H). |
| 051 | 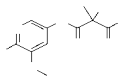 49-1 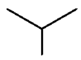 and | Referring to Example 45; compound **9-1** and ethanethiol were replaced, respectivel y | 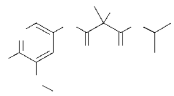 | 338.10 [M-H]$^-$ | $^1$H NMR (400 MHz, DMSO-$d_6$) δ10.51 (s, 1H), 8.93 (d, *J* = 2.1 Hz, 1H), 8.40 - 8.31 (m, 1H), 2.58 (s, 3H), 1.56 (s, 3H), 1.27 - 1.22 (m, 6H). |
| 052 | 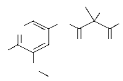 49-1 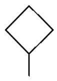 and | Referring to Example 13; compound **9-1** and n-propanol were replaced, respectivel y | 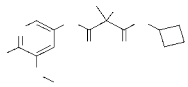 | 334.0 [M-H]$^-$ | $^1$H NMR (400 MHz, DMSO-$d_6$) δ 10.53 (s, 1H), 8.95 - 8.89 (m, 1H), 8.36 (d, *J* = 2.2 Hz, 1H), 7.02 (s, 1H), 5.00 - 4.88 (m, 1H), 2.58 (s, 3H), 2.34 - 2.21 (m, 2H), 2.10 - 1.90 (m, 2H), 1.81 - 1.67 (m, 1H), 1.68 - 1.56 (m, 1H), 1.56 (s, 3H). |
| 053 | 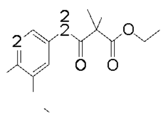 042 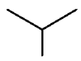 and | Referring to Example 47; compound **046** and n-propanol were replaced | 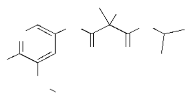 | 371.70 [M+H]$^+$ | $^1$H NMR (400 MHz, DMSO-$d_6$) δ 8.95 (d, *J* = 2.1 Hz, 1H), 8.43 (d, *J* = 2.2 Hz, 1H), 4.99 - 4.87 (m, 1H), 2.47 (s, 3H), 1.55 (s, 3H), 1.22 - 1.15 (m, 6H). |
| 054 | 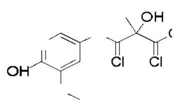 49-1 | Referring to Example 13; compound **9-1** was replaced | 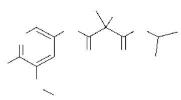 | 322.10 [M-H]$^-$ | $^1$H NMR (400 MHz, DMSO-$d_6$) δ 8.95 - 8.87 (m, 1H), 8.37 - 8.35 (m, 1H), 4.99 - 4.87 (m, 1H), 2.58 (s, 3H), 1.56 - 1.54 (m, 3H), 1.28 - 1.14 (m, 6H). |

(continued)

| Compound | Starting materials | Reference example | Structural formula | MS m/z (ESI) | Nuclear magnetic resonance ($^1$ H NMR) |
|---|---|---|---|---|---|
| 055 | 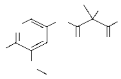 49-1 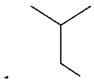 and | Referring to Example 13; compound **9-1** and isopropa- no l were re- placed, re- spectivel y | 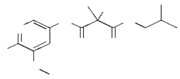 | 336.1 [M-H]$^-$ | $^1$H NMR (400 MHz, DMSO-$d_6$) δ 10.52 (s, 1H), 8.92 (d, *J* = 2.1 Hz, 1H), 8.35 (d, *J* = 2.1 Hz, 1H), 3.96 - 3.85 (m, 2H), 2.58 (s, 3H), 1.97 - 1.80 (m, 1H), 1.58 (s, 3H), 0.85 (d, *J* = 6.7 Hz, 6H). |
| 056 | 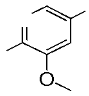 | Referring to Example 1; compound **1-3** was replaced | 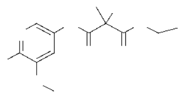 | 294.10 [M+H]$^+$ | $^1$H NMR (400 MHz, DMSO-$d_6$) δ 10.54 (s, 1H), 8.72 (d, *J* = 1.9 Hz, 1H), 8.25 (d, *J* = 1.9 Hz, 1H), 7.01 (s, 1H), 4.21 - 4.10 (m, 2H), 3.93 (s, 3H), 1.57 (s, 3H), 1.18 (t, *J* = 7.1 Hz, 3H). |
| 057 | 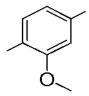 | Referring to Example 1; compound **1-3** was replaced | 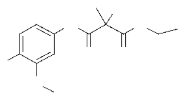 | 293.20 [M+H]$^+$ | $^1$H NMR (400 MHz, DMSO-$d_6$) *δ 7.77* (s, 1H), 7.65 (d, *J* = 8.5 Hz, 1H), 7.56 (d, *J* = 8.6 Hz, 1H), 6.96 (s, 1H), 4.19 - 4.09 (m, 2H), 3.86 (s, 3H), 1.55 (s, 3H), 1.26 - 1.14 (m, 3H). |
| 058 | 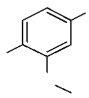 | Referring to Example 1; compound **1-3** was replaced | 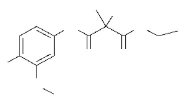 | 309.1 [M+H]$^+$ | $^1$H NMR (400 MHz, DMSO-$d_6$) δ 7.91 (d, *J* = 1.9 Hz, 1H), 7.85 - 7.77 (m, 1H), 7.72 (d, *J* = 8.5 Hz, 1H), 4.19 - 4.09 (m, 2H), 2.55 (s, 3H), 1.55 (s, 3H), 1.22 - 1.14 (m, 3H). |

**Experimental Example 1: Effect of compounds disclosed herein on AR antagonistic activity**

**(1). Materials and reagents:**

| Product | Supplier | Cat. No. |
|---|---|---|
| LNCaP cell | ATCC | CRL-1740 |
| RPMI-1640 | ATCC | 30-2001 |
| Serum | Ausgenex | FBS500-S |
| 96-Well plate | Corning | 3603 |
| Lipofectamine™ 3000 | Invitrogen | L3000015 |

(continued)

| Product | Supplier | Cat. No. |
|---|---|---|
| Bright-Lite Luciferase Assay System | Vazyme | DD1204-03 |
| 6-Well plate | Corning | 3516 |

**(2). Instrument:**

| Product | Supplier | Cat. No. |
|---|---|---|
| Biosafety cabinet | ESCO | AC2-6S1-TC |
| Carbon dioxide incubator | ESCO | CLM-240B-8-TC |
| Countess™ II cell counter | NanoEnTeK | EVE-MC2 |
| Microplate centrifuge | Monad | PlatePro 3200 |
| Microplate reader | BMG LABTECH | PHERAstar FSX |

**(3). Methodology:**

Day one: cell plating

LNCaP cells were plated in a 6-well plate (Corning, 3516) at $8 \times 10^5$ cells/well.

Day two: transfection

AR and PSA ARE1 reporter gene plasmids (Bio (Anhui) Co., Ltd.) and transfection reagents Lipo3000 and P3000 (Lipofectamine™ 3000) (a total of 2.5 μg of plasmids, i.e., 1.25 μg of each plasmid, were added to each well) were left to stand and incubated for 10 min, and then 250 μL of the above transfection reagents were added dropwise to each well containing cells and co-incubated with the cells for 24 h.

Day three: cell treatment, plating, and drug addition

The cells were washed with PBS, and the medium was replaced with a phenol red-free RPMI-1640 medium containing 5% carbon adsorption serum (or 10% fetal bovine serum and 1% penicillin and streptomycin). The cells were re-plated onto a 96-well plate (Corning, 3603) according to the number of cells plated ($2 \times 10^4$ cells/well).

After cell plating, the test compound (or DMSO or positive control) was added. After at least 30 min of incubation, DHT (dihydrotestosterone) was added to the culture system at a final concentration of 1 nM. The culture system was then incubated in a carbon dioxide incubator.

Test compound: 10 mM stock solution, with a final concentration of 3.3 μM or 6.7 μM. Negative control: 0.1% DMSO. Positive control: 30 μM enzalutamide.

Day four: luciferase signal detection and data processing

After the test compound (or DMSO or positive control) and DHT were co-incubated for 24 h, the luciferase signal was detected using the Vazyme Bright-Lite substrate. The medium was added at 70 μL/well, and the plate was shaken for 2 min, followed by reading of the luciferase signal.

**(4). Data processing:**

Assay robustness was confirmed using the DMSO and low concentration control data:

$$\text{Ave_H} = \text{Ave(DMSO)}$$

$$Ave_L = Ave\ (30\ \mu M\ enzalutamide)$$

$$Inhibition\ rate\ (\%) = (Ave_H - Sample)/(Ave_H - Ave_L) \times 100\%$$

Ave_H: the measurement of the negative control (0.1% DMSO);
Ave_L: the measurement of the positive control (30 μM enzalutamide);
Sample: the measurement of the test compound;
The corresponding activity test results for the test compounds were specified in Table 1.

Table 1. Corresponding activity test results for compounds disclosed herein

| Compound | AR antagonism (inhibition rate, %) |
|---|---|
| 001 | 86.6[a] |
| 002 | 89.16[a] |
| 003 | 75.26[a] |
| 004 | 87.30[a] |
| 005 | 58.52[a] |
| 006 | 93.92[a] |
| 008 | 86.95[a] |
| 010 | 82.14[a] |
| 011 | 88.48[a] |
| 013 | 90.0[a] |
| 014 | 90.3[a] |
| 015 | 65.06[a] |
| 016A | 99.98[a] |
| 016B | 90.50[a] |
| 022 | 81.05[a] |
| 023 | 52.26[a] |
| 032 | 47.7[a] |
| 048 | 62.18[b] |
| 049 | 90.02[b] |
| 050 | 82.63[b] |
| 056 | 68.39[a] |
| 057 | 79.69[a] |
| 058 | 87.65[a] |

a: treatment concentration: 3.3 μM; b: treatment concentration: 6.7 μM

**Experimental Example 2: Metabolic stability assay of compounds disclosed herein in liver microsomes**

The metabolic stability of compounds disclosed herein in liver microsomes was determined using the following method.

I. Materials and instruments

1. Liver microsome source: Human liver microsome (Corning, 452117)
2. $Na_2HPO_4$ (Tianjin Guangfu Fine Chemical Research Institute, 20180130)
3. $KH_2PO_4$ (Tianjin Guangfu Fine Chemical Research Institute, 20180920)
4. $MgCl_2$ (Tianjin Guangfu Fine Chemical Research Institute, 20191216)
5. NADPH (Solarbio, 1216C022)
6. Positive control compound verapamil (Sigma, MKBV4993V)
7. AB Sciex Triple Quad 4000 liquid chromatography-mass spectrometry system

II. Procedures

1. Preparation of 100 mM phosphate-buffered saline (PBS): 7.098 g $Na_2HPO_4$ was dissolved in 500 mL of purified water by sonication to give solution A. 3.400 g of $KH_2PO_4$ was dissolved in 250 mL of purified water by sonication to give solution B. Solution A was placed on a stirrer, and solution B was added slowly until the pH reached 7.4, so as to give a 100 mM PBS buffer.
2. Preparation of reaction system
The reaction system was prepared according to the table below.

| Reagent | Stock solution concentration | Volume | Final concentration |
|---|---|---|---|
| Liver microsome | 20 mg/mL | 10 μL | 0.5 mg/mL |
| Phosphate-buffered saline | 100 mM | 346 μL | 100 mM |

3. The reaction system was pre-incubated in a 37 °C water bath for 10 min. 40 μL of 10 mM NADPH solution (NADPH was dissolved in 100 mM phosphate-buffered saline) was added to the reaction system, with the final concentration of NADPH being 1 mM. A negative control was prepared by replacing the NADPH solution with 40 μL of phosphate-buffered saline. The negative control was used to exclude the effect of the chemical stability of the compound itself.
4. The reaction was initiated by adding 4 μL of 100 μM compound disclosed herein or positive control compound verapamil to the reaction system at a final concentration of 1 μM.
5. After 0.5 min, 15 min, 30 min, 45 min, and 60 min of mixing well by using a vortex oscillator, 50 μ L of the incubated sample was taken out, and glacial acetonitrile containing an internal standard of 4 folds was added to terminate the reaction. The sample was centrifuged at 3,220 g for 45 min. 90 μL of the supernatant was transferred to a feeding plate after the centrifugation was completed, and 90 μL ultrapure water was added. The mixture was well mixed for LC-MS/MS analysis.

All data were calculated by using Microsoft Excel software. The peak areas were determined by using an extracted ion chromatogram. The *in vitro* half-life ($T_{1/2}$) of the test compound was determined by linear fitting of the natural logarithm of the elimination proportion of the test compound over time. *In vitro* half-life ($T_{1/2}$) was calculated by the slope k:

$$\textit{in vitro}\ T_{1/2} = 0.693/k$$

The *in vitro* intrinsic clearance *(in vitro* $CL_{int}$, unit: μL/min/mg protein) was calculated using the following formula:

$$\textit{in vitro}\ CL_{int} = k \times \text{volume of incubation solution/enzyme protein content;}$$

The calculated $T_{1/2}$ and $CL_{int}$ according to the above formulas are shown in Table 2.

Table 2. Half-life values and intrinsic clearance values of compounds disclosed herein in liver microsomes

| Compound | In vitro $T_{1/2}$ (min) | In vitro $Cl_{int}$ (μL/min/mg protein) |
|---|---|---|
| 002 | 42.60 | 32.5 |
| 003 | 22.95 | 60.4 |
| 008 | 39.07 | 35.47 |
| 016 | 13.62 | 101.8 |
| 016A | 15.1 | 91.8 |
| 016B | 15.7 | 88.3 |

As can be seen from the above results, the compounds disclosed herein are characterized by high clearance and rapid metabolism, which may avoid the accumulation of the compound in the body caused by long-term treatment and thus the systemic impact on the androgen receptor signaling pathway, thereby further improving the safety of the treatment.

### Experimental Example 3: *In vivo* pharmacodynamic study 1

#### 3.1 Reagents

Vehicle: propylene glycol/ethanol (30:70, v/v)

#### 3.2 Procedures

Animal information: C57BL/6 mice (male, aged 4-6 weeks, about 18-20 g), purchased from Shanghai Lingchang Biotechnology Co., Ltd. The mice were bred in an SPF-level environment within independently ventilated cages. All animals had free access to a certified, commercial standard laboratory diet and drinking water.

Skin preparation: After 1-2 weeks of acclimation, the hairs in a 2 cm × 3 cm area of the back of the mice were shaved to confirm that the hairs of the mice were in the telogen phase (the skin was pink) and the skin was not damaged.

Treatment: On day 3 after shaving, the mice were grouped for treatment. Compound **001** disclosed herein was applied to the shaved area at a concentration of 2 wt% twice daily in the morning and evening, and compound **008** disclosed herein was applied to the shaved area at a concentration of 0.5 wt% twice daily in the morning and evening for 30 days. The control group received the control solvent. 12 mice were in each group.

#### 3.3 Mouse state observation and hair growth scoring

During the experiment, the states of the mice in each group were observed for erythema, dehiscence, desquamation, and the like on the skin.

The mice were weighed on days 10, 13, 18, 21, 25, and 30, and the hair growth state was scored and photographed.

The scoring criteria are as follows:

0: no growth, with pink skin in the shaved area;
1: the skin in the shaved area was gray (an increase of less than 20%);
2: the skin in the shaved area was black (more than 20% but less than 40%);
3: the skin in the shaved area was black with a little hair growth (more than 40% but less than 60%);
4: the skin in the shaved area was black with partial hair growth (more than 60% but less than 80%);
5: basically complete hair growth (80%-100% growth) in the shaved area.

#### 3.4 Results

The effects of the compounds disclosed herein on mouse hair growth are shown in Table 3.

Table 3. Scoring of effect of compounds disclosed herein on hair growth in mice

| Compound | Score (mean±SD) | | | | | | |
|---|---|---|---|---|---|---|---|
| | Day 0 | Day 10 | Day 13 | Day 18 | Day 21 | Day 25 | Day 30 |
| Vehicle control group | 0 | 0.1±0.3 | 0.1±0.3 | 0.4±0.5 | 0.5±0.7 | 0.8±1.0 | 1.0±1.1 |
| 001 | 0 | 0.6±0.5** | 0.9±0.9**** | 1.3±1.4** | 1.9±1.8**** | 2.4±2.2*** | 3.0±1.8**** |
| 008 | 0 | 0.4±0.5 | 0.6±0.5* | 1.0±0.6 | 1.1±0.7 | 1.9±1.5* | 2.8±2.1 *** |
| * P < 0.05, ** *P* < 0.01, *** *P* < 0.001, **** *P* < 0.0001 vs. vehicle control group | | | | | | | |

#### 3.5 Conclusion

In mice with telogen hair growth, compound **001** disclosed herein at a concentration of 2% and compound 008 disclosed herein at a concentration of 0.5%, administered BID topically at 20 μL/cm$^2$ exhibited a significant promoting effect on mouse hair growth. Compound 001 significantly promoted the hair growth ($P < 0.01$) from day 10 of the treatment, and compound 008 significantly promoted the hair growth ($P < 0.05$) from day 13 of the treatment. Compound **001** and

compound **008** disclosed herein exhibited no significant impact on the body weight of the mice at the investigated doses and no signs of erythema, dehiscence, desquamation, and the like on the skin.

**Experimental Example 4: *In vivo* pharmacodynamic study 2**

**4.1 Reagents**

Vehicle: propylene glycol/ethanol (30:70, v/v)

**4.2 Procedures**

Animal information: C57BL/6 mice (male, aged 6 weeks, about 18-20 g), purchased from Shanghai Lingchang Biotechnology Co., Ltd. The mice were bred in an SPF-level environment within independently ventilated cages. All animals had free access to a certified, commercial standard laboratory diet and drinking water.

Skin preparation: After 1-2 weeks of acclimation, the hairs in a 2 cm × 2 cm area of the back of the mice were shaved to confirm that the hairs of the mice were in the telogen phase (the skin was pink) and the skin was not damaged. A 1 cm × 1 cm area in the center of the back skin was selected as the treatment area.

Treatment: On day 3 after shaving, the mice were grouped for treatment. Compound **016A** disclosed herein was applied to the shaved area at a concentration of 0.5 wt% twice daily in the morning and evening for 22 days. The control group received the control solvent. 12 mice were in each group.

**4.3 Mouse state observation and hair growth scoring**

During the experiment, the states of the mice in each group were observed for erythema, dehiscence, desquamation, and the like on the skin.

The mice were weighed on days 9, 12, 15, 19, and 22, and the hair growth state was scored and photographed.

The scoring criteria are as follows:

0: no growth, with pink skin in the shaved area;
1: the skin in the shaved area was gray (an increase of less than 20%);
2: the skin in the shaved area was black (more than 20% but less than 40%);
3: the skin in the shaved area was black with a little hair growth (more than 40% but less than 60%);
4: the skin in the shaved area was black with partial hair growth (more than 60% but less than 80%);
5: basically complete hair growth (80%-100% growth) in the shaved area.

**4.4 Results**

The effects of the compounds disclosed herein on mouse hair growth are shown in Table 4.

Table 4. Scoring of effect of compounds disclosed herein on hair growth in mice

| **Compound** | **Score (mean±SD)** | | | | | |
|---|---|---|---|---|---|---|
| | **Day 0** | **Day 9** | **Day 12** | **Day 15** | **Day 19** | **Day 22** |
| **Vehicle control group** | 0 | 0.0±0.0 | 0.0±0.0 | 0.0±0.0 | 0.0±0.0 | 0.3±0.5 |
| **016A** | 0 | 0.1±0.3 | 0.1±0.3 | 0.5±0.8 | 1.7±1.5**** | 2.9±1.6**** |
| ****** *P* < 0.0001 vs. vehicle control group** | | | | | | |

**4.5 Conclusion**

In mice with telogen hair growth, compound **016A** disclosed herein at a concentration of 0.5 wt%, administered BID topically at 20 μL/cm$^2$, exhibited a significant promoting effect on hair growth. Compound **016A** significantly promoted the hair growth ($P$ < 0.0001) from day 19 of the treatment. Compound **016A** exhibited no significant impact on the body weight of the mice at the investigated doses and no signs of erythema, dehiscence, desquamation, and the like on the skin.

**Experimental Example 5: Whole blood stability**

Procedures:

1. Preparation of stock solution

1 mM stock solutions of the test compounds were prepared in DMSO, and a 1 mM stock solution of the reference compound propantheline was prepared in acetonitrile.

2. Stability assay

4 μL of test compound (or reference compound) stock solution was added to 796 μL of pre-incubated human blood (whole blood) at a final concentration of 5 μM. The final concentration of the solvent was 0.5%. At different time points (including 5 min, 15 min, 30 min, 60 min, and 120 min), a 50 μL aliquot of the whole blood containing the sample was added to a new tube and incubated in a 37 °C water bath with shaking at 60 rpm. The treatment was performed in duplicate. At designated time points, 300 μL of room-temperature quenching solution (a solution of acetonitrile containing internal standards (100 nM alprazolam, 500 nM labetalol, and 2 μM ketoprofen)) was added to the whole blood sample containing the sample to terminate the reaction. 50 μL of whole blood containing the sample was added to a new tube containing 300 μL of room-temperature quenching solution to prepare a sample at 0 min. All samples were vortexed for 5 min. The samples in the plate were centrifuged at 3220 g for 30 min at 4 °C to precipitate the proteins. 100 μL of the supernatant was transferred to a new 96-well plate containing 100 μL of water for LC-MS/MS analysis.

3. Data analysis

All calculations were performed using Microsoft Excel. The peak area ratio was determined by using an extracted ion chromatogram. The proportion of remaining compound at each time point was calculated according to the following formula:

$$\text{Remaining proportion t min (\%)} = \text{peak area ratio t min/peak area ratio 0 min} \times 100\%$$

Peak area ratio t min: peak area ratio of reference compound to test compound at t min;
Peak area ratio 0 min: peak area ratio of reference compound to test compound at time zero.

The slope value was determined by linear regression on the curve of the natural logarithm of the remaining proportion of the test compound versus the incubation time.

Determination of *in vitro* half-life (*in vitro* $t_{1/2}$) from the slope value:

$$\textit{in vitro}\ t_{1/2} = 0.693/k$$

in the formula, k is the rate constant (k = -slope value)

The determined half-lives of the compounds disclosed herein in human blood are shown in Table 5.

Table 5. Stability of compounds disclosed herein in human blood

| Compound | Human whole blood half-life $T_{1/2}$ (min) |
|---|---|
| **001** | 179.89 |
| **013** | 348.95 |
| **016A** | 131.96 |
| **016B** | 101.58 |

As can be seen from the above results, the compounds disclosed herein are characterized by short half-life, high clearance, and rapid metabolism in human blood, which may avoid the accumulation of the compound in the body caused by long-term treatment and thus the systemic impact on the androgen receptor signaling pathway, thereby further improving the safety of the treatment.

Although the specific embodiments of the present disclosure have been illustrated and described, those skilled in the art will appreciate that, where feasible, the technical features described in one embodiment can be applied to or combined with the technical features described in another embodiment. Therefore, those skilled in the art can make

various changes and modifications to the embodiments of the present disclosure without departing from the spirit and scope of the present disclosure.

## Claims

1. A compound of formula (I) or a stereoisomer thereof or a pharmaceutically acceptable salt thereof,

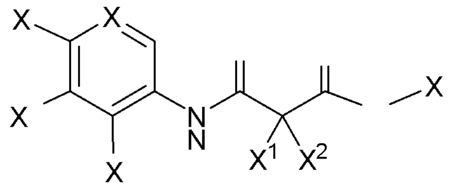

(I)

wherein

X is selected from CH or N;
Y is selected from S, O, or $NR^7$;
$R^1$ is selected from OH or -O-$C_1$-$C_6$ alkyl;
$R^2$ is selected from $C_1$-$C_6$ alkyl, $C_1$-$C_6$ deuterated alkyl, or $C_1$-$C_6$ haloalkyl;
$R^3$ is selected from $C_1$-$C_6$ alkyl, $C_1$-$C_6$ deuterated alkyl, $C_3$-$C_6$ cycloalkyl, 4- to 10-membered heterocyclyl, or 5- to 10-membered heteroaryl, wherein the $C_1$-$C_6$ alkyl, $C_3$-$C_6$ cycloalkyl, 4- to 10-membered heterocyclyl, or 5- to 10-membered heteroaryl is optionally substituted with $R^{3a}$;
$R^{3a}$ is selected from halogen, OH, CN, $NH_2$, -$COR^{3b}$, -$COOR^{3b}$, -$NHCOR^{3b}$, -$CONHR^{3b}$, -O-$C_1$-$C_6$ alkyl, phenyl, $C_1$-$C_6$ alkyl, 4- to 6-membered heterocyclyl, or 5- to 10-membered heteroaryl, wherein the phenyl, $C_1$-$C_6$ alkyl, 4- to 6-membered heterocyclyl, or 5- to 10-membered heteroaryl is optionally substituted with $R^{3c}$;
$R^{3b}$ is selected from H, $C_1$-$C_6$ alkyl, phenyl, or 5- to 6-membered heteroaryl;
$R^{3c}$ is selected from -$COOC_1$-$C_6$ alkyl, $C_1$-$C_6$ alkyl, -O-$C_1$-$C_6$ alkyl, phenyl, or 5- to 6-membered heteroaryl;
$R^4$ is selected from $NO_2$, halogen, or CN;
$R^5$ is selected from halogen, $C_1$-$C_6$ alkyl, $C_1$-$C_6$ haloalkyl, -$COOC_1$-$C_6$ alkyl, -S-$C_1$-$C_6$ alkyl, -O-$C_1$-$C_6$ alkyl, -Se-$C_1$-$C_6$ alkyl, -$S(O)_2$-$C_1$-$C_6$ alkyl, or -S(O)-$C_1$-$C_6$ alkyl;
$R^6$ is selected from H, halogen, $C_1$-$C_6$ alkyl, $C_1$-$C_6$ haloalkyl, or -O-$C_1$-$C_6$ alkyl;
$R^7$ is selected from H or $C_1$-$C_6$ alkyl.

2. The compound of formula (I) or the stereoisomer thereof or the pharmaceutically acceptable salt thereof according to claim 1, wherein $R^1$ is selected from OH.

3. The compound of formula (I) or the stereoisomer thereof or the pharmaceutically acceptable salt thereof according to claim 1 or 2, wherein $R^2$ is selected from $CH_3$ or $CF_3$.

4. The compound of formula (I) or the stereoisomer thereof or the pharmaceutically acceptable salt thereof according to any one of claims 1-3, wherein $R^3$ is selected from $C_1$-$C_6$ alkyl, 4- to 10-membered heterocyclyl, or 5- to 10-membered heteroaryl, wherein the $C_1$-$C_6$ alkyl, 4- to 10-membered heterocyclyl, or 5- to 10-membered heteroaryl is optionally substituted with $R^{3a}$;

   or $R^3$ is selected from $C_1$-$C_6$ alkyl or 4- to 6-membered heterocyclyl, wherein the $C_1$-$C_6$ alkyl or 4- to 6-membered heterocyclyl is optionally substituted with $R^{3a}$;
   or $R^3$ is selected from $C_1$-$C_6$ alkyl, wherein the $C_1$-$C_6$ alkyl is optionally substituted with $R^{3a}$.

5. The compound of formula (I) or the stereoisomer thereof or the pharmaceutically acceptable salt thereof according to any one of claims 1-4, wherein $R^{3a}$ is selected from halogen, OH, -$COR^{3b}$, - $COOR^{3b}$, -$NHCOR^{3b}$, -O-$C_1$-$C_6$ alkyl, phenyl, $C_1$-$C_6$ alkyl, 4- to 6-membered heterocyclyl, or 5- to 10-membered heteroaryl, wherein the phenyl, $C_1$-$C_6$ alkyl, 4- to 6-membered heterocyclyl, or 5- to 10-membered heteroaryl is optionally substituted with $R^{3c}$;

   or $R^{3b}$ is selected from $C_1$-$C_6$ alkyl, phenyl, or 5- to 6-membered heteroaryl;

or $R^{3c}$ is selected from $C_1$-$C_6$ alkyl, -O-$C_1$-$C_6$ alkyl, phenyl, or 5- to 6-membered heteroaryl;
or $R^{3c}$ is selected from $C_1$-$C_6$ alkyl or -O-$C_1$-$C_6$ alkyl.

**6.** The compound of formula (I) or the stereoisomer thereof or the pharmaceutically acceptable salt thereof according to any one of claims 1-5, wherein X is N.

**7.** The compound of formula (I) or the stereoisomer thereof or the pharmaceutically acceptable salt thereof according to any one of claims 1-6, wherein Y is selected from O or $NR^7$;

or Y is selected from S, O, or NH;
or Y is selected from S or O.

**8.** The compound of formula (I) or the stereoisomer thereof or the pharmaceutically acceptable salt thereof according to any one of claims 1-7, wherein $R^4$ is selected from halogen or CN;

or $R^4$ is selected from F, Cl, or CN;
or $R^4$ is CN.

**9.** The compound of formula (I) or the stereoisomer thereof or the pharmaceutically acceptable salt thereof according to any one of claims 1-8, wherein $R^5$ is selected from $C_1$-$C_6$ haloalkyl, -S-$C_1$-$C_6$ alkyl, -O-$C_1$-$C_6$ alkyl, -Se-$C_1$-$C_6$ alkyl, -$S(O)_2$-$C_1$-$C_6$ alkyl, or -S(O)-$C_1$-$C_6$ alkyl;

or $R^5$ is selected from halogen, $C_1$-$C_6$ alkyl, $C_1$-$C_6$ haloalkyl, -COO$C_1$-$C_6$ alkyl, -S-$C_1$-$C_6$ alkyl, or -O-$C_1$-$C_6$ alkyl;
or $R^5$ is selected from $C_1$-$C_6$ haloalkyl or -S-$C_1$-$C_6$ alkyl.

**10.** The compound of formula (I) or the stereoisomer thereof or the pharmaceutically acceptable salt thereof according to any one of claims 1-9, wherein $R^6$ is selected from H or halogen.

**11.** The compound of formula (I) or the stereoisomer thereof or the pharmaceutically acceptable salt thereof according to any one of claims 1-10, wherein the compound of formula (I) or the stereoisomer thereof or the pharmaceutically acceptable salt thereof is selected from a compound of formula (I-1) or a stereoisomer thereof or a pharmaceutically acceptable salt thereof:

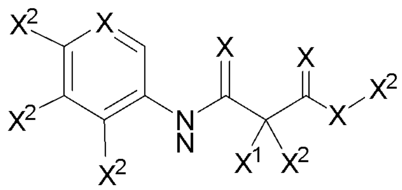

(I-1)

wherein $R^1$, $R^2$, $R^3$, $R^4$, $R^5$, $R^6$, and X are as defined in any one of claims 1-10.

**12.** The compound of formula (I) or the stereoisomer thereof or the pharmaceutically acceptable salt thereof according to claim 1, wherein the compound of formula (I) or the stereoisomer thereof or the pharmaceutically acceptable salt thereof is selected from the following compounds or stereoisomers thereof or pharmaceutically acceptable salts thereof:

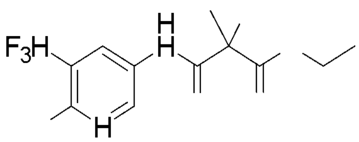 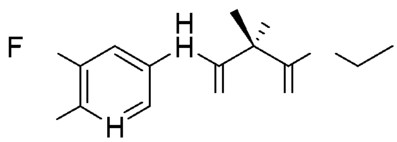 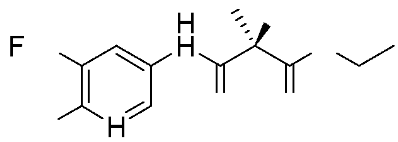

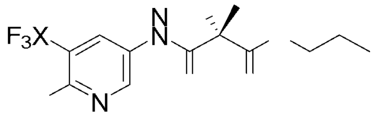
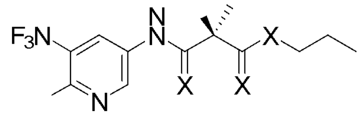
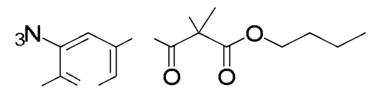
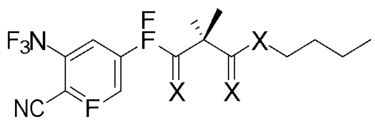
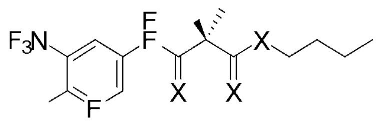
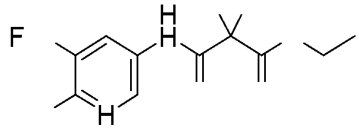
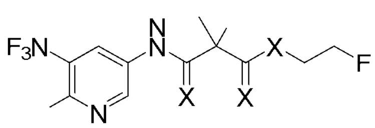
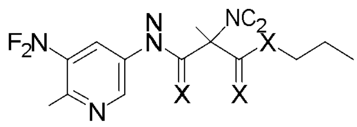
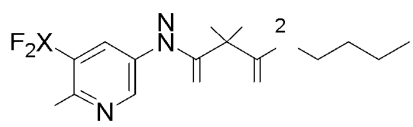
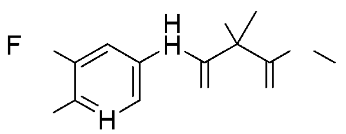
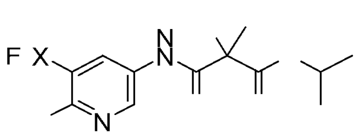
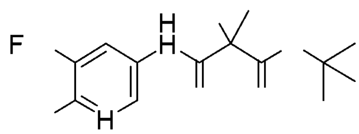
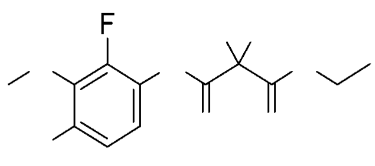
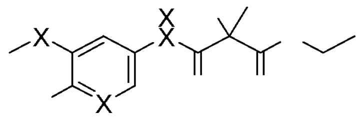
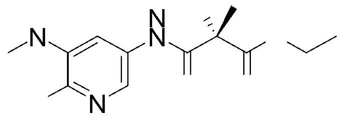
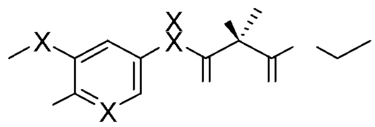
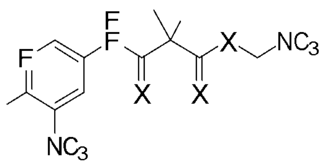
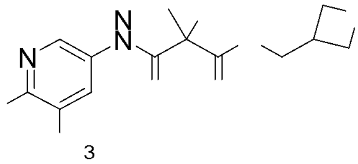
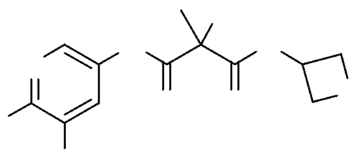
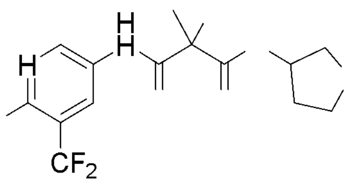
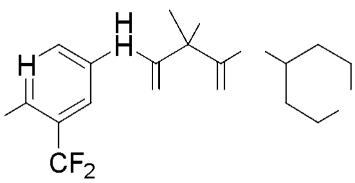
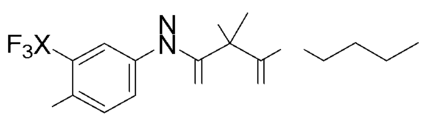
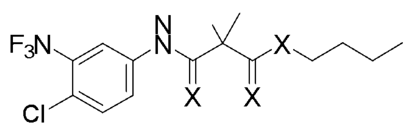
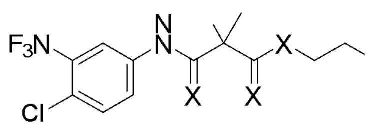
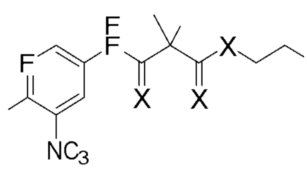
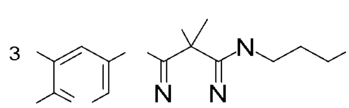
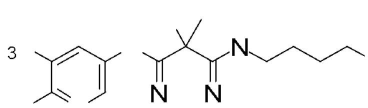
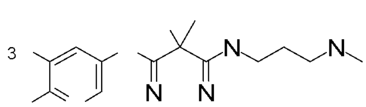
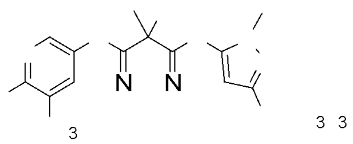
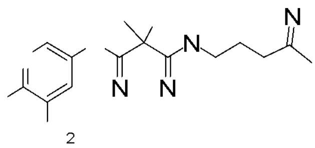

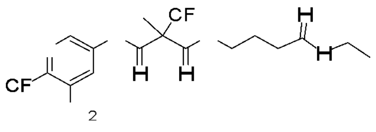
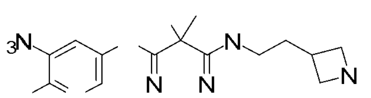
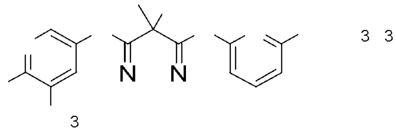

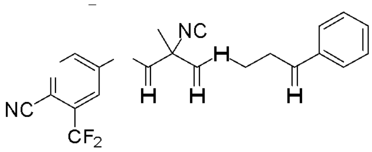
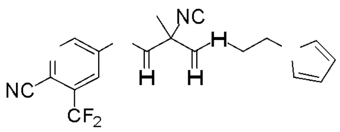
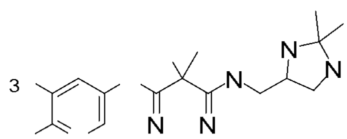

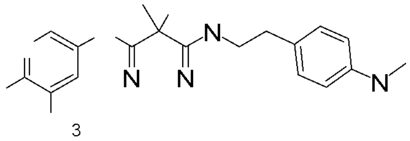
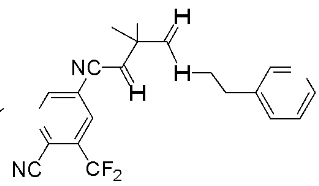
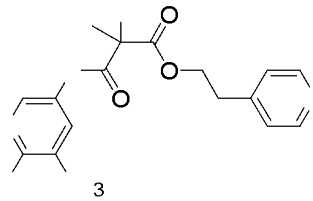

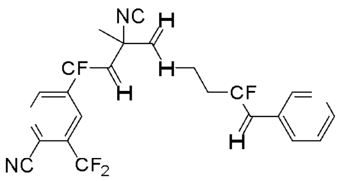
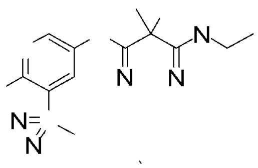
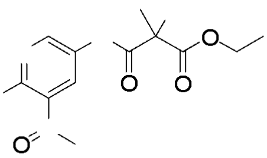

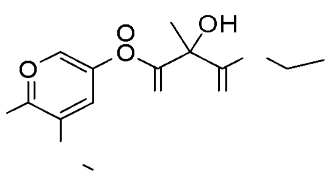
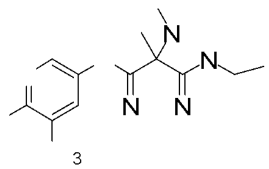
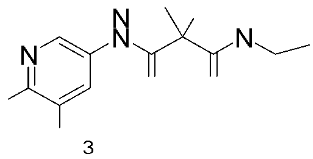

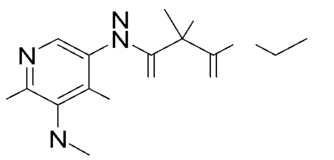
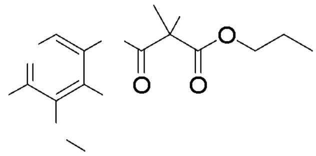
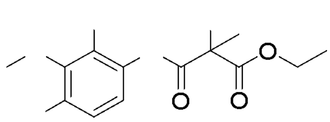

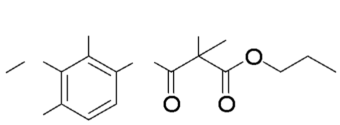
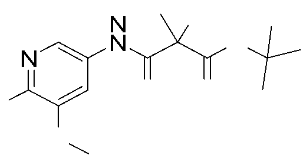
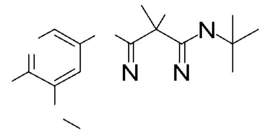

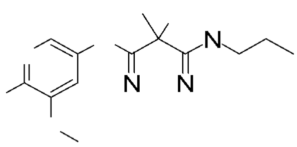
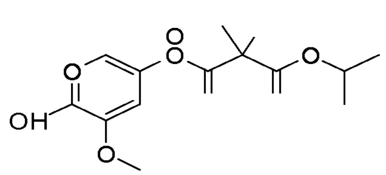
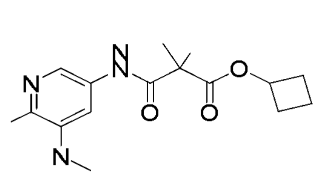

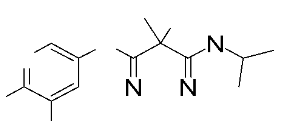
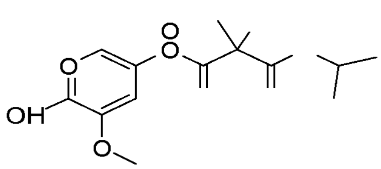
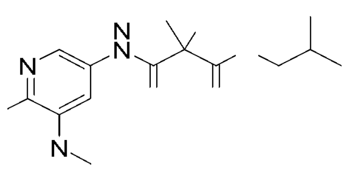

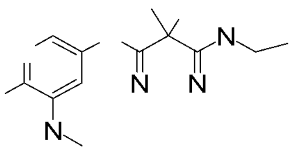 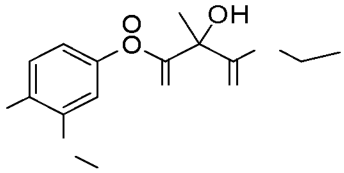 or 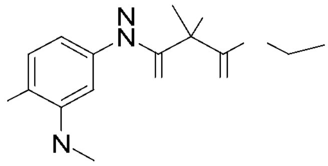 .

**13.** A pharmaceutical composition, comprising the compound of formula (I) or the stereoisomer thereof or the pharmaceutically acceptable salt thereof according to any one of claims 1-12 and a pharmaceutically acceptable excipient.

**14.** Use of the compound of formula (I) or the stereoisomer thereof or the pharmaceutically acceptable salt thereof according to any one of claims 1-12 or the pharmaceutical composition according to claim 13 in preparing a medicament for preventing or treating an androgen receptor-mediated disease.

**15.** A method for preventing or treating an androgen receptor-mediated disease in a mammal, comprising administering to a mammal, preferably a human, in need of the treatment a therapeutically effective amount of the compound of formula (I) or the stereoisomer thereof or the pharmaceutically acceptable salt thereof according to any one of claims 1-12 or the pharmaceutical composition according to claim 13.

**16.** The use according to claim 14 or the method according to claim 15, wherein the androgen receptor-mediated disease is selected from androgenetic alopecia.

**INTERNATIONAL SEARCH REPORT**

International application No.
**PCT/CN2024/114989**

**A. CLASSIFICATION OF SUBJECT MATTER**

C07D213/84(2006.01)i； A61K31/44(2006.01)i； A61P17/14(2006.01)i

According to International Patent Classification (IPC) or to both national classification and IPC

**B. FIELDS SEARCHED**

Minimum documentation searched (classification system followed by classification symbols)

IPC： C07D A61K A61P

Documentation searched other than minimum documentation to the extent that such documents are included in the fields searched

Electronic data base consulted during the international search (name of data base and, where practicable, search terms used)

CNTXT, ENTXT, DWPI, STN (REGISTRY, CAPLUS, MARPAT), CNKI, Google学术, Google Scholar: 中国药科大学, 江苏先声药业, 张雁, 焦宇, 黄炼成, 赵思奇, 唐锋, 高珍娜, 沈晓玲, 刘乐, 陈亚东, 陆涛, 彭少平, 王峰, 氰基, 羟基, 氧代, 丙酸, 雄激素, 脱发, cyano? , hydroxyl? , oxo, propionic acid? , androgen? , alopecia? , 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 1639110 A (UNIV TENNESSEE RES FOUNDATION) 13 July 2005 (2005-07-13) see claims 1-42, and description, pages 6-7 | 1-16 |
| A | US 4636505 A (ICI PLC) 13 January 1987 (1987-01-13) see claims 1-9, and description, columns 9-14, tables | 1-16 |
| A | CN 1471508 A (THE UNIVERSITY OF TENNESSEE RESEARCH CORPORATION) 28 January 2004 (2004-01-28) see claims 1-25 | 1-16 |
| A | CN 1812961 A (ORION CORPORATION) 02 August 2006 (2006-08-02) see claims 1-11 | 1-16 |
| A | WO 2008011072 A2 (OSURF (OHIO STATE UNIVERSITY RESEARCH FOUNDATION) et al.) 24 January 2008 (2008-01-24) see claims 1-4, 7, 23-35, 114-115, etc., abstract, and description, paragraphs 0078-0079 | 1-16 |
| A | WO 2021170464 A1 (BASF SE) 02 September 2021 (2021-09-02) see description, embodiments 12-13, and table 1 | 1-16 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 October 2024** | **08 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/114989**

**C. DOCUMENTS CONSIDERED TO BE RELEVANT**

| Category* | Citation of document, with indication, where appropriate, of the relevant passages | Relevant to claim No. |
|---|---|---|
| A | YANG, Z.K. et al. "Iodine-Catalyzed α-Hydroxylation of β-Dicarbonyl Compounds" *Asian Journal of Organic Chemistry,* Vol. 12, No. (2), 08 January 2023 (2023-01-08), ISSN: 2193-5807, see e202200639, pages (1-8) | 1-16 |
| A | NORA C. et al. "Analytical Strategies to Detect Enobosarm Administration in Bovines" *Food Additives & Contaminants, Part A,* Vol. 34, No. (4), 28 November 2016 (2016-11-28), ISSN: 1944-0057, see pages 632-640, table 1 | 1-16 |

Form PCT/ISA/210 (second sheet) (July 2022)

**INTERNATIONAL SEARCH REPORT**

International application No.
**PCT/CN2024/114989**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-16**
because they relate to subject matter not required to be searched by this Authority, namely:

Claims 15-16 relate to a method for treating a human/animal body. The present search report is provided on the basis of the use of the compound or pharmaceutical composition for preparing a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/114989**

| Patent document cited in search report | | | Publication date (day/month/year) | Patent family member(s) | | | Publication date (day/month/year) |
|---|---|---|---|---|---|---|---|
| CN | 1639110 | A | 13 July 2005 | HRP | 20040818 | A2 | 28 February 2005 |
| | | | | CA | 2476657 | A1 | 12 September 2003 |
| | | | | EP | 1487780 | A1 | 22 December 2004 |
| | | | | EP | 1487780 | A4 | 16 November 2005 |
| | | | | IL | 163743 | A0 | 18 December 2005 |
| | | | | US | 2004147489 | A1 | 29 July 2004 |
| | | | | WO | 03074471 | A1 | 12 September 2003 |
| | | | | MXPA | 04008413 | A | 08 June 2005 |
| | | | | BR | 0307981 | A | 17 January 2006 |
| | | | | JP | 2006506318 | A | 23 February 2006 |
| | | | | AU | 2003217303 | A1 | 16 September 2003 |
| US | 4636505 | A | 13 January 1987 | JPS | 5933250 | A | 23 February 1984 |
| | | | | JPH | 0432061 | B2 | 28 May 1992 |
| | | | | CS | 399991 | A3 | 17 June 1992 |
| | | | | ES | 8607936 | A1 | 01 June 1986 |
| | | | | LU | 88769 | I2 | 05 November 1996 |
| | | | | IE | 831732 | L | 23 January 1984 |
| | | | | IE | 55941 | B1 | 27 February 1991 |
| | | | | ZA | 8305182 | B | 30 May 1984 |
| | | | | NL | 950029 | I1 | 01 February 1996 |
| | | | | NL | 950029 | I2 | 03 March 1997 |
| | | | | NO | 1996014 | I1 | 10 December 1996 |
| | | | | FI | 832644 | A0 | 20 July 1983 |
| | | | | FI | 832644 | A | 24 January 1984 |
| | | | | FI | 83770 | B | 15 May 1991 |
| | | | | FI | 83770 | C | 26 August 1991 |
| | | | | ATE | 28864 | T1 | 15 August 1987 |
| | | | | HU | 191296 | B | 27 February 1987 |
| | | | | AU | 1693783 | A | 26 January 1984 |
| | | | | AU | 556328 | B2 | 30 October 1986 |
| | | | | ES | 544189 | A0 | 16 September 1986 |
| | | | | GR | 79232 | B | 22 October 1984 |
| | | | | JPH | 02131462 | A | 21 May 1990 |
| | | | | ZA | 835182 | B | 30 May 1984 |
| | | | | ES | 8607915 | A1 | 01 June 1986 |
| | | | | EP | 0100172 | A1 | 08 February 1984 |
| | | | | EP | 0100172 | B1 | 12 August 1987 |
| | | | | ES | 524392 | A0 | 01 November 1985 |
| | | | | IL | 69217 | A0 | 30 November 1983 |
| | | | | IL | 69217 | A | 31 March 1987 |
| | | | | NO | 832599 | L | 24 January 1984 |
| | | | | NO | 164974 | B | 27 August 1990 |
| | | | | NO | 164974 | C | 05 December 1990 |
| | | | | PT | 77087 | A | 01 August 1983 |
| | | | | PT | 77087 | B | 27 January 1986 |
| | | | | HK | 92690 | A | 16 November 1990 |
| | | | | DK | 322783 | D0 | 13 July 1983 |
| | | | | CA | 1249823 | A | 07 February 1989 |
| | | | | MX | 9203451 | A | 01 August 1992 |
| | | | | NZ | 204995 | A | 30 August 1985 |

Form PCT/ISA/210 (patent family annex) (July 2022)

**INTERNATIONAL SEARCH REPORT**
**Information on patent family members**

International application No.
**PCT/CN2024/114989**

| Patent document cited in search report | Publication date (day/month/year) | Patent family member(s) | Publication date (day/month/year) |
|---|---|---|---|
| | | DE 3372965 D1 | 17 September 1987 |
| CN 1471508 A | 28 January 2004 | CY 1110459 T1 | 29 April 2015 |
| | | SI 1401801 T1 | 30 April 2007 |
| | | CY 1105956 T1 | 06 April 2011 |
| | | EA 200300283 A1 | 28 August 2003 |
| | | EA 007101 B1 | 30 June 2006 |
| | | BR 0114801 A | 14 October 2003 |
| | | PT 1491524 E | 16 April 2009 |
| | | HRP 20030118 A2 | 28 February 2005 |
| | | HRP 20030118 B1 | 30 June 2008 |
| | | EP 1401801 A1 | 31 March 2004 |
| | | EP 1401801 A4 | 18 August 2004 |
| | | EP 1401801 B1 | 02 November 2006 |
| | | WO 0216310 A1 | 28 February 2002 |
| | | WO 0216310 A9 | 18 July 2002 |
| | | ES 2275717 T3 | 16 June 2007 |
| | | GEP 20053586 B | 25 July 2005 |
| | | KR 20030061783 A | 22 July 2003 |
| | | JP 2006306880 A | 09 November 2006 |
| | | DK 1491524 T3 | 04 May 2009 |
| | | AU 2001285230 B2 | 25 January 2007 |
| | | AU 2001285230 C1 | 13 March 2008 |
| | | EP 2284149 A1 | 16 February 2011 |
| | | PT 1401801 E | 28 February 2007 |
| | | DE 60124322 D1 | 14 December 2006 |
| | | DE 60124322 T2 | 31 May 2007 |
| | | EA 200501269 A1 | 24 February 2006 |
| | | EA 014224 B1 | 29 October 2010 |
| | | EP 1491524 A2 | 29 December 2004 |
| | | EP 1491524 A3 | 20 April 2005 |
| | | EP 1491524 B1 | 18 February 2009 |
| | | ES 2321933 T3 | 15 June 2009 |
| | | ATE 423092 T1 | 15 March 2009 |
| | | HK 1062011 A1 | 15 October 2004 |
| | | CA 2420279 A1 | 28 February 2002 |
| | | CA 2420279 C | 19 July 2011 |
| | | US 2002099096 A1 | 25 July 2002 |
| | | US 6569896 B2 | 27 May 2003 |
| | | ATE 344233 T1 | 15 November 2006 |
| | | DE 60137728 D1 | 02 April 2009 |
| | | AU 8523001 A | 04 March 2002 |
| | | EP 1705174 A1 | 27 September 2006 |
| | | HK 1072243 A1 | 19 August 2005 |
| | | JP 2004518617 A | 24 June 2004 |
| | | DK 1401801 T3 | 05 March 2007 |
| | | MXPA 03001632 A | 10 September 2004 |
| CN 1812961 A | 02 August 2006 | ZA 200510385 A | 29 November 2006 |
| WO 2008011072 A2 | 24 January 2008 | AU 2007275736 A1 | 24 January 2008 |
| | | EA 200900203 A1 | 30 June 2009 |
| | | US 2009156614 A1 | 18 June 2009 |

Form PCT/ISA/210 (patent family annex) (July 2022)

**INTERNATIONAL SEARCH REPORT**
**Information on patent family members**

International application No.
**PCT/CN2024/114989**

| Patent document cited in search report | Publication date (day/month/year) | Patent family member(s) | Publication date (day/month/year) |
|---|---|---|---|
| | | US 2011077221 A1 | 31 March 2011 |
| | | US 8110562 B2 | 07 February 2012 |
| | | JP 2009543871 A | 10 December 2009 |
| | | MX 2009000714 A | 27 July 2009 |
| | | WO 2008011073 A2 | 24 January 2008 |
| | | WO 2008011073 A3 | 23 October 2008 |
| | | WO 2008011072 A3 | 05 June 2008 |
| | | IL 196534 A0 | 18 November 2009 |
| | | AR 063208 A1 | 14 January 2009 |
| | | CA 2658098 A1 | 24 January 2008 |
| | | BRPI 0713200 A2 | 10 April 2012 |
| | | EA 200900202 A1 | 30 June 2009 |
| | | BRPI 0713198 A2 | 16 October 2012 |
| | | CA 2658096 A1 | 24 January 2008 |
| | | KR 20090035707 A | 10 April 2009 |
| | | AU 2007275737 A1 | 24 January 2008 |
| | | KR 20090030345 A | 24 March 2009 |
| | | JP 2009543872 A | 10 December 2009 |
| | | EP 2041077 A2 | 01 April 2009 |
| | | MX 2009000715 A | 22 July 2009 |
| | | IL 196533 A0 | 18 November 2009 |
| | | EP 2040709 A2 | 01 April 2009 |
| WO 2021170464 A1 | 02 September 2021 | CO 2022012118 A2 | 20 September 2022 |
| | | KR 20220148818 A | 07 November 2022 |
| | | CL 2022002310 A1 | 03 February 2023 |
| | | EP 4110763 A1 | 04 January 2023 |
| | | MX 2022010566 A | 21 September 2022 |
| | | AU 2021227378 A1 | 22 September 2022 |
| | | US 2023150989 A1 | 18 May 2023 |
| | | AR 121464 A1 | 08 June 2022 |
| | | IL 295901 A | 01 October 2022 |
| | | BR 112022017121 A2 | 16 November 2022 |
| | | CR 20220420 A | 03 October 2022 |
| | | JP 2023514787 A | 10 April 2023 |
| | | CA 3169141 A1 | 02 September 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311091809
- CN 202410802546
- DD 120403


### Non-patent literature cited in the description


- **MAEHR**. *J. Ch*, 1985, vol. 62, 114-120